# FASCICULE DE BREVET EUROPEEN

(11) **EP 1 647 548 B1**
(45) Date de publication et mention de la délivrance du brevet: **25.08.2010**
(21) Numéro de dépôt: 05292147.5
(22) Date de dépôt: 13.10.2005
(51) Int. Cl.: C07D 215/12, C07D 401/06, C07D 233/54, C07D 235/14, C09B 44/16

(54) **Composé bis-hydrazone dicationique, composition tinctoriale comprenant au moins un tel composé, procédé de mise en oeuvre et utilisations**
Dikationische Bishydrazonverbindungen , Färbezusammensetzungen enthaltend mindestens eine solche Verbindung, Verfahren zu deren Herstellung und ihre Verwendung
Dictionic bishydrazones, dyeingcompositions containig at least one of these compounds, process of their preparation and uses thereoff

(30) Priorité: 14.10.2004 FR 0410870
(43) Date de publication de la demande: 19.04.2006
(73) Titulaire: L'OREAL S.A., 75008 Paris Cedex (FR)
(72) Inventeur: Hervé, David, 94210 La Varenne Saint Hilaire (FR); Greaves, Andrew, 77144 Montevrain (FR); Hercouet, Leïla, 93360 Neuilly Plaisance (FR); Lagrange, Alain, 77700 Coupvray (FR)
(74) Mandataire: Casalonga, Axel

(56) Documents cités:
- WO-A-01/74950
- WO-A-95/15144
- FR-A- 2 757 388
- FR-A- 2 825 623
- FR-A- 2 825 702
- FR-A- 2 825 703

## Description

La présente demande a pour objet une nouvelle famille de composés particuliers de type bis-hydrazone dicationique, leur utilisation en tant que colorants directs pour la teinture des fibres kératiniques ainsi que les compositions tinctoriales pour la teinture des fibres kératiniques comprenant dans un milieu de teinture approprié au moins un tel composé bis-hydrazone dicationique.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

Ce procédé de coloration d'oxydation consiste à appliquer sur les fibres kératiniques, des bases d'oxydation ou un mélange de bases d'oxydation et de coupleurs avec un agent oxydant, par exemple de l'eau oxygénée, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliquées à pH basique, les compositions permettent d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est à dire de la rendre plus visible.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer les fibres.

Il est connu par exemple d'utiliser des colorants directs du type nitrés benzèniques, anthraquinoniques, nitropyridiniques, des colorants du type azoïques, xanthéniques, acridiniques aziniques ou des colorants triarylméthane, par exemple.

Il est également connu de la demande WO 2004/083312 des colorants dimères cationiques dans lesquels deux chromophores cationiques sont reliés par une chaîne carbonée comprenant au moins un noyau aromatique.

Les colorations qui en résultent sont des colorations particulièrement chromatiques qui sont cependant temporaires ou semi-permanentes. En effet, la nature des interactions qui lient les colorants directs à la fibre kératinique, et leur désorption de la surface et/ou du coeur de la fibre font que la puissance tinctoriale et la tenue aux lavages ou à la transpiration des colorations peuvent être encore considérées insuffisantes. Certains colorants directs peuvent en outre être sensibles à la lumière du fait de la faible résistance du chromophore vis-à-vis des attaques photochimiques et conduire dans le temps à un affadissement de la coloration des cheveux.

Il est connu d'utiliser des colorants directs en combinaison avec des agents oxydants. Cependant, les colorants directs peuvent être sensibles à l'action des agents oxydants tels que l'eau oxygénée, et les agents réducteurs tels que le bisulfite de sodium, ce qui les rend généralement difficilement utilisables dans ces conditions.

Il existe donc un réel besoin de rechercher des colorants directs chromatiques qui permettent de teindre les fibres kératiniques aussi puissamment que les colorants d'oxydation, qui soient aussi stables qu'eux à la lumière, qui soient également résistants aux intempéries, aux lavages et à la transpiration, et en outre, suffisamment stables en présence d'agents oxydants et réducteurs pour pouvoir obtenir simultanément un éclaircissement de la fibre soit par utilisation de compositions directes éclaircissantes les contenant, soit par l'utilisation de compositions de coloration d'oxydation les contenant. Il existe aussi un réel besoin de rechercher des colorants directs qui permettent de teindre les fibres kératiniques pour obtenir une gamme très large de couleurs, en particulier très chromatiques, sans oublier les nuances dites « fondamentales » comme les noirs et les marrons et les nuances à reflets.

De manière surprenante et avantageuse, la Demanderesse vient de découvrir une nouvelle famille de composés bis-hydrazones dicationiques utilisables en tant que colorants directs pour la teinture des fibres kératiniques, en particulier des fibres kératiniques humaines telles que les cheveux. Les compositions tinctoriales comprenant au moins un composé bis-hydrazone dicationique présentent ces améliorations.

Les compositions selon la présente demande permettent l'obtention de teintures résistantes aux agents extérieurs (soleil, intempéries) ainsi qu'aux shampooings et à la transpiration.

En outre, ces compositions présentent une montée améliorée. L'invention a aussi pour objet le procédé de teinture des fibres kératiniques à partir de composés bis-hydrazones dicationiques ainsi que l'utilisation de ces composés pour obtenir des teintures présentant une bonne résistance aux agents extérieurs et aux shampooings.

Au sens de la présente invention, et à moins qu'une indication différente ne soit donnée :
- un radical alkyle ou alkylène est linéaire ou ramifié ;
- les radicaux alkyle ou alkylène ou la partie alkyle d'un radical peuvent être substitués par au moins un substituant choisi parmi les groupements :
   - hydroxyle,
   - alcoxy en C₁-C₄, (poly)hydroxyalcoxy en C₂-C₄,
   - amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, saturé ou insaturé éventuellement substitué comprenant éventuellement au moins un autre hétéroatome différent ou non de l'azote ;
- les radicaux aryles ou hétéroaryles ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄ , éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxyle ;
   - un radical alcoxy en C₁-C₂ ; un radical (poly)-hydroxyalcoxy en C₂-C₄ ;
   - un radical amino ; un radical amino substitué par un ou deux radicaux alkyle, identiques ou différents, en C₁-C₄ éventuellement porteurs d'au moins un groupement hydroxyle ou amino, par deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfénylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂-) dans lequel les radicaux R, identiques ou non, représentent un atome d'hydrogène, un radical alkyle en C₁-C₄ éventuellement porteur d'au moins un groupement hydroxyle,
   - un radical thiol ;
   - un radical alkylthio en C₁-C₈, de préférence en C₁-C₄, linéaire ou ramifié, substitué ou non ;
   - un radical carboxylique sous forme acide ou salifiée (de préférence avec un métal alcalin ou un ammonium, substitué ou non) ;
   - un radical nitro;
   - un groupement nitrile (CN) ;
   - un groupement trifluorométhyle(CF₃) ;
- la partie cyclique ou hétérocyclique d'un radical non aromatique peut être substituée par au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- une chaîne hydrocarbonée est insaturée lorsqu'elle comporte une ou plusieurs liaisons doubles et/ou une ou plusieurs liaisons triples ;
- un radical hétéroaromatique ou hétéroaryle, correspond à un radical aromatique dans lequel au moins l'un des atomes de carbone est remplacé par un hétéroatome, choisi parmi l'azote, l'oxygène ou le soufre.

En outre, on dit que deux chromophores COL sont différents lorsque la structure chimique des chromophores est différente, ou si les chromophores ont une même structure chimique alors les substituants sont différents d'un chromophore à l'autre, ou encore si les chromophores ont une même structure chimique et des radicaux identiques, la position respective desdits radicaux est différente d'un chromophore à l'autre.

De plus, sauf indication contraire, les bornes délimitant l'étendue d'une plage de valeurs sont comprises dans cette plage de valeurs.

Les composés de l'invention sont des composés bis-hydrazones dicationiques correspondant à la formule générale (I) :

COL -L- COL

dans laquelle chacun des chromophores COL, identique ou différent, correspond aux formules (Ia), (Ib), (Ic), (Id), (Ie), (If) et (Ig) suivantes : 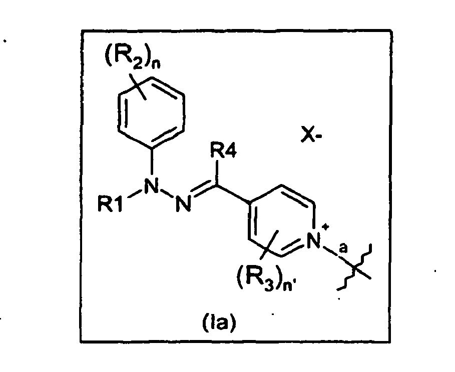 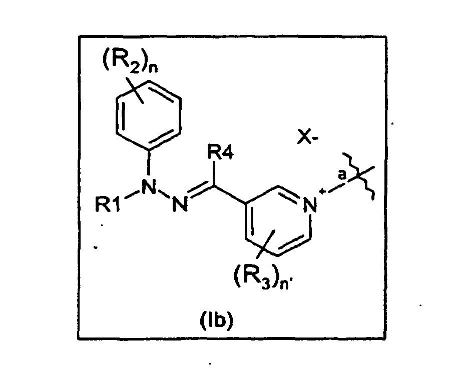 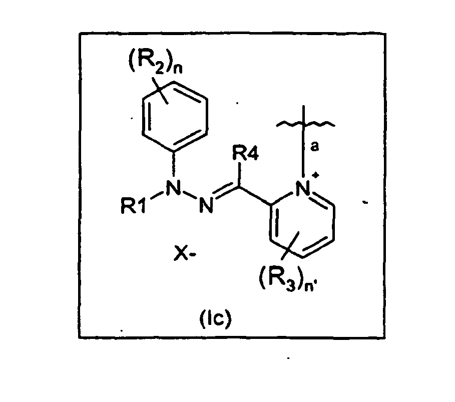 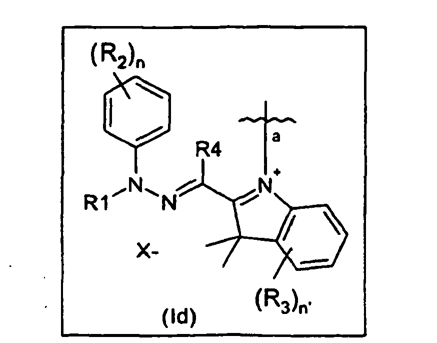 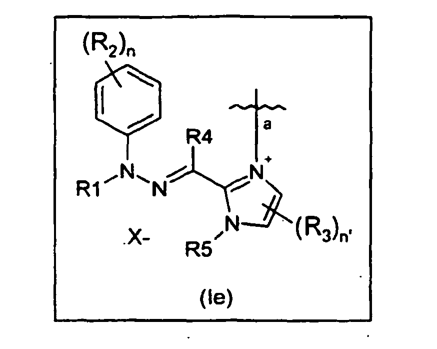 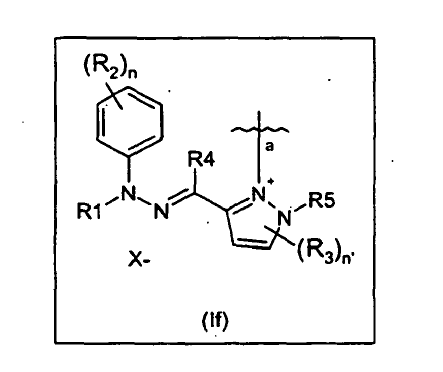 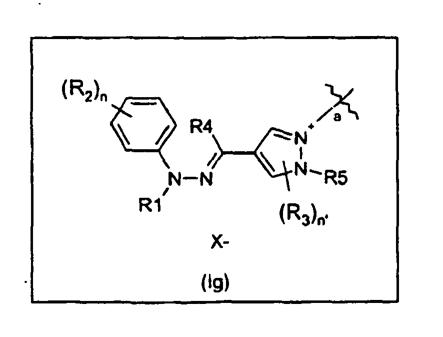 dans lesquelles :
- les groupes R₁ et R₅, indépendamment les uns des autres représentent une chaîne hydrocarbonée en C₁-C₂₀, de préférence en C₁-C₁₆ linéaire ou ramifiée, saturée ou insaturée, pouvant former un ou plusieurs cycles carbonés comportant de 3 à 7 chaînons éventuellement substitués, éventuellement aromatiques, cette chaîne étant éventuellement substituée, éventuellement interrompue par un ou plusieurs hétéroatomes ou par un ou plusieurs groupements portant un hétéroatome, ces hétéroatomes étant de préférence choisi parmi l'oxygène, l'azote ;
- les groupes R₂ et R₃, indépendamment l'un de l'autre, représentent:
   - un atome d'halogène choisi parmi le brome, le chlore ou le fluor ;
   - une chaîne hydrocarbonée en C₁-C₁₆, linéaire ou ramifiée, saturée ou insaturée, pouvant former un ou plusieurs cycles carbonés comportant de 3 à 6 chaînons éventuellement aromatiques, cette chaîne étant éventuellement substituée, éventuellement interrompue par un ou plusieurs hétéroatomes ou par un ou plusieurs groupements portant au moins un hétéroatome, ces hétéroatomes étant de préférence choisi parmi l'oxygène, l'azote ;
   - un groupement hydroxyle,
   - un groupement alcoxy en C₁-C₄ ; un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle en C₁-C₄ ; un groupement aryloxy éventuellement substitué.
   - un groupement amino, un groupement amino substitué par un ou plusieurs radicaux alkyle en C₁-C₄, identiques ou différents, éventuellement porteurs d'au moins un groupement hydroxyle, les deux radicaux alkyle pouvant éventuellement former avec l'atome d'azote auquels ils sont rattachés, un hétérocycle à 5 ou 6 chaînons saturé ou insaturé éventuellement substitué, éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄ ; un groupement carbamoyle ((R)₂N-CO-) dans lequel les radicaux R indépendamment les uns des autres, représentent un atome d'hydrogène, un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ; un groupement sulfonamide ((R)₂N-SO₂-) dans lequel les radicaux R indépendamment les uns des autres, représentent un atome d'hydrogène, un radical alkyle en C₁-C₄, ; un groupement alkylthio (R-S-) dans lequel le groupement R représente un radical alkyle en C₁-C₄ ;un groupement alkylsulfonylamino (RSO₂-NR'-) dans lequel les radicaux R, R', indépendamment les uns des autres, représentent un atome d'hydrogène, un radical alkyle en C₁-C₄ ;
   - un groupement nitro ; un groupement cyano ; un atome d'halogène, de préférence le chlore, le fluor ;
   un groupement trifluorométhyle(CF₃) ;
- il est à noter que lorsque le cycle principal ne porte pas le nombre de substituants maximum, alors la position non substituée porte un atome d'hydrogène ;
- R₁ et R₂ peuvent également former avec l'atome d'azote substitué par R₁, un hétérocycle saturé ou insaturé à 5 ou 6 chaînons substitué ou non substitué ;
- deux radicaux R₂ adjacents peuvent former entre eux et avec les atomes de carbone auxquels ils sont reliés un cycle aromatique ou un hétérocycle saturé ou insaturé à 5 ou 6 chaînons substitué ou non ;
- deux radicaux R₃ adjacents peuvent former entre eux un cycle aromatique ou un hétérocycle saturé ou insaturé à 5 ou 6 chaînons substitué ou non ;
- les groupes R₄, indépendamment les uns des autres, représentent :
   - un atome d'hydrogène ;
   - une chaîne hydrocarbonée en C₁-C₁₆, linéaire ou ramifiée, saturée ou insaturée, pouvant former un ou plusieurs cycles carbonés comportant de 3 à 6 chaînons éventuellement aromatiques, cette chaîne étant éventuellement substituée, éventuellement interrompue par un ou plusieurs hétéroatomes ou par un ou plusieurs groupements portant au moins un hétéroatome, ces hétéroatomes étant de préférence choisi parmi l'oxygène, l'azote ;
   - un groupement amino, un groupement amino substitué par un ou plusieurs radicaux alkyle en C₁-C₄, identiques ou différents, éventuellement porteurs d'au moins un groupement hydroxyle, les deux radicaux alkyle pouvant éventuellement former avec l'atome d'azote auquels ils sont rattachés, un hétérocycle à 5 ou 6 chaînons saturé ou insaturé éventuellement substitué, éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ;un groupement alkylsulfonylamino (RSO₂-NR'-) dans lequel les radicaux R, R', indépendamment les uns des autres, représentent un atome d'hydrogène, un radical alkyle en C₁-C₄ ;
- n est un entier compris entre 0 et 5,
- n'est un entier compris entre 0 et 4,
- la liaison *a* issue des formules (Ia), (Ib), (Ic), (Id), (Ie), (If) et (Ig) relie chacun des chromophores COL au bras de liaison L de la formule (I).
- X est un anion ou un mélange d'anions, organiques ou minéraux permettant d'équilibrer la ou les charges des composés (Ia), (Ib), (Ic), (Id), (Ie), (If) et (Ig) par exemple choisi parmi un halogénure tel que chlorure, bromure, fluorure, iodure ; un hydroxyde ; un sulfate ; un hydrogénosulfate ; les alkylsulfates pour lesquels la partie alkyle, linéaire ou ramifiée, est en C₁-C₆, comme l'ion méthylsulfate, éthylsulfate ; les carbonates et hydrogénocarbonates tels que l'acétate, le citrate, le tartrate, l'oxalate ; les alkylsulfonates pour lesquels la partie alkyle, linéaire ou ramifiée, est en C₁-C₆ comme l'ion méthylsulfonate ; les arylsulfonates pour lesquels la partie aryle, de préférence phényle, est éventuellement substituée par un ou plusieurs radicaux alkyle en C₁-C₄ tel que par exemple le 4-toluylsulfonate ; les alkylsulfonyle tel que le mésylate,

Le groupe L est un bras de liaison reliant les deux chromophores COL, choisi parmi les radicaux alkylène (CₙH₂ₙ) comprenant de 1 à 60 atomes de carbone, de préférence de 2 à 40 atomes de carbone, et plus préféré de 2 à 20 atomes de carbone, éventuellement substitués et/ou interrompus par un ou plusieurs hétéroatomes choisis parmi l'oxygène, l'azote, le soufre et/ou parmi un groupement SO₂, CO L ne comportant pas de groupe peroxyde, nitro, diazo ou nitroso, le bras de liaison L étant relié à l'atome d'azote quaternisé de chacun des chromophores COL par l'intermédiaire d'un atome de carbone et L n'étant pas cationique.

Selon une variante, le bras de liaison L est une chaîne hydrocarbonée qui isole chacun des chromophores de façon à stopper la délocalisation électronique de chacun des chromophores.

Ces radicaux alkylène sont par exemple les propylène, butylène, pentylène, hexylène, etc ;

A titre de bras de liaison, on peut citer les alkylènes cités dans US 5 708 151.

De préférence, les composés de formule (I) selon la présente invention sont tels que les groupes R₁ sont identiques et représentent préférentiellement un radical alkyle en C₁-C₄, éventuellement substitué par un ou plusieurs radicaux choisis parmi les radicaux hydroxy, alcoxy en C₁-C₂, amino, (di)alkylamino en C₁-C₂ ; un radical alkylcarbonyle (R-CO-) dans lequel le radical R représente un radical alkyle en C₁-C₄ ; un groupement carbamoyle ((R)₂N-CO-) dans lequel les radicaux R indépendamment les uns des autres, représentent un atome d'hydrogène, un radical alkyle en C₁-C₄ ; une radical alkylsulfonyle (R-SO₂-) dans lequel le radical R représente un radical alkyle en C₁-C₄.

Selon un mode de réalisation particulièrement préféré, les groupes R₁ sont identiques et représentent préférentiellement un radical méthyle, éthyle, 2-hydroxyéthyle, 2-méthoxyéthyle, acétyle (CH³CO-), méthylsulfonyle (CH₃SO₂-).

Selon une autre variante préférée, R₁ et R₂ forment avec l'atome d'azote substitué par R₁, un hétérocycle saturé ou insaturé à 5 ou 6 chaînons substitué par un ou plusieurs radicaux alkyle.

R₁ et R₃ représentent préférentiellement et indépendamment l'un de l'autre :
- un atome d'hydrogène ;
- un atome d'halogène choisi parmi le brome, le chlore ou le fluor ;
- un radical alkyle en C₁-C₄, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, thio (-SH), alkyl(C₁-C₄)sulfinyl, alkyl(C₁-C₄)sulfonyle, thioalkyle(C₁-C₄)
- un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, ou un atome d'halogène tel que le chlore, le fluor ou le brome ;
- un radical alcoxy en C₁-C₄ ;
- un radical alkyl(en C₁-C₄)sulfonylamino ;
- un radical (poly)-hydroxyalcoxy en C₂-C₄ ;
- un radical amino ;
- un radical (di)alkylamino en C₁-C₂ ;
- un radical (poly)-hydroxyalkylamino en C₅-C₄ ;
- un radical alkylcarbonyle (R-CO-) dans lequel le radical R représente un radical alkyle en C₁-C₄ ;
- un radical carbamoyle ((R)₂N-CO-) dans lequel les radicaux R indépendamment les uns des autres, représentent un atome d'hydrogène, un radical alkyle en C₁-C₄ ;
- un radical alkylsulfonylamino (RSO₂N-) dans lequel le radical R représente un radical alkyle en C₁-C₄ ;
- un radical aminosulfonyl ((R)₂NSO₂-) dans lequel les radicaux R indépendamment les uns des autres représentent un atome d'hydrogène, un radical alkyle en C₁-C₄ ;
- un radical alkylthio (RS-) dans lequel le radical R représente un radical alkyle en C₁-C₄.
- un radical thio (HS-)
- un radical alkylsulfinyle (RSO-) dans lequel le radical R représente un radical alkyle en C₁-C₄.
- un radical alkylsulfonyle (R-SO₂-) dans lequel le radical R représente un radical alkyle en C₁-C₄.
- un radical alkylcarbonylamino (RCONR'-) dans lequel le radical R représente un atome d'hydrogène, un radical alkyle en C₁-C₄ et le radical R' représente un atome d'hydrogène, un radical alkyle en C₁-C₄.

Selon un mode de réalisation particulièrement préféré, les groupes R₂ et R₃ indépendamment l'un de l'autre représentent préférentiellement un hydrogène, un radical méthyle, éthyle, 2-hydroxyéthyle, 2-méthoxyéthyle, acétyle (CH₃CO-), méthylsulfonyle (CH₃SO₂-) amide (CH₃CONH-), hydroxyle, amino, méthylamino, diméthylamino, 2-hydroxyéthylamino, méthoxy, éthoxy, phényle.
- les groupes R₄, indépendamment les uns des autres, représentent de préférence :
   - un atome d'hydrogène ;
   - un radical alkyle en C₁-C₄, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, thio (-SH), alkyl(C₁-C₄)sulfinyle, alkyl(C₁-C₄)sulfonyle, thioalkyle(C₁-C₄)
   - un groupement amino, un groupement amino substitué par un ou plusieurs radicaux alkyle en C₁-C₄, identiques ou différents, éventuellement porteurs d'au moins un groupement hydroxyle, un groupement alkylcarbonylamino (RCO-NR-) dans lequel les radicaux R indépendamment les uns des autres représentent un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ; un groupement alkylsulfonylamino (RSO₂-NR'-) dans lequel les radicaux R, R', indépendamment les uns des autres, représentent un atome d'hydrogène, un radical alkyle en C₁-C₄ ;
   - un radical phényle éventuellement éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, thio (-SH), alkyl(C₁-C₄)sulfinyl, alkyl(C₁-C₄)sulfonyle, thioalkyle(C₁-C₄).

De façon encore plus préférée, R₄ représente un hydrogène, un radical méthyle, éthyle, hydroxyéthyle ; un radical amino, un groupement alkylcarbonylamino (RCO-NR-) ; un groupement uréido (N(R)₂-CO-NR'-) dans lequel les radicaux R et R', indépendamment les uns des autres, représentent un atome d'hydrogène, un radical alkyle en C₁-C₄ ; un groupement alkylsulfonylamino (RSO₂-NR'-) dans lequel les radicaux R, R', indépendamment les uns des autres, représentent un atome d'hydrogène, un radical alkyle en C₁-C₄ ; un radical phényle éventuellement substitué par au moins un groupement hydroxy.

De préférence, les composés de formule (I) selon la présente invention sont tels que les groupes R₅ sont identiques et représentent préférentiellement un radical alkyle en C₁-C₄, éventuellement substitué par un ou plusieurs radicaux choisis parmi les radicaux hydroxy, alcoxy en C₁-C₂, amino, (di)alkylamino en C₁-C₂ ; un radical phényle éventuellement substitué.

Selon un mode de réalisation particulièrement préféré, les groupes R₅ sont identiques et représentent préférentiellement un radical méthyle, éthyle, 2-hydroxyéthyle, 2-méthoxyéthyle, phényle.

Selon un mode de réalisation particulier, dans les formules (la) et (Ic) n'est égal à 4 et chacun des groupes de deux radicaux R₃ adjacents forme un radical aromatique, de préférence benzénique.

Selon une autre variante, dans les formules (Ia), (Ib), (Ic), (Ie) et (If) n'est égal à 2, et les deux groupes R₃ sont adjacents et forment un radical aromatique, de préférence benzénique.

De préférence, le bras de liaison L représente un radical alkylène en C₁-C₁₀, linéaire ou ramifié, éventuellement interrompu par un ou plusieurs hétéroatomes, choisis parmi l'oxygène, l'azote, le soufre, éventuellement substitué par un ou plusieurs radicaux choisis parmi les radicaux hydroxy, alcoxy en C₁-C₂, dialkylamino en C₁-C₂, alkylecarbonyle (R-CO-) dans lequel le radical R représente un radical alkyle en C₁-C₄, un groupement carbamoyle ((R)₂N-CO-) dans lequel les radicaux R indépendamment les uns des autres, représentent un atome d'hydrogène, un radical alkylsulfonyle (R-SO₂-) dans lequel le radical R représente un radical alkyle en C₁-C₄.

De manière plus préférée, le bras de liaison L représente un radical alkylène en C₁-C₁₀ de préférence linéaire éventuellement interrompu par un ou plusieurs hétéroatomes choisi parmi l'oxygène, le soufre, éventuellement substitué par un ou plusieurs radicaux choisis parmi les radicaux hydroxy, alcoxy en C₁-C₂, un radical alkylsulfonyle (R-SO₂-) dans lequel le radical R représente un radical alkyle en C₁-C₄.

Préférentiellement X- représente Br-, Cl-, l'acétate, le mésylate.

Selon un mode de réalisation particulier, les composés de formule (I) sont représentés par les formules suivantes : 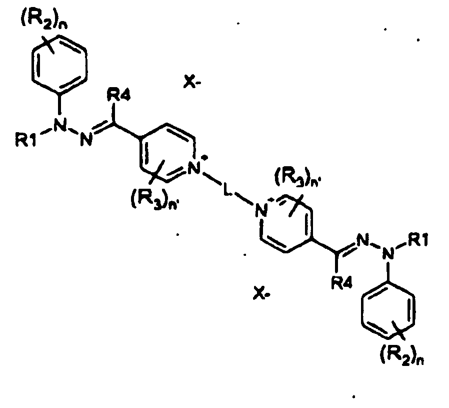 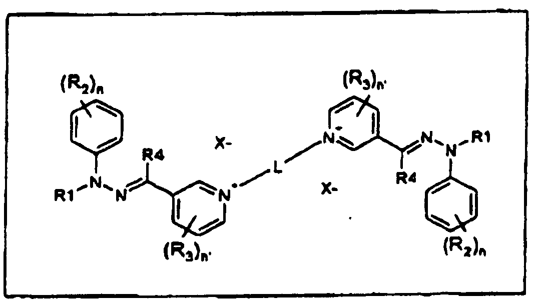 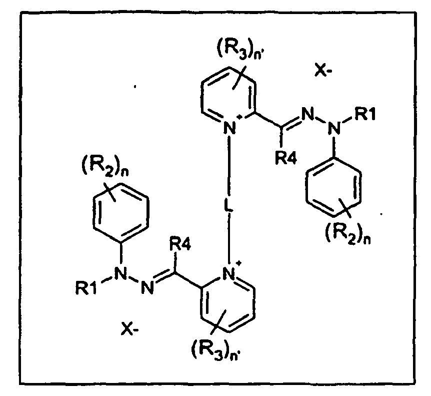 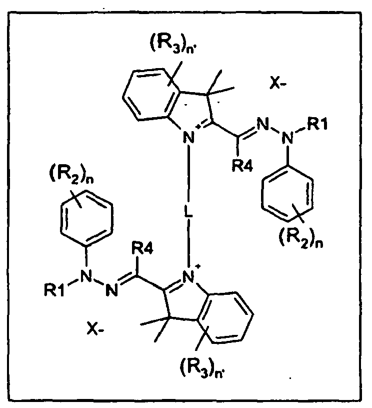 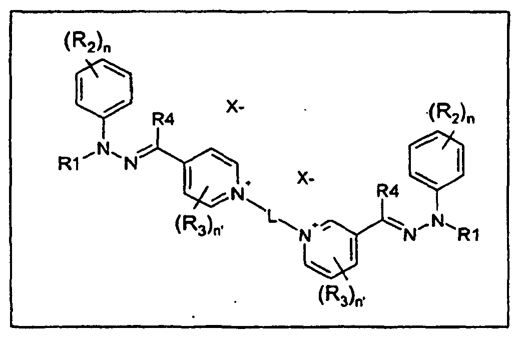 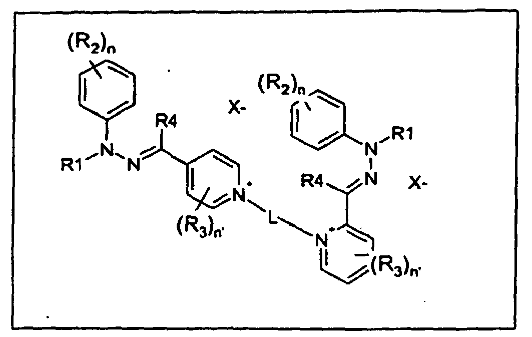 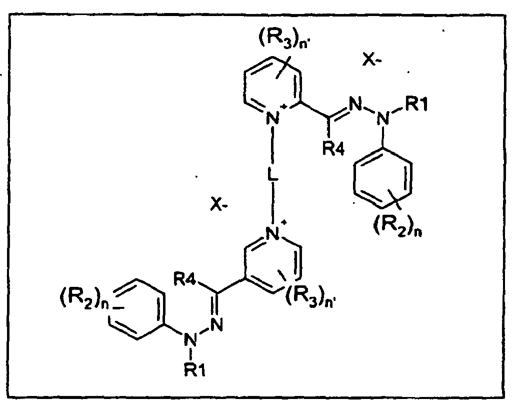 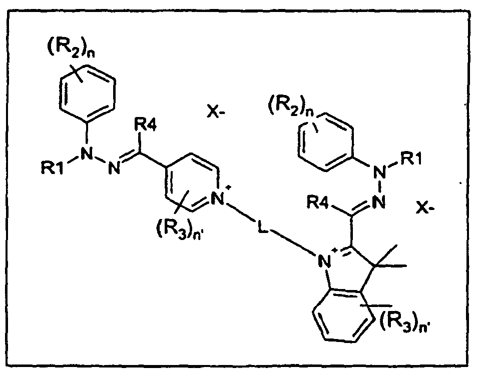 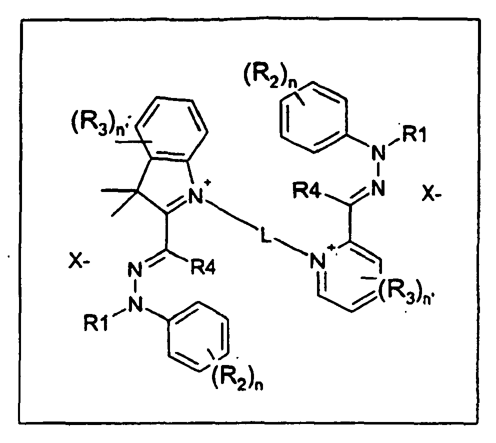 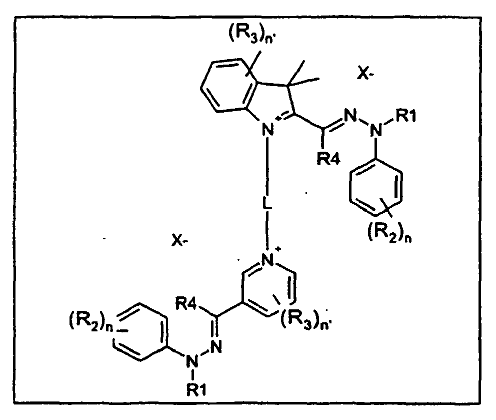 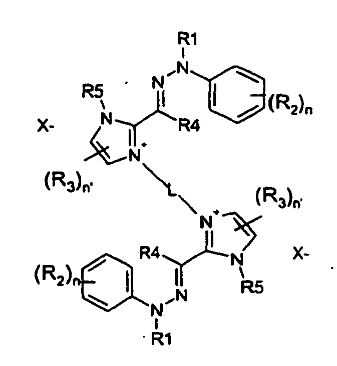 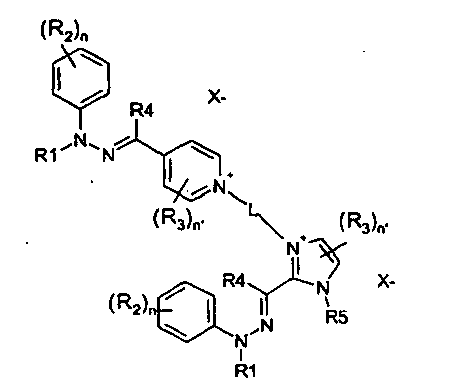 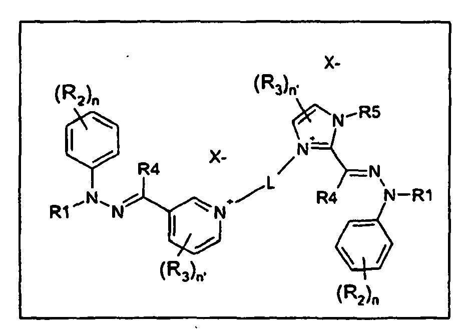 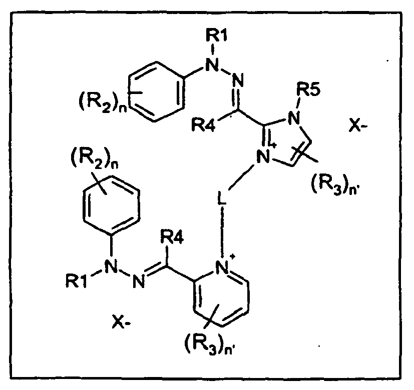 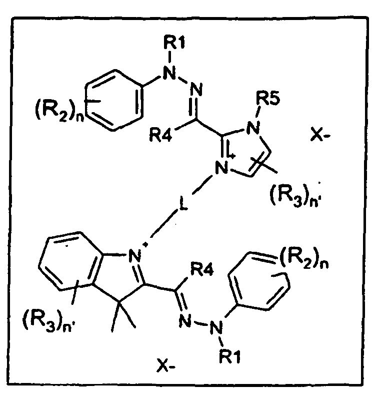 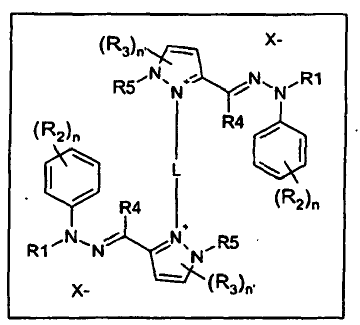 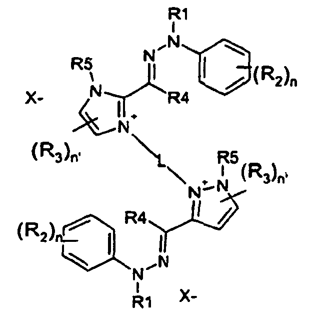 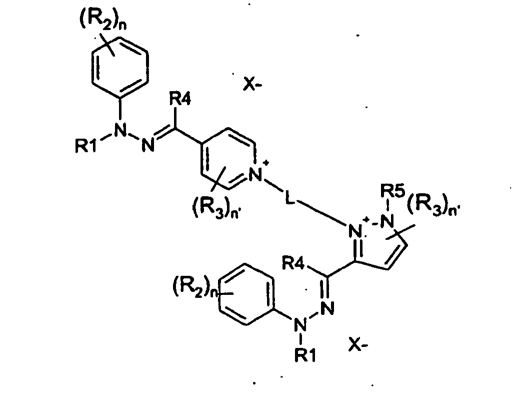 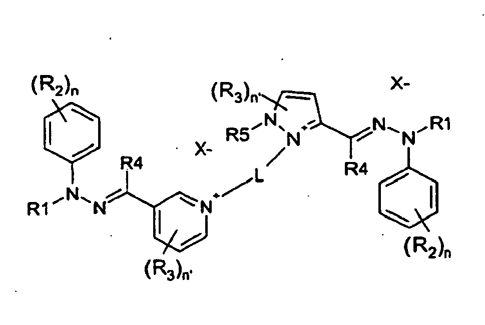 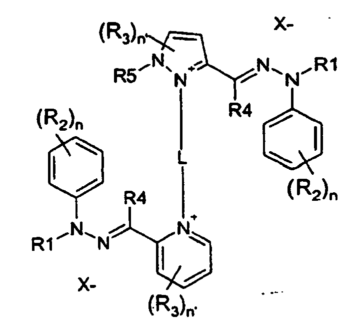 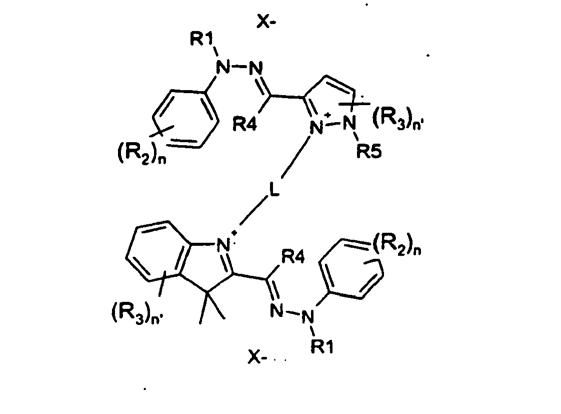 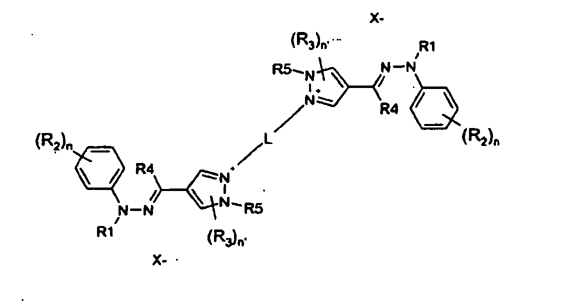 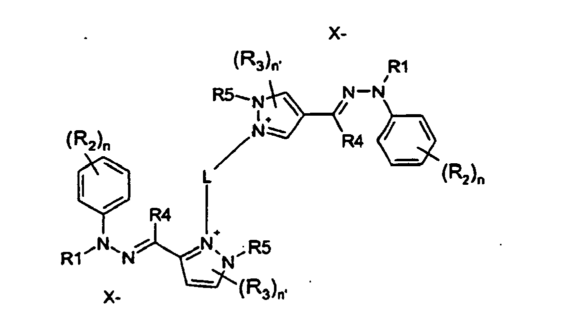 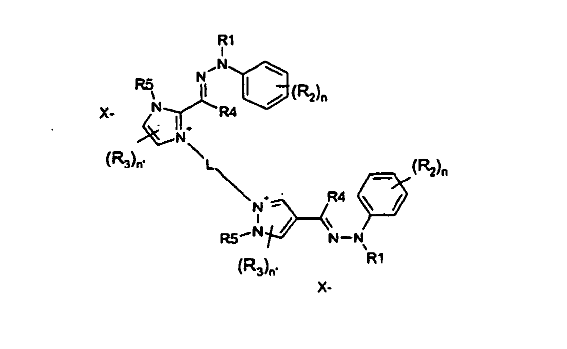 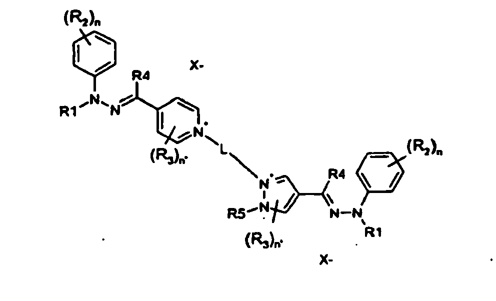 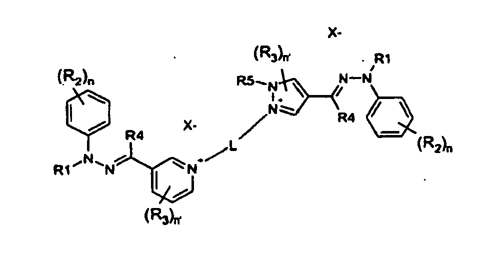 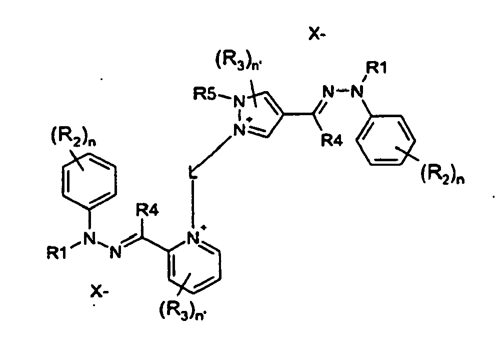 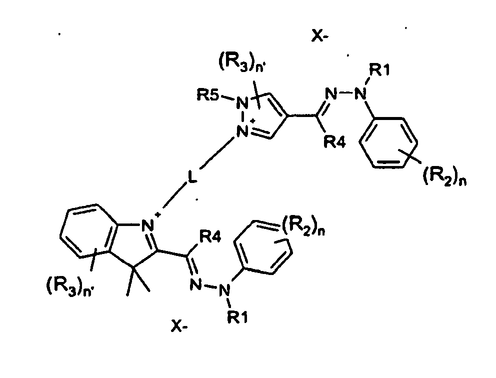 dans lesquelles les radicaux R₁, R₂, R₃, R₄, R₅, X, n, n'et L sont tels que définis précédemment.

De manière particulière, les composés de type bis hydrazone dicationique de formule (I) selon la présente invention sont représentés par les composés suivants : 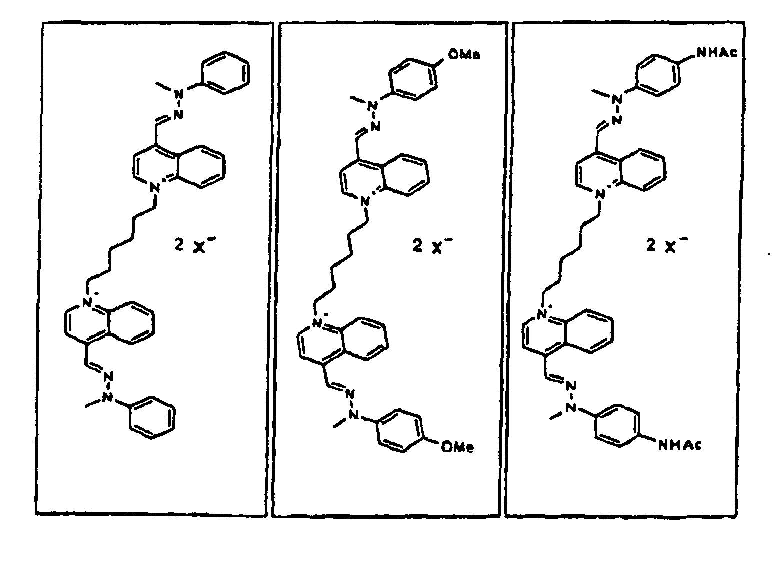 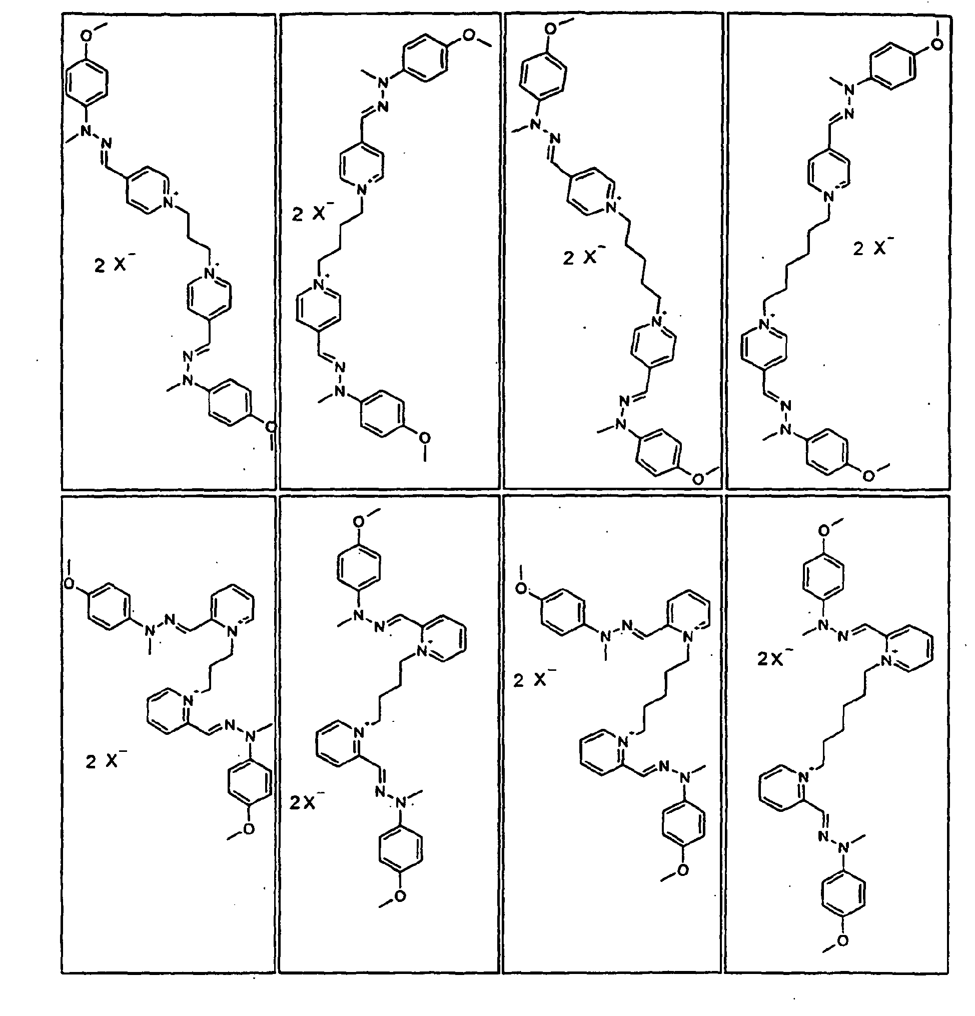 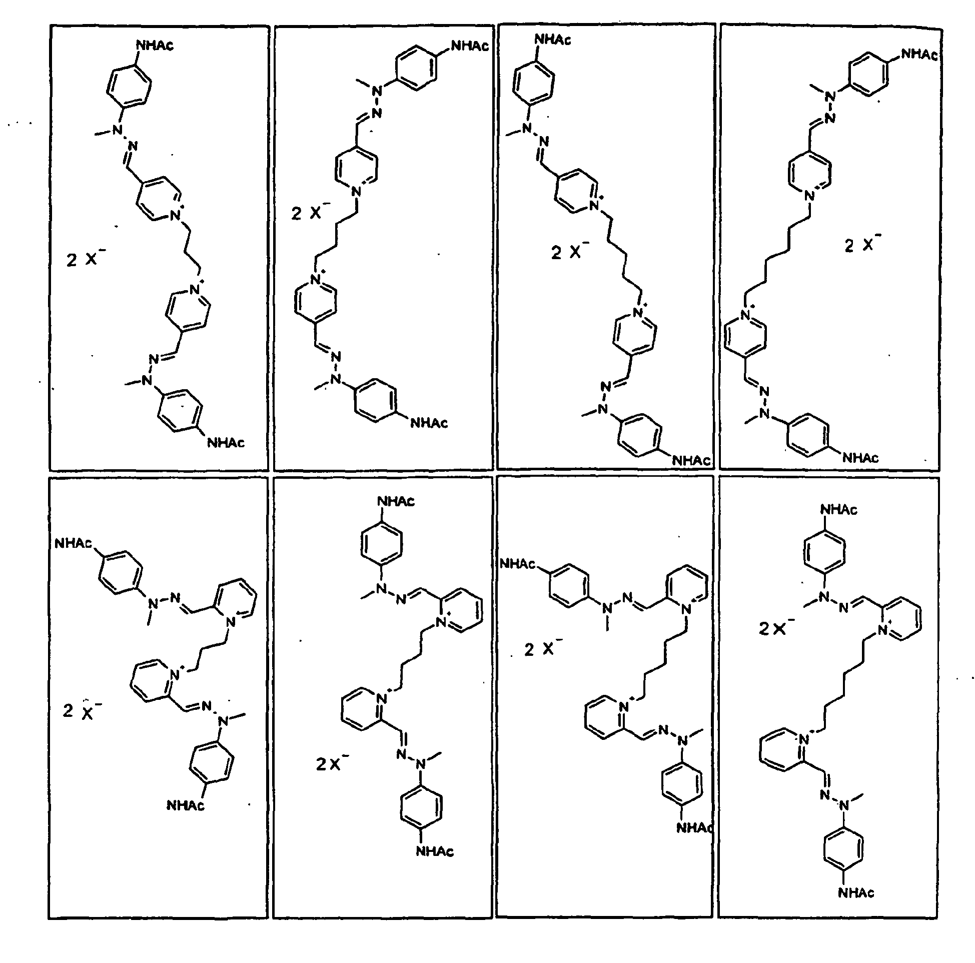 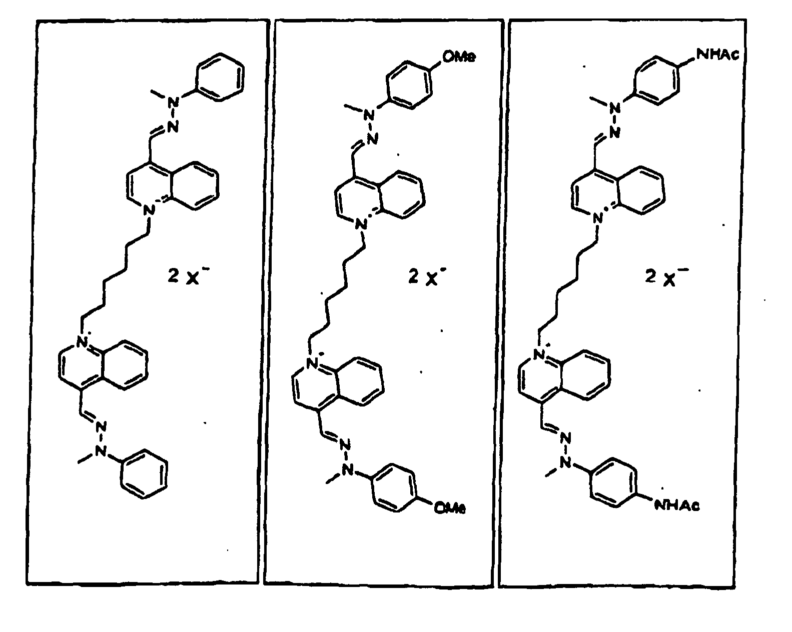 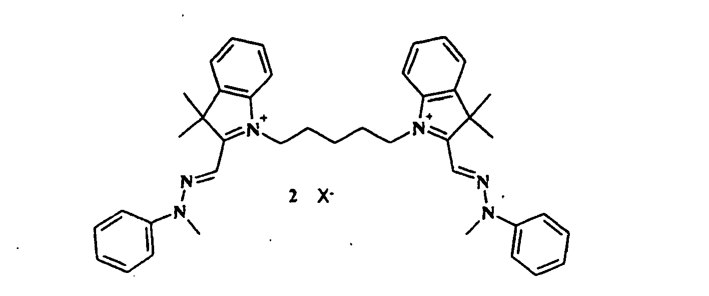 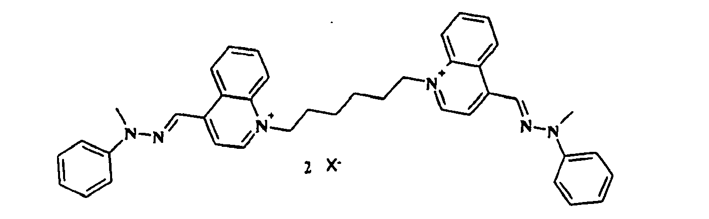 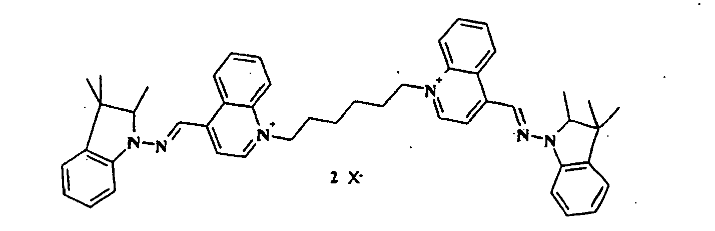 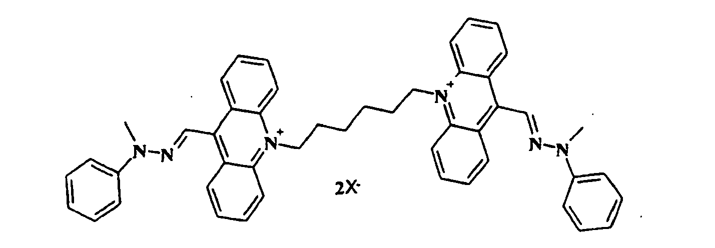 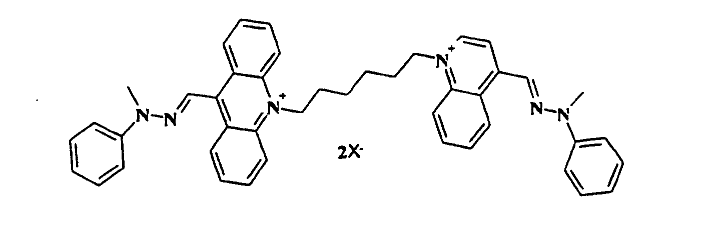 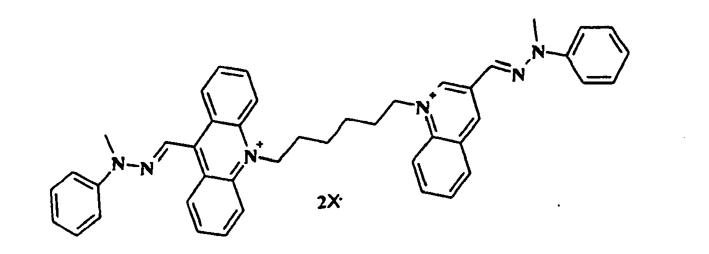 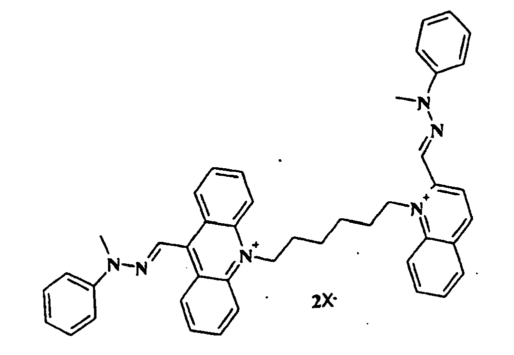 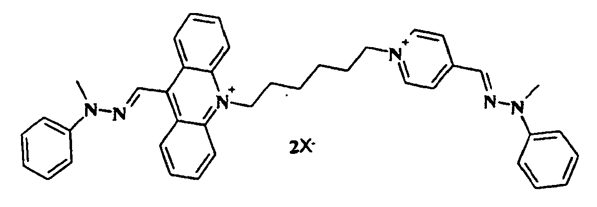 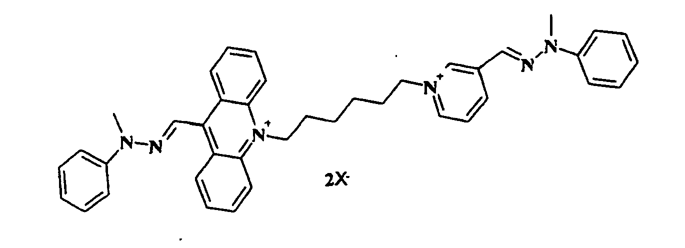 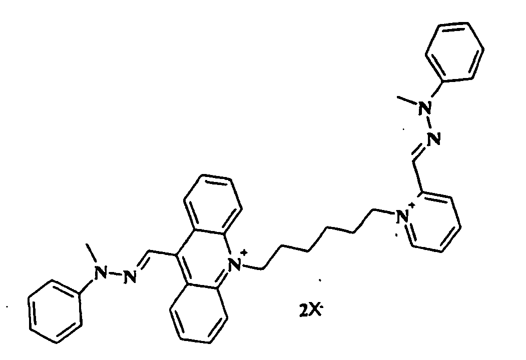 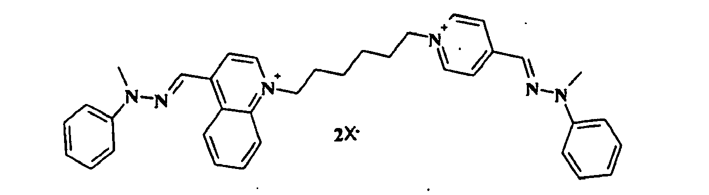 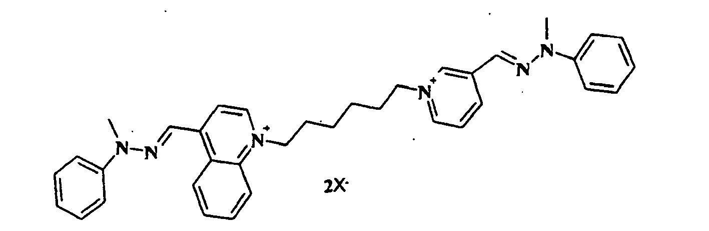 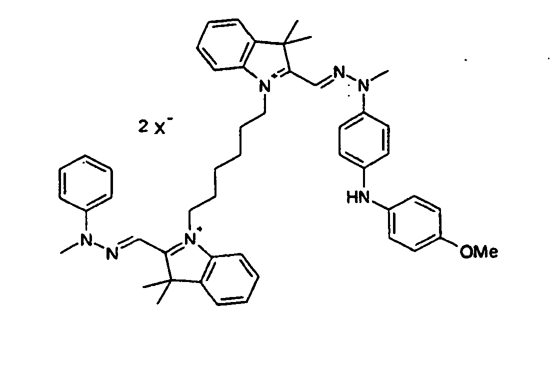 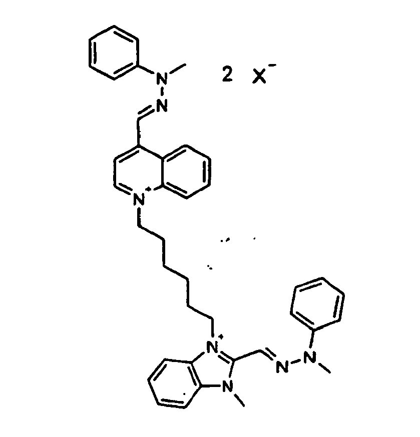 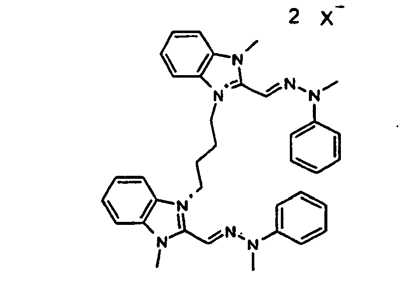 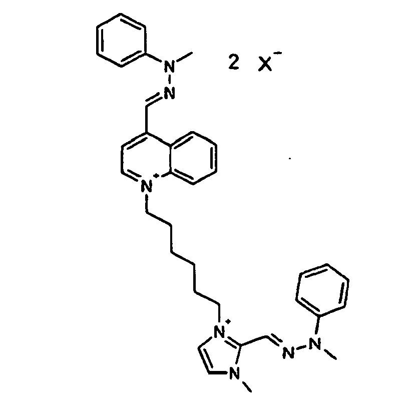 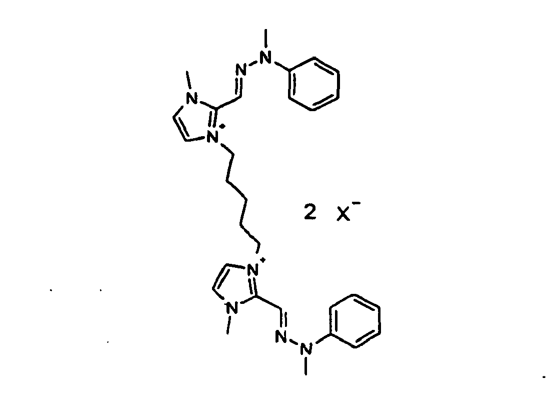 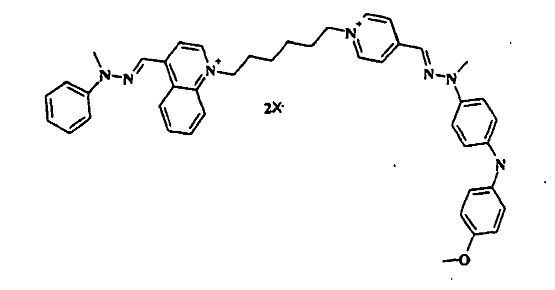 X- représente de préférence Cl- ou Br-

La composition selon la présente demande comprend de 0,001 à 10%, de préférence 0,01 à 10% en poids de composés de type bis-hydrazones dicationiques de formule (I) par rapport au poids total de la composition.

La composition selon l'invention peut contenir un ou plusieurs composés de type bis-hydrazones dicationiques de formule (I), les mélanges de ces composés étant alors possibles en toutes proportions relatives.

La composition tinctoriale conforme à l'invention peut en outre contenir un ou plusieurs colorants directs additionnels autres que les colorants directs de type bis-hydrazone dicationiques de formule (Ia) et/ou (Ib), et/ou (Ic), et/ou (Id) et/ou (Ie) et/ou (If) et/ou (Ig) pouvant notamment être choisis parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques, les colorants de type tétraazapentaméthinique et les colorants directs naturels.

Parmi les colorants directs benzéniques utilisables selon l'invention, on peut citer de manière non limitative les composés suivants :
- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-Bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1 -Hydroxy-2-chloro-6-éthylamino-4-nitrobenzène
- 1 -Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoïques cationiques décrits dans les demandes de brevets WO 95/15144, WO-95/01772 et EP-714954 dont le contenu fait partie intégrante de l'invention.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An représentant en général un anion organique ou minéral par exemple choisi parmi un halogénure tel que chlorure, bromure, fluorure, iodure ; un hydroxyde ; un sulfate ; un hydrogénosulfate ; un alkyl(C1-C6)sulfate tel que par exemple un méthylsulfate ou un éthylsulfate ; un acétate ; un tartrate ; un oxalate ; un alkyl(C1-C6)sulfonate tel que méthylsulfonate ; un arylsulfonate substitué ou non substitué par un radical alkyle en C1-C4 tel que par exemple un 4-toluylsulfonate. An est de préférence un chlorure ; un méthylsulfate :

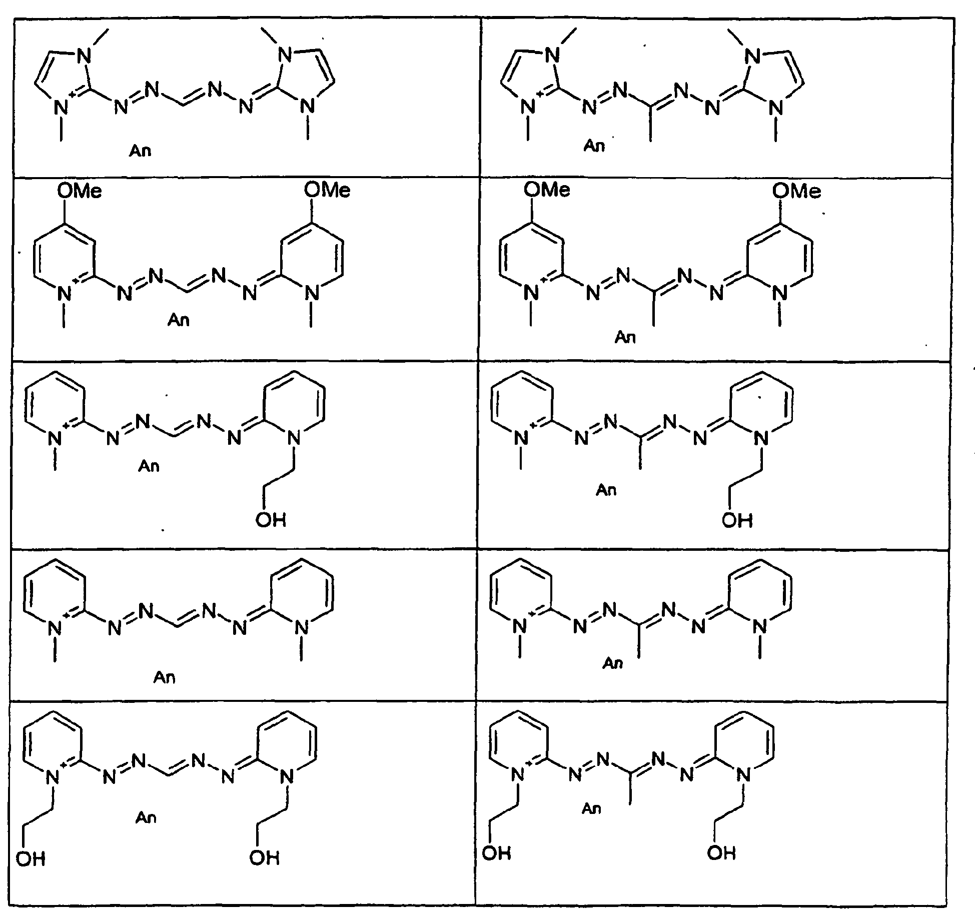

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition de la présente invention peut en outre comprendre un ou plusieurs précurseurs de colorant d'oxydation : une ou plusieurs bases d'oxydation et/ou un ou plusieurs coupleurs.

A titre d'exemple, les bases d'oxydation sont choisies parmi les phénylènediamines, les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraniéthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylarnine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation additionnelles présentes dans la composition de l'invention sont en général présentes en quantité comprise allant de 0,001 à 20 % en poids environ du poids total de la composition tinctoriale, de préférence allant de 0,005 à 6 %.

La composition selon l'invention contient de préférence un ou plusieurs coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en quantité comprise allant de 0,001 à 20 % en poids environ du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates; les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 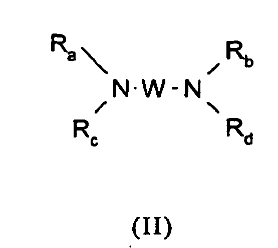 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment.

Selon un mode de réalisation particulier, la composition de l'invention est appliquée sur les fibres kératiniques en présence d'un agent oxydant pendant un temps suffisant pour obtenir l'éclaircissement souhaité. L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention comprend au moins un précurseur de colorant d'oxydation.

Le mélange obtenu est ensuite appliqué sur les fibres kératiniques. Après un temps de pause compris entre 3 minutes et 1 heure et de préférence entre 5 et 50 minutes, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydoréductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Les exemples qui suivent servent à illustrer l'invention sans toutefois présenter un caractère limitatif.

### EXEMPLES

### Exemple 1 : Synthèse du dibromure de 4- {(E)-[methyl(phenyl)hydrazono]methyl}-1-[3-(4-{(E)-[methyl(phenyl)hydrazono]methyl}pyridinium-1-yl)propyl]pyridinium

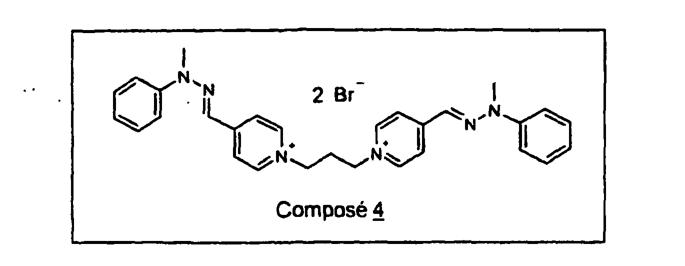

### Etape 1 : synthèse de l'isonicotinaldéhyde méthyl(phényl)hydrazone

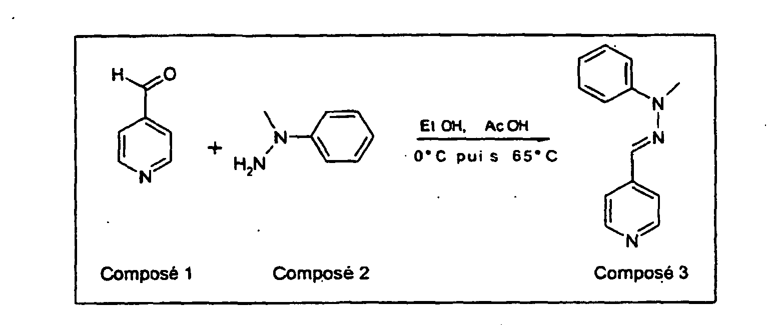

Dans un tricol surmonté d'un réfrigérant, on dilue un équivalent de méthylphénylhydrazine (58,76g ; 0,4809 mol, composé 2) dans 50ml d'éthanol et 1,5ml d'acide acétique. Le milieu réactionnel est alors mis sous agitation à 0°C. A l'aide d'une ampoule à brome, on ajoute 1 équivalent de 4-pyridinecarboxaldéhyde (51,5g ; 0,4808 mol, composé 1). Après ajout de l'aldéhyde, on chauffe le milieu réactionnel à 65°C (température du bain d'huile) pendant 13 heures.

Par la suite, le milieu réactionnel (huile foncée) est versé dans un mélange eau-glace (1/3). On place alors le mélange sous agitation. Le produit huileux finit par durcir jusqu'à l'obtention d'une poudre jaune. Celle-ci est alors filtrée puis lavée plusieurs fois à l'eau. Après séchage, on obtient 83,9g du composé 3.

### Données analytiques :

Poudre jaune.
RMN(¹H, MeOD, 400 MHZ) : 3,45 ppm, 3H, s ; 6,94-6,98 ppm, 1H, m ; 7,32-7,35 ppm, 2H, m ; 7,44-7,47 ppm, 2H, m ; 7,60-7,63 ppm, 3H, m ; 8,53-8,55 ppm, 2H, m
RMNe³C, DMSO-d6, 100,62 MHz) : 35,59 ppm ; 115,62 ppm ; 120,29 ppm ; 121,46 ppm ; 129,45 ppm ; 130,02 ppm ; 144,08 ppm ; 147,31 ppm ; 150,31 ppm
Masse (MS ES+) : m/z = 212

### Etape 2 :

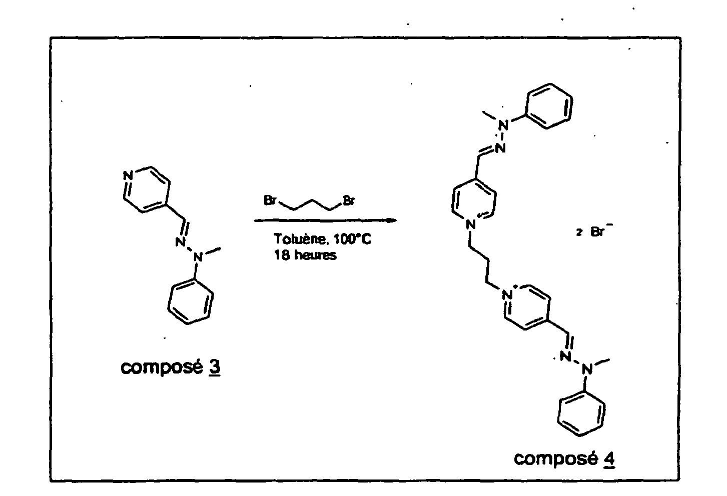

Dans un tricol surmonté d'un réfrigérant, on met sous agitation et à 100 °C pendant 18 heures, le composé 3 (10 g, 0.047 mole) en présence de 2.40 ml de 1,3-dibromopropane (0.023 mole) dans 200 ml de toluène.

Après réaction, le milieu réactionnel est refroidi à température ambiante et le produit souhaité, ayant précipité est filtré, puis nettoyé à l'aide d'acétate d'éthyle et enfin séché. Une reprécipitation est nécessaire et l'on procède comme suit : le brut réactionnel est solubilisé dans 150 ml de méthanol. Sur une période très courte, 550 ml d'acétate d'éthyle sont de nouveau ajoutés faisant précipiter le produit attendu. Celui-ci est filtré puis séché sous vide. On récupère 10.1 g de composé 4 (poudre jaune).

Analyses du produit :
**RMN (1H, 400 MHz, CD3OD) :**

| | | |
|---|---|---|
| 2,77 | m | CH2 |
| 3,6 | d | 2 x CH3 |
| 4,7 | t | 2 x CH2 |
| 7,15 | m | 2 H |
| 7,4 | m | 4 H |
| 7,51 | m | 4 H |
| 7,66 | se | 2 H |
| 8,1 | m | 4 H |
| 8,71 | m | 4 H |

**Masse (LC/MS ; ESI +) :** le dication attendu [C₂₉H₃₂N₆]²⁺ est détecté à m/z, ESI+=232.

### Exemple 2 : Synthèse du dibromure de 4-{(E)-[methyl(phenyl)hydrazono]methyl}-1-[4-(4-{(E)-[methyl(phenyl)hydrazono]methyl}pyridinium-1-yl)butyl]pyridinium

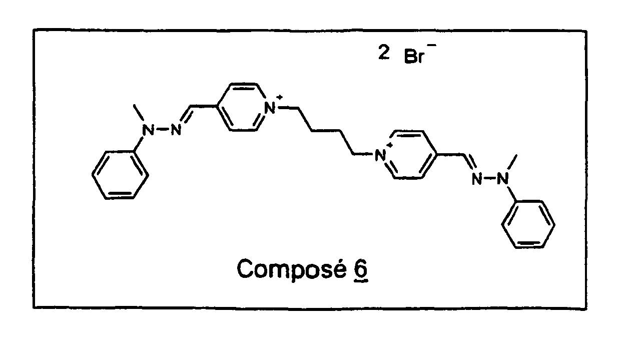

### Synthèse du composé 6 :

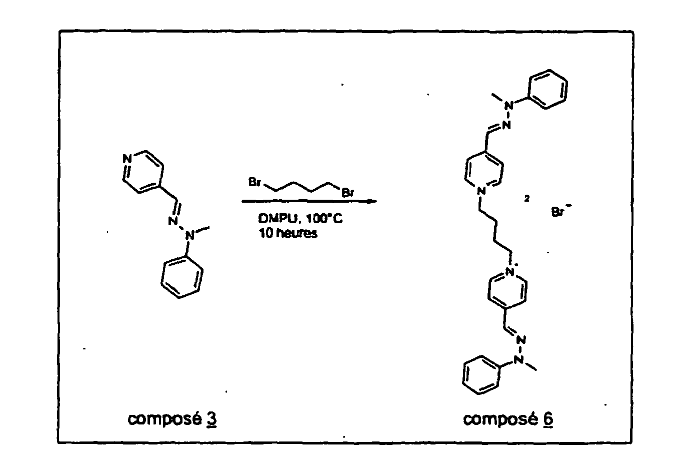

Dans un tricol surmonté d'un réfrigérant, on met sous agitation et à 100 °C pendant 10 heures, le composé 3 (10 g, 0.047 mole) en présence de 2.82 ml de 1,4-dibromobutane (0.023 mole) dans 200 ml de 1,3-diméthyle-3,4,5,6-tétrahydro-2(1H)-pyrimidinone

Après réaction, le milieu réactionnel est refroidi à température ambiante et le produit souhaité, ayant précipité est filtré, puis nettoyé à l'aide d'acétate d'éthyle et enfin séché. Une reprécipitation est nécessaire et l'on procède comme suit : le brut réactionnel est solubilisé dans 150 ml de méthanol. Sur une période très courte, 550 ml d'acétate d'éthyle sont de nouveau ajoutés faisant précipiter le produit attendu. Celui-ci est filtré puis séché sous vide. On récupère 12.4 g de composé **6** (poudre jaune).

Analyses du produit :
**RMN (1H, 400 MHz, CD3OD) :**

| | | |
|---|---|---|
| 2,15 | m | 2 CH2 |
| 3,65 | m | 2 CH3 |
| 4,58 | m | 2 CH2 |
| 7,17 | m | 2 H |
| 7,43 | m | 4 H |
| 7,58 | m | 4 H |
| 7,72 | se | 2 H |
| 8,15 | m | 4 H |
| 8,71 | m | 4 H |

Masse **(ESI** +) :le dication attendu (C₃₀H₃₄N₆]²⁺ est détecté à m/z, ESI+=239.

### Exemple 3 : Synthèse du dibromure de 4-{(E)-[methyl(phenyl)hydrazono]methyl}-1-[5-(4-{(E)-[methyl(phenyl)hydrazono]methyl}pyridinium-1-yl)pentyl]pyridinium

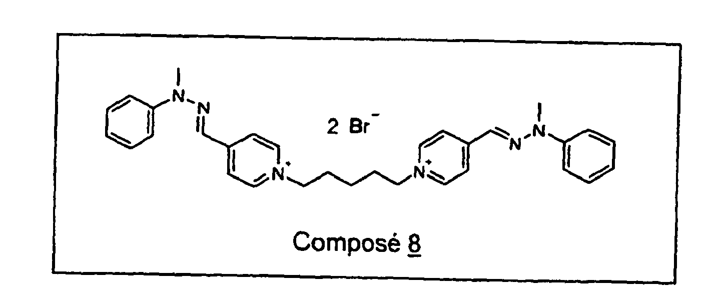

### Synthèse du composé 8 :

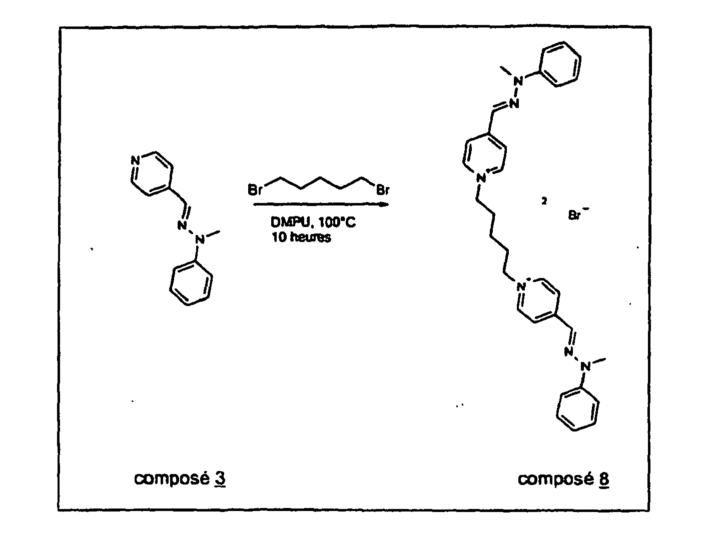

Dans un tricol surmonté d'un réfrigérant, on met sous agitation et à 100 °C pendant 10 heures, le composé 3 (10 g, 0.047 mole) en présence de 3.22 ml de 1,5-dibromopentane (0.023 mole) dans 100 ml de 1,3-diméthyle-3,4,5,6-tétrahydro-2(1H)-pyrimidinone

Après réaction, le milieu réactionnel est refroidi à température ambiante et le produit souhaité, précipité en présence de 20 ml d'acétate d'éthyle puis filtré et enfin séché. Une reprécipitation est nécessaire et l'on procède comme suit : le brut réactionnel est solubilisé dans 150 ml de méthanol. Sur une période très courte, 550 ml d'acétate d'éthyle sont de nouveau ajoutés faisant précipiter le produit attendu. Celui-ci est filtré puis séché sous vide. On récupère 12.4 g de composé 8 (poudre jaune).
Analyses du produit :
**RMN (1H, 400 MHz, CD₃OD) :**

| | | |
|---|---|---|
| 1,5 | m | CH2 |
| 2,09 | m | 2 CH2 |
| 3,61 | d | 2 CH3 |
| 4,52 | t | 2 CH2 |
| 7,14 | m | 2 H |
| 7,41 | m | 4 H |
| 7,53 | m | 4 H |
| 7,69 | se | 2 H |
| 8,13 | d | 4 H |
| 8,71 | d | 4 H |

**Masse (ESI +) :** le dication attendu [C₃₁H₃₆N₆]²⁺ est détecté à m/z, ESI+=246.

### Exemple 4 : Synthèse du dibromure de 4-{(E)-[methyl(phenyl)hydrazono]methyl}-1-[6-(4-{(E)-[methyl(phenyl)hydrazono]methyl}pyridiriium-1-yl)hexyl]pyridinium

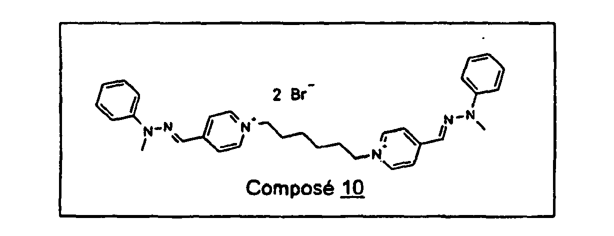

### Synthèse du composé 10 :

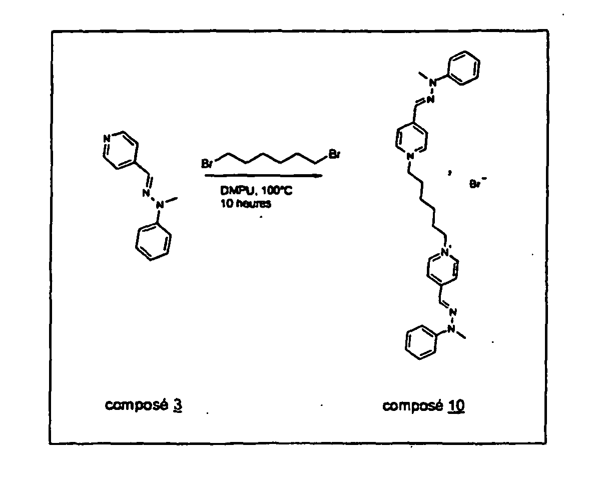

Dans un tricol surmonté d'un réfrigérant, on met sous agitation et à 100 °C pendant 10 heures, le composé 3 (10 g, 0.047 mole) en présence de 3.61 ml de 1,6-dibromohexane (0.023 mole) dans 100 ml de 1,3-diméthyle-3,4,5,6-tétrahydro-2(1H)-pyrimidinone

Après réaction, le milieu réactionnel est refroidi à température ambiante et le produit souhaité, précipité en présence de 20 ml d'acétate d'éthyle puis filtré et enfin séché. Une reprécipitation est nécessaire et l'on procède comme suit : le brut réactionnel est solubilisé dans 150 ml de méthanol. Sur une période très courte, 550 ml d'acétate d'éthyle sont de nouveau ajoutés faisant précipiter le produit attendu. Celui-ci est filtré puis séché sous vide. On récupère 11.2 g de composé **10** (poudre jaune).
**Données analytiques** :
**RMN (1H, 400 MHz, CD₃OD) :**

| | | |
|---|---|---|
| 1,51 | m | 2 x CH2 |
| 2,04 | m | 2 x CH2 |
| 3,63 | s | 2 x CH3 |
| 4,51 | t | 2 x CH2 |
| 7,14 | m | 2 H |
| 7,41 | m | 4 H |
| 7,55 | m | 4 H |
| 7,7 | s | 2 H |
| 8,13 | d | 4 H |
| 8,72 | d | 4 H |

**Masse (ESI +) :**
le dication attendu (C₃₂ H₃₈N₆)²⁺ est détecté à m/z=254.

### Exemple 5 (non conforme à l'invention): Synthèse du dibromure de 4-{(E)-[methyl(pbenyl)hydrazono]methyl}-1-(6-{[4-({([6-(4-{(E)-[methyl(phenyl)hydrazono]methyl}pyridinium-1-yl)hexyl]amino}carbonyl)benzoyl]amino}hexyl)pyridinium

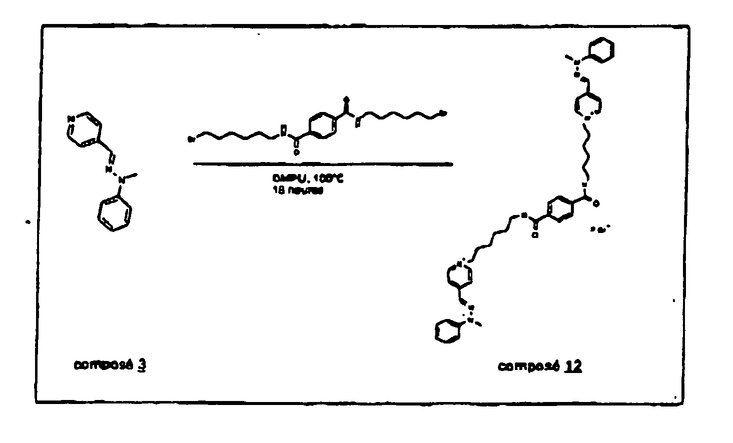

Dans un tricol surmonté d'un réfrigérant, on met sous agitation et à 100 °C pendant 18 heures, le composé **3** (10 g, 0.047 mole) en présence de 3.02 g de N,N"-bis(6-bromohexyl)terephthalamide (0.023 mole) dans 200 ml de 1,3-diméthyle-3,4,5,6-tétrahydro-2(1H)-pyrimidinone.

Après réaction, le milieu réactionnel est refroidi à température ambiante et le produit souhaité est précipité par ajout de 200 ml d'acétate d'éthyle. Le précipité est filtré, puis séché. Une reprécipitation est nécessaire et l'on procède comme suit : le brut réactionnel est solubilisé dans 150 ml de méthanol. Sur une période très courte, 550 ml d'acétate d'éthyle sont de nouveau ajoutés faisant précipiter le produit attendu. Celui-ci est filtré puis séché sous vide. On récupère 14.1 g de composé **12** (poudre jaune).
Analyses du produit :
**RMN (1H, 400 MHz, CD3OD) :**

| | | |
|---|---|---|
| 1,48 | m | 4 CH2 |
| 1,67 | m | 2 CH2 |
| 2,03 | m | 2 CH2 |
| 3,32 | d | 2 CH2 |
| 3,63 | s | 2 CH3 |
| 4,48 | t | 2 CH2 |
| 7,13 | m | 2 H |
| 7,4 | m | 4 H |
| 7,51 | m | 4 H |
| 7,66 | se | 2 H |
| 7,87 | s | 4 H |
| 8,09 | d | 4 H |
| 8,64 | d | 4 H |

**Masse (ESI +) :** Le dication [C₄₆H₅₆N₈O₂]²⁺ est détecté à m/z=376.

### Exemple 6 : Synthèse du dibromure de 4-{(E)-[methyl(phenyl)hydrazono]methyl}-1-[6-(4-{(E)-[methyl(phenyl)hydrazono]methyl}quinolinium-1-yl)hexyl]quinolinium

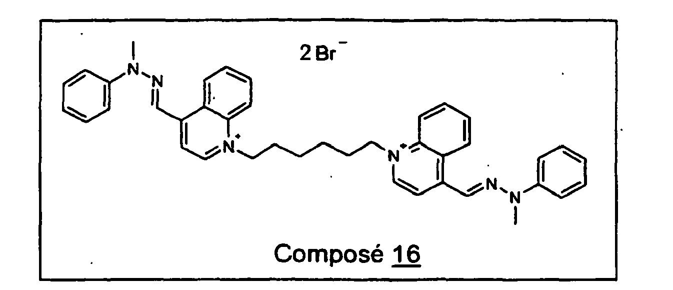

### Etape 1 : Synthèse de la quinoline-4-carbaldehyde methyl(phenyl)hydrazone

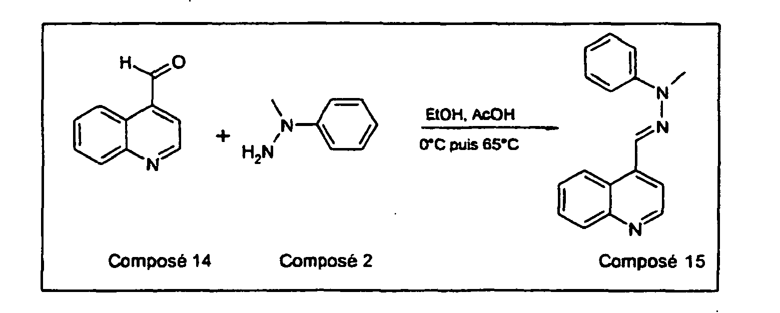

Dans un tricol surmonté d'un réfrigérant, on dilue un équivalent de méthylphénylhydrazine (4.1 g ; 0,033 mol, composé **2**) dans 25 ml d'éthanol et 0,5ml d'acide acétique glacial. Le milieu réactionnel est alors mis sous agitation à 0°C. On ajoute à la spatule 1 équivalent de 4-formylquinoline (5.28 g ; 0,033 mol, composé **14**). Après ajout de l'aldéhyde, on chauffe le milieu réactionnel à 70°C (température du bain d'huile) pendant 4 heures.

Par la suite, le milieu réactionnel (huile foncée) est versé dans un mélange eau-glace (1/3). On place alors le mélange sous agitation. Le produit huileux finit par durcir jusqu'à l'obtention d'une poudre jaune-orangée. Celle-ci est alors filtrée puis lavée plusieurs fois à l'eau. Après séchage, on obtient 7.9 g du composé **15**
**Données analytiques :**
**RMN (1H, 400 MHz, CD₃OD) :**

| | | |
|---|---|---|
| 3,58 | d | CH3 |
| 7,01 | m | 1 H |
| 7,36 | m | 2 H |
| 7,48 | m | 2 H |
| 7,65 | m | 1 H |
| 7,76 | m | 1 H |
| 7,92 | d | 1 H |
| 8,01 | m | 1 H |
| 8,16 | se | 1 H |
| 8,69 | d | 1 H |
| 8,75 | d | 1 H |

**Masse (MS ES+) :** L'ion quasi-moléculaire (MH)+ de la molécule attendue, C₁₇H₁₅N₃, est principalement détecté à m/z=262.

### Etape 2 : Synthèse du dibromure de 4-{(E)-[methyl(phenyl)hydrazono]methyl}-1-[6-(4-{(E)-[methyl(phenyl)hydrazono]methyl}quinolinium-1-yl)hexyl]quinolinium

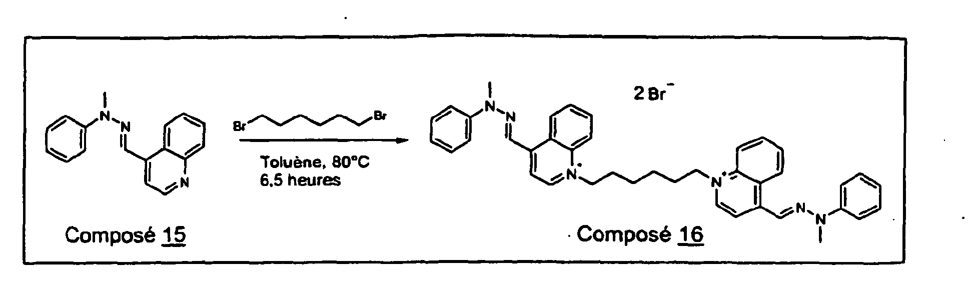

Dans un tricol surmonté d'un réfrigérant, on met sous agitation et à 80 °C pendant 6,5 heures, le composé 15 (1 g, 3,82 mmoles) en présence de 3,7 ml de 1,6-dibromohexane (23.96 mmoles) dans 23 ml de toluène.

Après réaction, le milieu réactionnel est refroidi à température ambiante puis versé sur de l'acétone (50 ml). Le précipité obtenu est filtré puis lavé plusieurs fois à l'acétone et enfin sous vide. On récupère 73 mg de composé **16** (poudre orange).

Analyses du produit :
**RMN (1H, 400 MHz, CD3OD) :**

| | | |
|---|---|---|
| 1,06 | m | 2 x CH2 |
| 1,92 | m | 2 x CH2 |
| 3,8 | s | 2 x CH3 |
| 4,92 | t | 2 x CH2 |
| 7,19 | m | 2 H |
| 7,46 | m | 4 H |
| 7,68 | m | 4 H |
| 7,99 | t | 2 H |
| 8,2 | t | 2 H |
| 8,39 | d | 2 H |
| 8,48 | m | 2 H |
| 8,49 | m | 2 H |
| 9,09 | m | 2 H |
| 9,13 | d | 2 H |

**Masse (LC/MS ; ESI +) :** Le dication attendu, (C₄₀H₄₂N₆)2+, est détecté à m/z=303.

### Exemple 7 : Synthese de dibromure de 4-{(E)-[methyl(phenyl)hydrazono]methyl}-1-[6-(4-{(E)-[methyl(phenyl)hydrazono]methyl}pyridinium-1-yl)hexyl]quinolinium

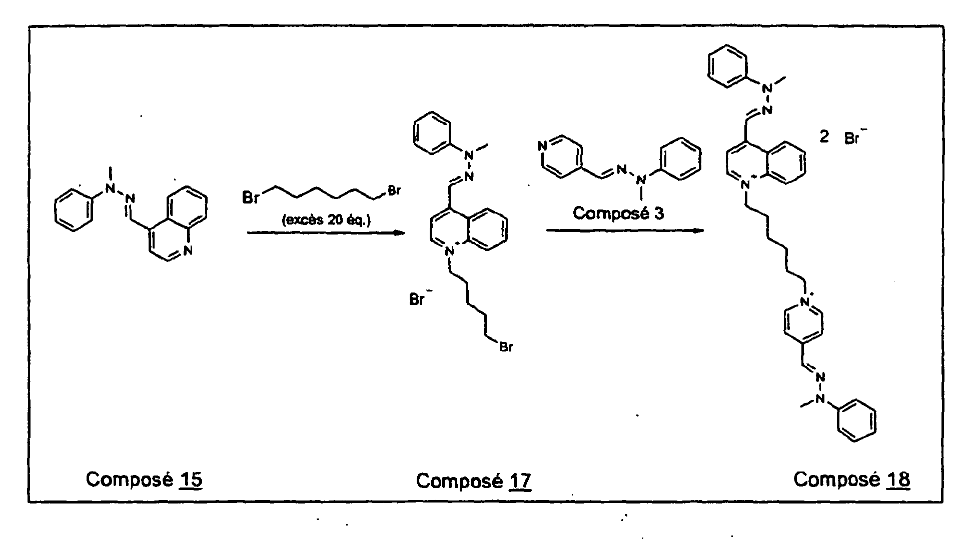

Dans un tricol surmonté d'un réfrigérant, on met sous agitation et à 80 °C pendant 6 heures, le composé 15 (5.83 g) en présence de 1,6-dibromohexane (70 ml).

Après réaction, un précipité orange est obtenu. Le milieu réactionnel est filtré à chaud sur fritté. Le précipité obtenu est lavé plusieurs fois au toluène puis à l'éther de pétrole et enfin séché sous vide. On récupère 7.47 g du composé 17 (poudre orange).

Dans un tricol, on solubilise 1.19 g (1 éq, 2.3 mmol) de composé 17 dans 5 ml d'éthanol. A l'aide d'une ampoule de coulée, on verse au milieu réactionnel sous agitation et préalablement chauffé à 50°C, 1 g (2 éq, 4.6 mmol) de composé 3 solubilisé dans 10 ml d'éthanol. Le milieu réactionnel est maintenu à 50°C pendant 48 heures.

Après réaction, le composé 18 est précipité en ajoutant 75 ml d'acétate d'éthyle froid. Le précipité obtenu est filtré puis rincé avec 25 ml d'acétate d'éthyle. On obtient 1.2 g d'une poudre rouge après séchage au dessiccateur sous vide.
**Masse (ESI** +**)** : le dication attendu est détecté à m/z = 278.

### Exemple 8 : Synthese de dibromure de 1-methyl-3-[5-(1-methyl-2-{(E)-[methyl(phenyl)hydrazono]methyl}-1H-imidazol-3-ium-3-yl)pentyl]-2-{(E)-[methyl(phenyl)hydrazono]methyl}-1H-imidazol-3-ium

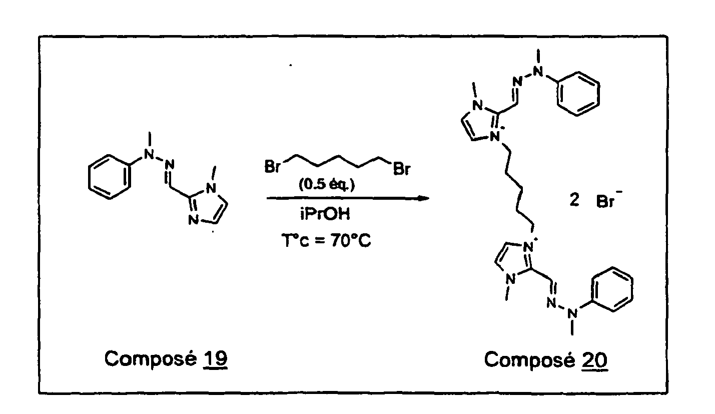

Dans un tricol surmonté d'un réfrigérant, on met sous agitation et à 70 °C pendant 48 heures, le composé 19 (0.15 g) en présence de 1,5-dibromopentane (80 mg) et de 1 ml d'isopropanol.

Après réaction, le composé 20 est précipité en ajoutant 5 ml d'acétate d'éthyle froid. Le précipité obtenu est filtré puis rincé avec 2 x 5 ml d'acétate d'éthyle. On obtient 90 mg d'une poudre jaune après séchage au dessiccateur sous vide.

**Masse (ESI +)** : le dication attendu est détecté à m/z = 249.

### Exemple 9 : Synthese de dibromure de 1-methyl-3-[4-(1-methyl-2-{(E)-[methyl(phenyl)hydrazono]methyl}-1H-benzimidazol-3-ium-3-yl)butyl]-2-{(E)-[methyl(phenyl)hydrazono]methyl}-1H-benzimidazol-3-ium.

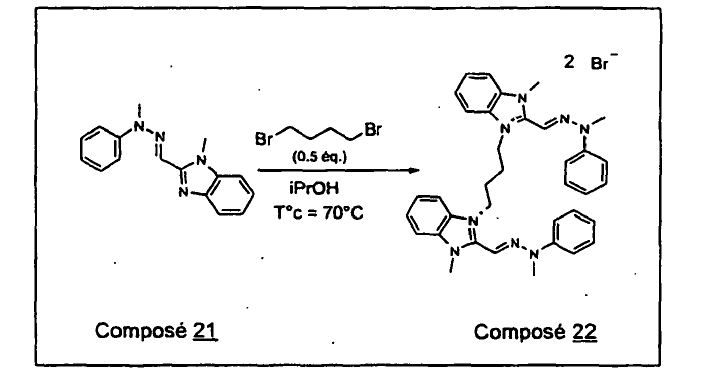

Dans un tricol surmonté d'un réfrigérant, on met sous agitation et à 70 °C pendant 48 heures, le composé 21 (0.15 g) en présence de 1,4-dibromobutane (61 mg) et de 1 ml d'isopropanol.

Après réaction, le composé 22 est précipité en ajoutant 5 ml d'acétate d'éthyle froid. Le précipité obtenu est filtré puis rincé avec 2 x 5 ml d'acétate d'éthyle. On obtient 77 mg d'une poudre jaune après séchage au dessicateur sous vide.
**Masse (ESI +)** : le dication attendu est détecté à m/z = 292.

### Exemple 10 : Synthese de dibromure de 1-[6-(1-methyl-2-{(E)-[methy](phenyl)hydrazono]methyl}-1H-imidazo]-3-ium-3-yl)hexyl]-4-{(E)-[methyl(phenyl)hydrazono]methyl}quinolinium

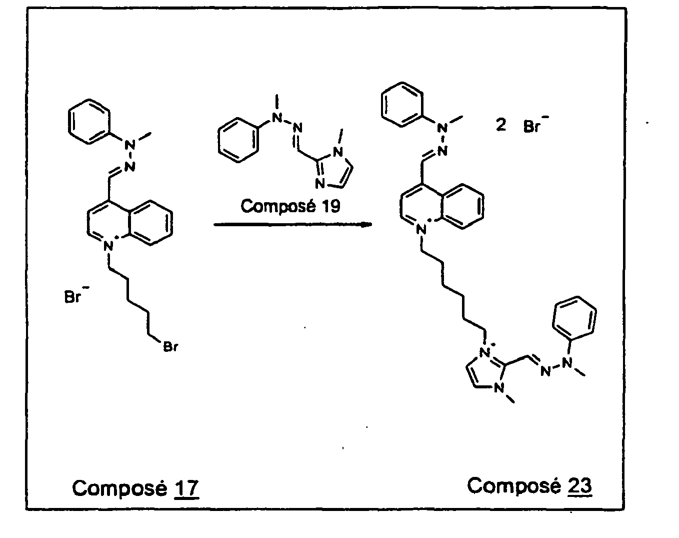

Dans un tricol surmonté d'un réfrigérant, on met sous agitation et à 70 °C pendant 48 heures, le composé 17 (0.26 g) en présence du composé 19 (120 mg) et de 2 ml d'isopropanol.

Après réaction, le composé 23 est précipité en ajoutant 5 ml d'acétate d'éthyle froid. Le précipité obtenu est filtré puis rincé avec 2 x 5 ml d'acétate d'éthyle. On obtient 125 mg d'une poudre jaune après séchage au dessicateur sous vide.
**Masse (ESI +) :** le dication attendu est détecté à m/z = 280.

### Exemple 11 : Synthese de dibromure 1-[6-(1-methyl-2-{(E)-[methyl(phenyl)hydrazono]methyl}-1H-benzimidazol-3-ium-3-yl)hexyl]-4-{(E)-[methyl(phenyl)hydrazono]methyl}quinolinium.

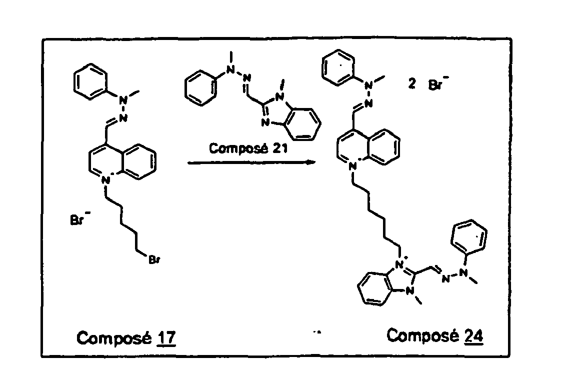

Dans un tricol surmonté d'un réfrigérant, on met sous agitation et à 70 °C pendant 48 heures, le composé 17 (0.21 g) en présence du composé 21 (120 mg) et de 2 ml d'isopropanol.

Après réaction, le composé 24 est précipité en ajoutant 5 ml d'acétate d'éthyle froid. Le précipité obtenu est filtré puis rincé avec 2 x 5 ml d'acétate d'éthyle. On obtient 111 mg d'une poudre jaune après séchage au dessiccateur sous vide.
**Masse (ESI +)** : le dication attendu est détecté à m/z = 305.

### Exemple 12 : Synthèse du dibromure de 1,1'-pentane-1,5-diylbis(4-{(E)-[methyl(phenyl)hydrazono]methyl}quinolinium).

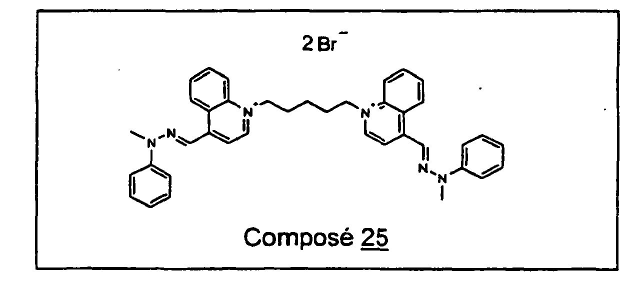

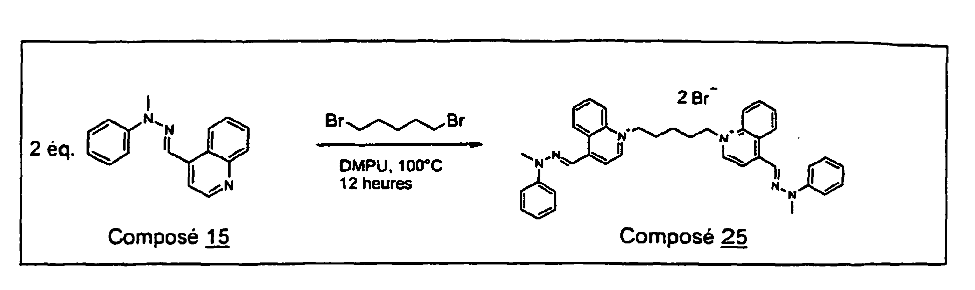

Dans un tricol surmonté d'un réfrigérant, on met sous agitation et à 100 °C pendant 12 heures, le composé **15** (1 g, 3,82 mmoles) en présence de 0,26 ml de 1,5-dibromohexane (1,91 mmoles) dans 20 ml de DMPU.

Après réaction, le milieu réactionnel est refroidi à température ambiante puis versé sur de l'acétone (50 ml) puis de l'acétate d'éthyle (50 ml). Le précipité obtenu est filtré puis lavé plusieurs fois à l'acétone et enfin séché sous vide. On récupère 750 mg de composé **25** (poudre orange).

Les analyses sont conformes au produit attendu.

### Exemple 13 : Synthèse du dibromure de 1,1'-butane-1,4-diylbis(4-{(E)[methyl(phenyl)hydrazono]methyl}quinolinium).

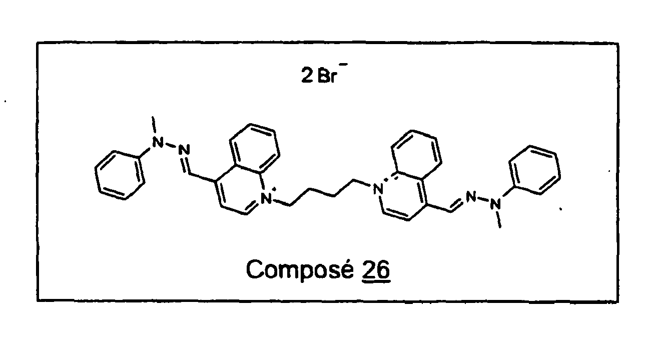

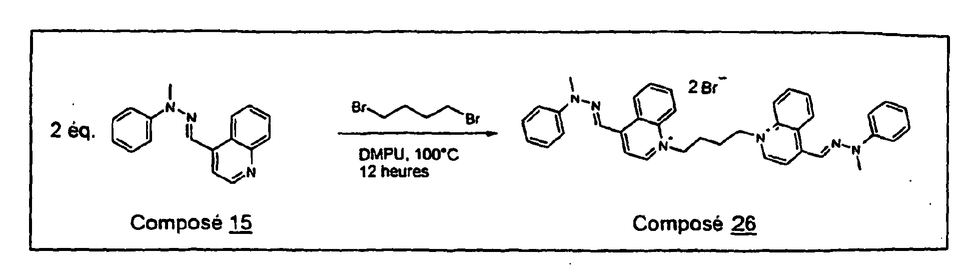

Dans un tricol surmonté d'un réfrigérant, on met sous agitation et à 100 °C pendant 12 heures, le composé **15** (1 g, 3,82 mmoles) en présence de 0,23 ml de 1,4-dibromobutane (1,91 mmoles) dans 20 ml de DMPU.

Après réaction, le milieu réactionnel est refroidi à température ambiante puis versé sur de l'acétone (50 ml) puis de l'acétate d'éthyle (50 ml). Le précipité obtenu est filtré puis lavé plusieurs fois à l'acétone et enfin séché sous vide. On récupère 655 mg de composé **26** (poudre orange).

Les analyses sont conformes au produit attendu.

### Exemple 14 : Synthèse du dibromure de 1,1'-propane-1,3-diylbis(4-{(E)-[methyl(phenyl)hydrazono]methyl}quinolinium)

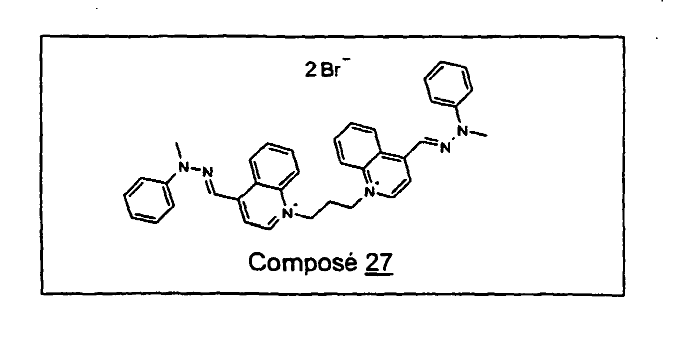

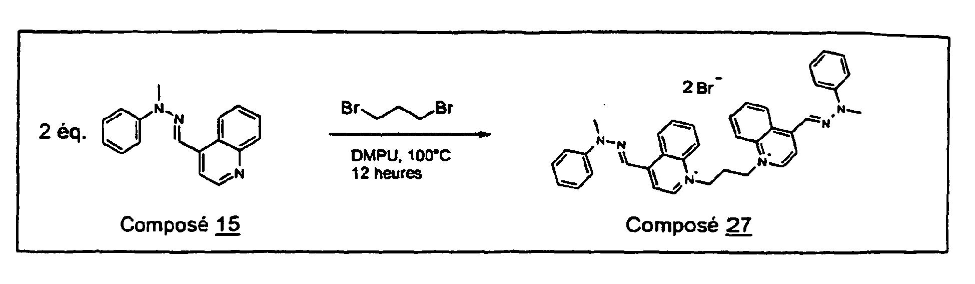

Dans un tricol surmonté d'un réfrigérant, on met sous agitation et à 100 °C pendant 12 heures, le composé **15** (1 g, 3,82 mmoles) en présence de 0,20 ml de 1,3-dibromopropane (1,91 mmoles) dans 20 ml de DMPU.

Après réaction, le milieu réactionnel est refroidi à température ambiante puis versé sur de l'acétone (50 ml) puis de l'acétate d'éthyle (50 ml). Le précipité obtenu est filtré puis lavé plusieurs fois à l'acétone et enfin séché sous vide. On récupère 810 mg de composé **27** (poudre orange).

Les analyses sont conformes au produit attendu.

### Exemple 15 : Synthèse du dichlorure de 2- {(E)-[{4-[(4-methoxyphenyl)(methyl)amino]phenyl}(methyl)hydrazono]methyl}-3,3-dimethyl-1-[6-(4-{(E)-[methyl(phenyl)hydrazono]methyl}pyridinium-1-yl)hexyl]-3H-indolium

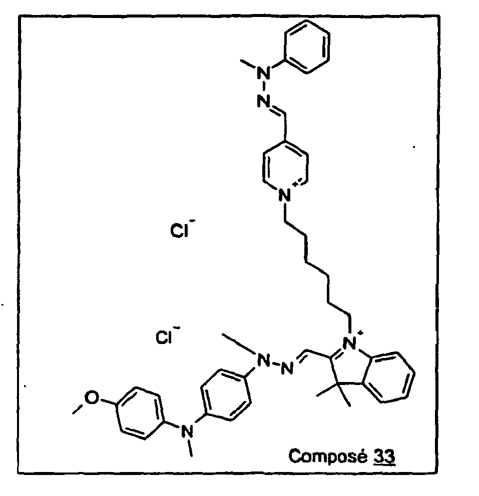

### Etape 1 : Synthèse du bromure de 1-(6-bromohexyl)-4- {(E)-[methyl(phenyl)hydrazono]methyl}pyridinium.

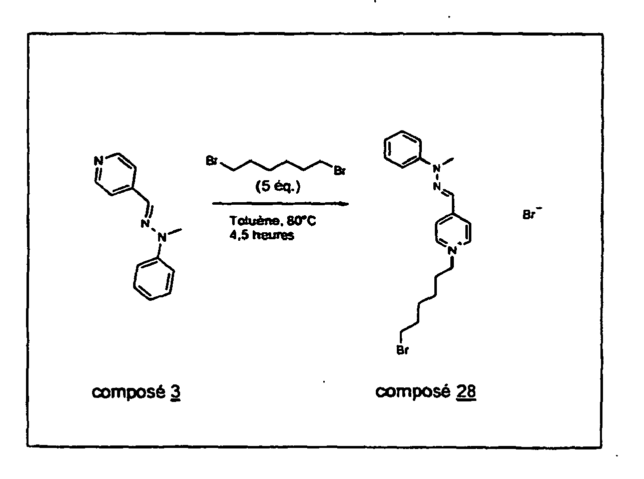

Dans un tricol de 3 litres surmonté d'un réfrigérant, on dissout le composé **3** (80,1 g, 0.379 mole) dans 250 ml de toluène. On porte à 80°C le milieu réactionnel et on additionne en 10 minutes une solution de 1,6-dibromohexane (1.94 moles) dans 750 ml de toluène. Un précipité se forme au cours de la réaction. Après 4,5 heures de réaction, le milieu réactionnel est refroidi puis filtré.

Purification : Le précipité obtenu est lavé avec 200 ml de toluène puis 100 ml d'éther de pétrole. Par la suite, ce précipité est solubilisé dans 1 litre de dichlorométhane puis extrait à l'eau. La phase organique est lavée 4 fois à l'eau, séchée sur sulfate de magnésium, filtrée puis concentrée. On obtient une huile marron qui est reprise par 50 ml de toluène. On obtient un précipité jaune qui est séparé par filtration puis séché pour conduire à 95,1 g du composé **28** sous la forme d'une poudre jaune.

Les analyses sont conformes au produit attendu.

### Etape 2: Synthèse du dibromure de 2,3,3-trimethyl-1-[6-(4-{(E)-[methyl(phenyl)hydrazono]methyl}pyridinium-1-yl)hexyl]-3H-indolium

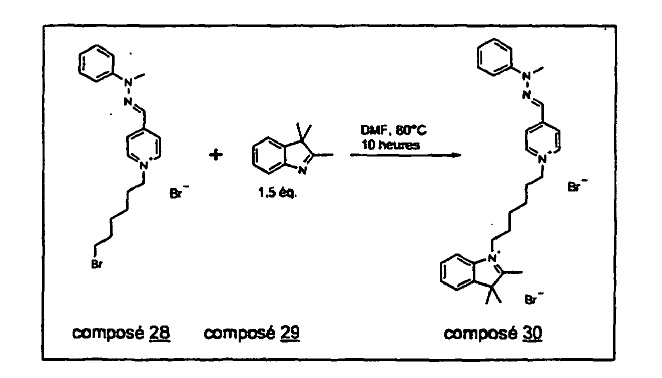

Dans un tricol surmonté d'un réfrigérant, on met sous agitation et à 80°C pendant 10 heures, le composé **28** (10 g, 21,96 mmoles) en présence de 5,3 ml de composé **29** (33,02 mmoles) dans 10 ml de DMF.

Après réaction, le milieu réactionnel est refroidi à température ambiante puis versé sur de l'éther diéthylique (400 ml). Un résidu huileux est obtenu. L'éther diéthylique et la DMF sont séparés de ce résidu. Le résidu est repris avec de l'isopropanol. La solution alcoolique est versée lentement sur de l'éther diéthylique. Le précipité obtenu est alors filtré puis séché sous vide. On récupère 12,31 g de composé **30** (poudre rouge).

Les analyses sont conformes au produit attendu.

### Etape 3 : Synthèse du bromure de 1-(6-{(2E)-2-[((E)-{4-[(4-methoxyphenyl)amino]phenyl}diazenyl)methylene]-3,3-dimethyl-2,3-dihydro-1H-indol-1-yl}hexyl)-4-{(E)-[methyl(phenyl)hydrazono]methyl}pyridinium

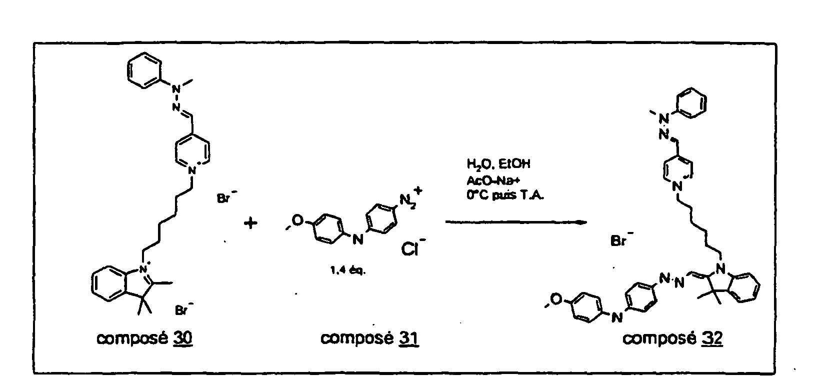

Dans un tricol de 1 litre, on dissout le composé **30** (7,24 g, 0.013 mole) dans 270 ml d'eau et 400 ml d'éthanol. Le milieu réactionnel est refroidi à 0°C. 4,38 g du composé **31** est ajouté lentement au milieu réactionnel. Le pH est ajusté à 5,7 par ajout d'acétate de sodium. Après 4,5 heures de réaction, le milieu réactionnel est ramené à température ambiante et le pH à 11 par ajout d'une solution de soude. Un précipité marron noire se forme. Le précipité est filtré puis séché sous vide. On obtient 8,79 g du composé **32** sous la forme d'une poudre noire.

Les analyses sont conformes au produit attendu.

### Etape 4 : Synthèse du dichlorure de 2-{(E)-[{4-[(4-methoxyphenyl)(methyl)amino]phenyl}(methyl)hydrazono]methyl}-3,3-dimethyl-1-[6-(4-{(E)-[methyl(phenyl)hydrazono]methyl}pyridinium-1-yl)hexyl]-3H-indolium

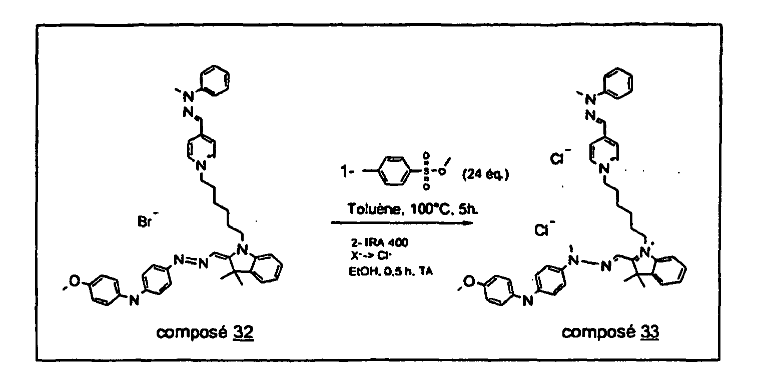

Dans un tricol de 50 ml surmonté d'un réfrigérant, on dissout le composé **32** (0,20 g, 0.26 mmole) dans 5 ml de toluène et 1,18 g de *p-*toluène sulfonic acid methyl ester (24 éq. par rapport au composé **32**) sont ajoutés au milieu réactionnel. On porte le milieu réactionnel à 100°C pendant 5 heures. Après réaction, le milieu réactionnel est concentré puis repris avec de l'éthanol (50 ml). 10 g de résine Amberlite IRA 400 sont ajoutés au milieu réactionnel précédent. On laisse sous agitation pendant 0,5 heure à température ambiante. La résine est par la suite retirée par filtration. Les jus sont alors évaporés à sec. Le solide obtenu est lavé avec de l'éther diéthylique. Après filtration, puis séchage sous vide, 0,21 g du composé **33** est obtenu sous la forme d'une poudre noire.

Les analyses sont conformes au produit attendu.

### Exemple 16 : Synthèse du dichlorure de 1,1'-hexane-1,6-diylbis(2-{(E)-[{4-[(4-methoxyphenyl)(methyl)amino]phenyl}(methyl)hydrazono] methyl}-3,3-dimethyl-3H-indolium)

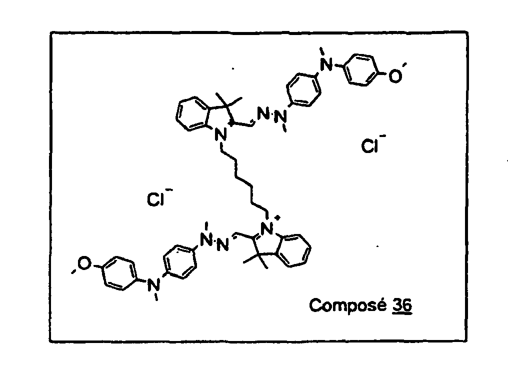

### Etape 1: Synthèse du dibromure de 1,1'-hexane-1,6-diylbis(2,3,3-trimethyl-3H-indolium)

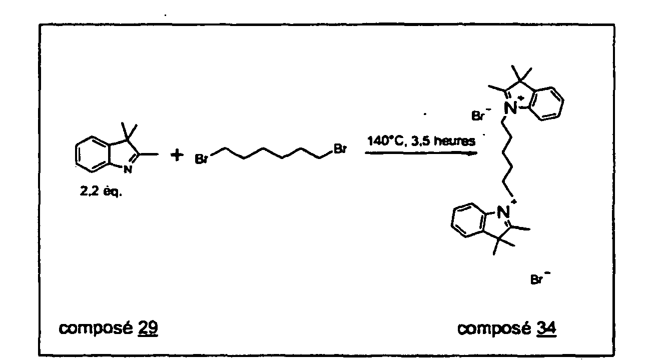

**Référence bibliographique:** F. Ribes, R. Guglielnetti, J. Metzger, Bulletin de la Société Chimique de France, 1972, n°1, pages 143-147.

Dans un tricol surmonté d'un réfrigérant, on met sous agitation et à 140°C pendant 3,5 heures, le composé **29** (11 ml, 68,52 mmoles) en présence de 1,6-dibromohexane (4,8 ml,31,20 mmoles).

Après réaction, le milieu réactionnel est refroidi à température ambiante puis versé sur de l'acétone (200 ml). Un précipité se forme. Celui-ci est filtré sur fritté puis séché sous vide.

**Purification :** Le solide précédemment obtenu est solubilisé dans 10 ml d'isopropanol. La solution alcoolique est versée lentement sur de l'éther diéthylique. Un précipité rougêatre se forme lentement. Le précipité obtenu est alors filtré puis séché sous vide. On récupère 12,70 g de composé **34** (poudre rouge).

Les analyses sont conformes au produit attendu.

### Etape 2 : Synthèse du N,N'-{hexane-1,6-diylbis[(3,3-dimethyl-1H-indol-1-yl-2-ylidene)(E)methylylidene(E)diazene-2,1-diyl-4,1-phenylene]}bis(4-methoxyaniline)

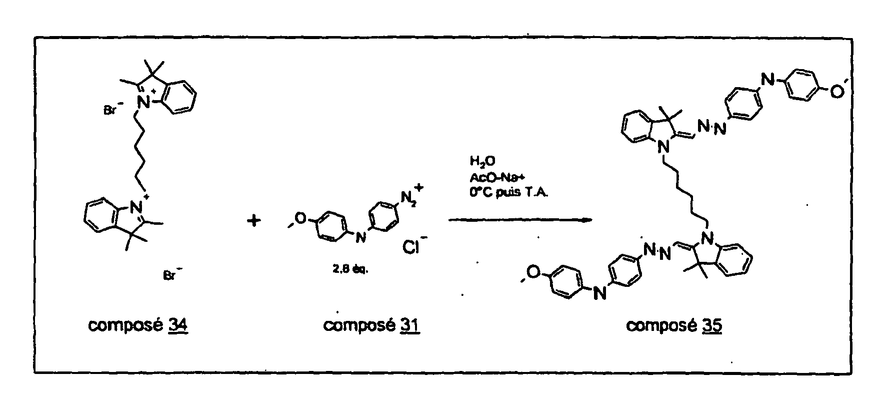

Dans un tricol de 500 ml, on dissout le composé **34** (2,15 g, 3,82 mmoles) dans 45 ml d'eau. Le milieu réactionnel est refroidi à 0°C. 2,8 g du composé **31** (10,69 mmoles) sont ajoutés lentement au milieu réactionnel. Le pH est ajusté à 5,7 par ajout d'acétate de sodium. Après 7,5 heures de réaction, le milieu réactionnel est ramené à température ambiante et le pH à 11 par ajout d'une solution de soude. Un précipité se forme. Le précipité est filtré puis séché sous vide. On obtient 3,58 g du composé **35** sous la forme d'une poudre marron.

Les analyses sont conformes au produit attendu.

### Etape 3: Synthèse du dichlorure de 1,1'-hexane-1,6-diylbis(2-{(E)-[{4-[(4-methoxyphenyl)(methyl)amino]phenyl}(methyl)hydrazono] methyl}-3,3-dimethyl-3H-indolium)

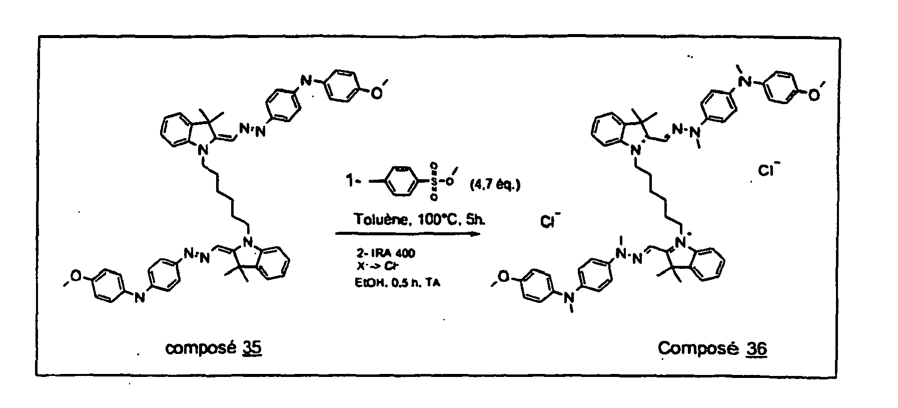

Dans un tricol de 50 ml surmonté d'un réfrigérant, on dissout le composé **35** (0,62 g, 0.68 mmole) dans 5 ml de toluène et 0,6 g de *p-*toluène sulfonic acid methyl ester (4,7 éq. par rapport au composé **35**) est ajouté au milieu réactionnel. On porte le milieu réactionnel à 100°C pendant 5 heures. Après réaction, le milieu réactionnel est concentré puis repris avec de l'éthanol (50 ml). 10 g de résine Amberlite IRA 400 sont ajoutés au milieu réactionnel précédent. On laisse sous agitation pendant 0,5 heure à température ambiante. La résine est par la suite retirée par filtration. Les jus sont alors évaporés à sec. Le solide obtenu est lavé avec de l'éther diéthylique. Après filtration, puis séchage sous vide, 0,44 g du composé **36** est obtenu sous la forme d'une poudre violette foncée.

Les analyses sont conformes au produit attendu.

### Exemples de teintures :

On a préparé les compositions tinctoriales dans les proportions suivantes

**Solution 1**

| | |
|---|---|
| Alkyl (C8/C10 50/50) polyglucoside (2) en solution aqueuse à 60% | 120 g |
| Ethanol absolu pur | 200 g |
| Polyéthylène glycol(8 OE) 400 | 60 g |
| Alcool benzylique pur | 40 g |
| Eau déminéralisée | qsp 1000g |

**Solution 2 : TAMPON pH 9,5**

| | |
|---|---|
| Chlorure d'ammonium (NH4Cl) | 54 g |
| Ammoniaque en sol. à 20% | qsp pH=9,5 (env. 40 ml) |
| Eau déminéralisée | qsp 1000 ml |

**Solution 3 : TAMPON pH 7**

| | |
|---|---|
| KH₂PO₄ | 0,026mol/l |
| Na₂PO₄ | 0,041mol/l |
| Eau déminéralisée | qsp 500ml |

Les compositions de coloration sont obtenues en dissolvant le colorant indiqué ci dessous (5x10⁻³mol/l) dans la solution 1 puis en ajoutant un volume équivalent de solution tampon 2 ou 3 (pH 7 ou 9,5).

Chaque composition est appliquée sur des cheveux gris à 90% de blancs, (1g de mèche pour 6g de solution). Après 30 min de pause, les mèches sont rincées, lavées avec un shampooing standard, rincées à nouveau puis séchées.

Les résultats de teinture suivants ont été obtenus :

| | Après coloration | |
|---|---|---|
| | pH7 | pH 9,5 |
| 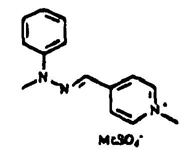 | Jaune chromatique | Jaune chromatique |
| C.1 Basic Yellow | | |
| 87: composé 1 non-conforme à l'invention | | |
| Composé 4 | Jaune | Jaune |
| | chromatique | chromatique |
| | intense | intense |
| Composé 6 | Jaune | Jaune |
| | chromatique | chromatique |
| | intense | intense |
| Composé 8 | Jaune | Jaune |
| | chromatique | chromatique |
| | intense | intense |
| Composé 10 | Jaune | Jaune |
| | chromatique | chromatique |
| | intense | intense |
| Composé 12 (non conforme à l'invention) | Jaune | Jaune |
| | chromatique | chromatique |
| | intense | intense |
| Composé 25 | Orange | Orange |
| | chromatique | chromatique |
| Composé 26 | Orange | Orange |
| | chromatique | chromatique |
| Composé 27 | Orange | Orange |
| | chromatique | chromatique |
| Composé 33 | violet | violet |
| Composé 36 | Violet | Violet |

Les mèches ainsi colorées sont soumises à un test de résistance aux lavages qui consiste à effectuer 10 shampoings et à évaluer la couleur après ces 10 shampooings. La couleur est évaluée par la mesure du L*a*b* dans le système L* a* b*, au moyen d'un spectrophotomètre CM 2002 MINOLTA ®, (Illuminant D65). Dans ce système L* a* b*, les trois paramètres désignent respectivement l'intensité (L*), a* indique l'axe de couleur vert/rouge et b* l'axe de couleur bleu/jaune.

Les résultats suivants ont été obtenus.

| | Après 10 shampooings (% de perte de la couleur) |
|---|---|
| Composé 1 C 1 Basic Yellow 87 | 20-30 |
| Composé 4 | 0-10 |
| Composé 6 | 0-10 |
| Composé 8 | 0-10 |
| Composé 10 | 0-10 |

Ces résultats montrent que les composés de l'invention permettent d'obtenir une teinture améliorée en terme de tenue aux shampooings par rapport à la composition non conforme à l'invention contenant le composé I.

### Chromaticité

Les compositions tinctoriales suivantes sont préparées :

| | |
|---|---|
| Colorant | 10-3 mole |
| Support de teinture (1) | (*) |
| Eau déminéralisée q.s.p. | 100g |

| | |
|---|---|
| (*) : support de teinture (1) pH 7 | |

| Exemple | A | B |
|---|---|---|
| Colorant | 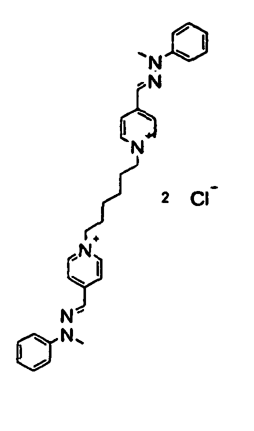 | 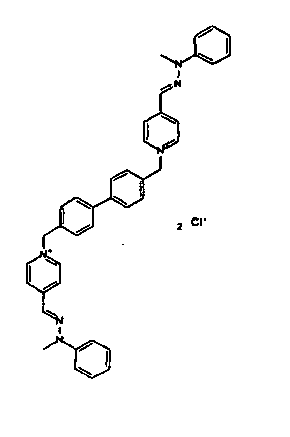 |

L'exemple A illustre la présente invention. L'exemple B est représentatif de l'enseignement du document CIBA WO04083312

### Support de teinture (1) pH7 :

| | |
|---|---|
| Alcool éthylique à 96° | 20,8 g |
| Sel pentasodique de l'acide diéthylène-triamine-pentaacétique en solution aqueuse à 40% | 0,48 g M.A |
| Alkyl en C8-C10 polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous.

La couleur des mèches a été évaluée dans le système L* a* b*, au moyen d'un spectrophotomètre CM 2002 MINOLTA ®, (Illuminant D65). Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Ces essais montrent que l'on obtient une coloration plus intense avec la composition de l'invention.

| Exemple | A | B |
|---|---|---|
| L* | 46 | 51.2 |

Les compositions tinctoriales suivantes sont préparées:

| | |
|---|---|
| Colorant | 10-3 mole |
| Support de teinture (2) | (*) |
| Eau déminéralisée q.s.p. | 100g |

| | |
|---|---|
| (*) : support de teinture (2) pH 9.5 | |

| Exemple | C | D |
|---|---|---|
| Colorant | 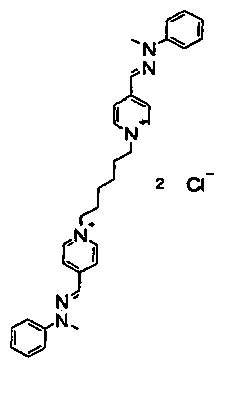 | 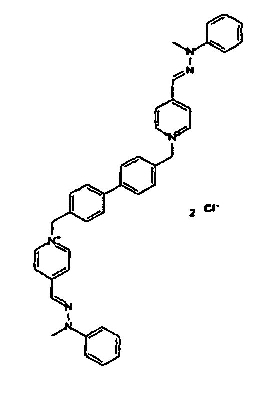 |

### Support de teinture (2) pH 9.5 :

| | |
|---|---|
| Alcool éthylique à 96° | 20,8 g |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous. La couleur des mèches a été évaluée dans le système L* a* b*, au moyen d'un spectrophotomètre CM 2002 MINOLTA ®, (Illuminant D65). Comme précédemment, ces exemples montrent que la composition de l'invention permet d'obtenir une coloration des fibres kératiniques plus intenses.

| Exemple | C | D |
|---|---|---|
| Nuance observée | Jaune intense | jaune |
| L* | 48.7 | 56.5 |

## Revendications

1. Composé bis-hydrazone dicationique de formule générale (I)
COL -L- COL
dans laquelle chacun des chromophores COL, identique ou différent, correspond aux formules (Ia), (Ib), (Ic), (Id), (Ie), (If) et (Ig) suivantes : 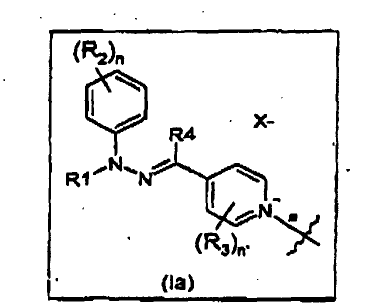 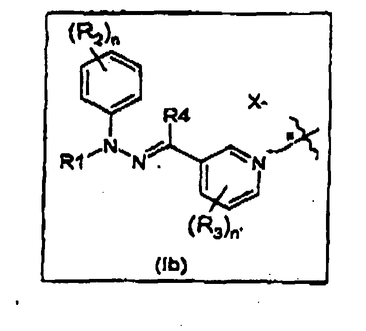 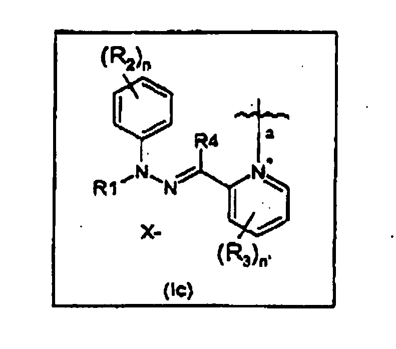 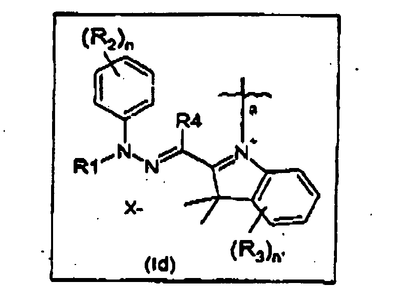 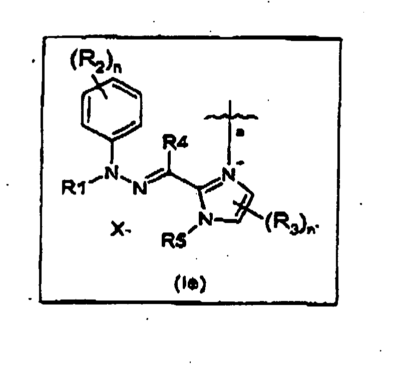 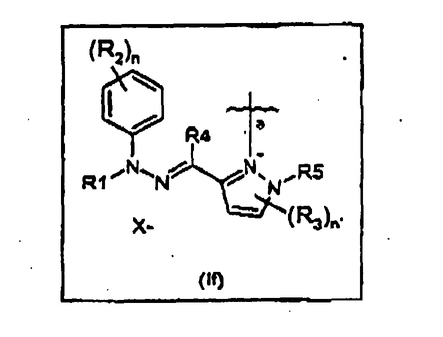 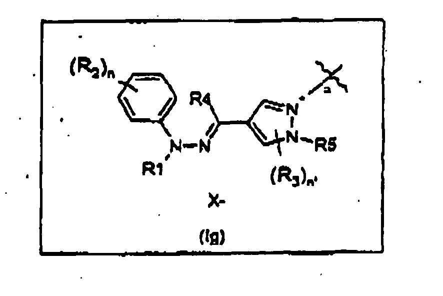 dans lesquelles :
- les groupes R₁ et R₅, indépendamment les uns des autres représentent une chaîne hydrocarbonée en C₁-C₂₀, de préférence en C₁-C₁₆ linéaire ou ramifiée, saturée ou insaturée, pouvant former un ou plusieurs cycles carbonés comportant de 3 à 7 chaînons éventuellement substitués, éventuellement aromatiques, cette chaîne étant éventuellement substituée, éventuellement interrompue par un ou plusieurs hétéroatomes ou par un ou plusieurs groupements portant un hétéroatome, ces hétéroatomes étant de préférence choisi parmi l'oxygène, l'azote ;
- les groupes R₂ et R₃, indépendamment l'un de l'autre, représentent :
• un atome d'halogène choisi parmi le brome, le chlore ou le fluor ;
• une chaîne hydrocarbonée en C₁-C₁₆, linéaire ou ramifiée, saturée ou insaturée, pouvant former un ou plusieurs cycles carbonés comportant de 3 à 6 chaînons éventuellement aromatiques, cette chaîne étant éventuellement substituée, éventuellement interrompue par un ou plusieurs hétéroatomes ou par un ou plusieurs groupements portant au moins un hétéroatome, ces hétéroatomes étant de préférence choisi parmi l'oxygène, l'azote ;
• un groupement hydroxyle,
• un groupement alcoxy en C₁-C₄ ; un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle en C₁-C₄ ; un groupement aryloxy éventuellement substitué.
• un groupement amino, un groupement amino substitué par un ou plusieurs radicaux alkyle en C₁-C₄, identiques ou différents, éventuellement porteurs d'au moins un groupement hydroxyle, les deux radicaux alkyle pouvant éventuellement former avec l'atome d'azote auquels ils sont rattachés, un hétérocycle à 5 ou 6 chaînons saturé ou insaturé éventuellement substitué, éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄; un groupement carbamoyle ((R)₂N-CO-) dans lequel les radicaux R indépendamment les uns des autres, représentent un atome d'hydrogène, un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ : un groupement sulfonamide ((R)₂N-SO₂-) dans lequel les radicaux R indépendamment les uns des autres, représentent un atome d'hydrogène, un radical alkyle en C₁-C₄, ; un groupement alkylthio (R-S-) dans lequel le groupement R représente un radical alkyle en C₁-C₄ ; un groupement alkylsulfonylamino (RSO₂-NR'-) dans lequel les radicaux R, R', indépendamment les uns des autres, représentent un atome d'hydrogène, un radical alkyle en C₁-C₄ ;
• un groupement nitro ; un groupement cyano ; un atome d'halogène, de préférence le chlore, le fluor ;
• un groupement trifluorométhyle(CF₃) ;
- R₁ et R₂ peuvent également former avec l'atome d'azote substitué par R₁, un hétérocycle saturé ou insaturé à 5 ou 6 chaînons substitué ou non substitué ;
- deux radicaux R₂ adjacents peuvent former entre eux et avec les atomes de carbone auxquels ils sont reliés un cycle aromatique ou un hétérocycle saturé ou insaturé à 5 ou 6 chaînons substitué ou non ;
- deux radicaux R₂ adjacents peuvent former entre eux un cycle aromatique ou un hétérocycle saturé ou insaturé à 5 ou 6 chaînons substitué ou non ;
- les groupes R₄, indépendamment les uns des autres, représentent :
• un atome d'hydrogène ;
• une chaîne hydrocarbonée en C₁-C₁₆, linéaire ou ramifiée, saturée ou insaturée, pouvant former un ou plusieurs cycles carbonés comportant de 3 à 6 chaînons éventuellement aromatiques, cette chaîne étant éventuellement substituée, éventuellement interrompue par un ou plusieurs hétéroatomes ou par un ou plusieurs groupements portant au moins un hétéroatome, ces hétéroatomes étant de préférence choisi parmi l'oxygène, l'azote ;
• un groupement amino, un groupement amino substitué par un ou plusieurs radicaux alkyle en C₁-C₄, identiques ou différents, éventuellement porteurs d'au moins un groupement hydroxyle, les deux radicaux alkyle pouvant éventuellement former avec l'atome d'azote auquels ils sont rattachés, un hétérocycle à 5 ou 6 chaînons saturé ou insaturé éventuellement substitué, éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ;un groupement alkylsulfonylamino (RSO₂-NR'-) dans lequel les radicaux R, R', indépendamment les uns des autres, représentent un atome d'hydrogène, un radical alkyle en C₁-C₄ ;
- n est un entier compris entre 0 et 5,
- n'est un entier compris entre 0 et 4,
- la liaison *a* issue des formules (Ia), (Ib), (Ic), (Id), (Ie), (If) et (Ig) relie chacun des chromophores COL au bras de liaison L de la formule (I),
- X est un anion ou un mélange d'anions, organiques ou minéraux permettant d'équilibrer la ou les charges des composés (Ia), (Ib), (Ic), (Id), (Id), (Ie), If, (Ig)
- le groupe L est choisi parmi les radicaux alkylène (CₙH₂ₙ) comprenant de 1 à 60 atomes de carbone, de préférence de 2 à 40 atomes de carbone, et plus préféré de 2 à 20 atomes de carbone, éventuellement substitués et/ou interrompus par un ou plusieurs hétéroatomes, L ne comportant pas de groupe peroxyde, nitro, diazo ou nitroso, le bras de liaison L étant relié à l'atome d'azote quaternisé de chacun des chromophores COL par l'intermédiaire d'un atome de carbone, L n'étant pas cationique.

2. Composé bis hydrazone dicationique selon la revendication 1 tel que les groupes R₁ sont identiques et représentent un radical alkyle en C₁-C₄, éventuellement substitué par un ou plusieurs radicaux choisis parmi les radicaux hydroxy, alcoxy en C₁-C₂, amino, (di)alkylamino en C₁-C₂ ; un radical alkylcarbonyle (R-CO-) dans lequel le radical R représente un radical alkyle en C₁-C₄ ; un groupement carbamoyle ((R)₂N-CO-) dans lequel les radicaux R indépendamment les uns des autres, représentent un atome d'hydrogène, un radical alkyle en C₁-C₄ ; un radical alkylsulfonyle (R-SO₂-) dans lequel le radical R représente un radical alkyle en C₁-C₄

3. Composé bis hydrazone dicationique selon la revendication 2 tel que les groupes R₁ sont identiques et représentent un radical méthyle, éthyle, 2-hydroxyéthyle, 2-méthoxyéthyle, acétyle (CH₃CO-), méthylsulfonyle (CH₃SO₂-).

4. Composé bis hydrazone dicationique selon la revendication 1 tel que les groupes R₁ et R₂ forment avec l'atome d'azote substitué par R₁, un hétérocycle saturé ou insaturé à 5 ou 6 chaînons substitué par un ou plusieurs radicaux alkyle

5. Composé bis hydrazone dicationique selon la revendication 1 tel que les groupes R₂ et R₃ représentent indépendamment l'un de l'autre :
- un atome d'hydrogène ;
- un atome d'halogène ;
- un radical alkyle en C₁-C₄, linéaire ou ramifié, éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, thio (-SH), alkyl(C₁-C₄)sulfinyl, alkyl(C₁-C₄)sulfonyle, thioalkyle(C₁-C₄) ;
- un radical phényle éventuellement substitué par un ou plusieurs radicaux choisis parmi les radicaux hydroxy, alcoxy en C₁-C₂, (poly)-hydroxyalcoxy en C₂-C₄, amino, (di)alkylamino en C₁-C₂, ou un atome d'halogène tel que le chlore, le fluor ou le brome ;
- un radical alcoxy en C₁-C₄ ;
- un radical alkyl(en C₁-C₄)sulfonylamino ;
- un radical (poly)-hydroxyalcoxy en C₂-C₄ ;
- un radical amino ;
- un radical (di)alkylamino en C₁-C₂ ;
- un radical (poly)-hydroxyalkylamino en C₂-C₄ ;
- un radical alkylcarbonyle (R-CO-) dans lequel le radical R représente un radical alkyle en C₁-C_{4 ;}
- un radical carbamoyle ((R)₂N-CO-) dans lequel les radicaux R indépendamment les uns des autres, représentent un atome d'hydrogène, un radical alkyle en C₁-C₄ ;
- un radical alkylsulfonylamino (RSO₂N-) dans lequel le radical R représente un radical alkyle en C₁-C₄ ;
- un radical aminosulfonyl ((R)₂NSO₂-) dans lequel les radicaux R indépendamment les uns des autres représentent un atome d'hydrogène, un radical alkyle en C₁-C₄ ;
- un radical alkylthio (RS-) dans lequel le radical R représenté un radical alkyle en C₁-C₄.
- un radical thio (HS-)
- un radical alkylsulfinyle (RSO-) dans lequel le radical R représente un radical alkyle en C₁-C₄.
- un radical alkylsulfonyle (R-SO₂-) dans lequel le radical R représente un radical alkyle en C₁-C₄ ;
- un radical alkylcarbonylamino (RCONR'-) dans lequel le radical R représente un atome d'hydrogène, un radical alkyle en C₁-C₄ et le radical R' représente un atome d'hydrogène, un radical alkyle en C₁-C_{4.}

6. Composé bis hydrazone dicationique selon la revendication 5 tel que les groupes R₂ et R₃ représentent indépendamment l'un de l'autre un atome d'hydrogène, un radical méthyle, éthyle, 2-hydroxyétbyle, 2-méthoxyéthyle, acétyle (CH₃CO-), méthylsulfonyle (CH₃SO₂-), amide (CH₃CONH-), hydroxyle, amino, méthylamino, diméthylamino, 2-hydroxyéthylamino, méthoxy, éthoxy, phényle.

7. Composé bis hydrazone dicationique selon l'une des revendications précédentes tel que les groupes R₅ sont identiques et représentent un radical alkyle en C₁-C₄, éventuellement substitué par un ou plusieurs radicaux choisis parmi les radicaux hydroxy, alcoxy en C₁-C₂, amino, (di)alkylamino en C₁-C₂ ; un radical phényle éventuellement substitué.

8. Composé bis hydrazone dicationique selon la revendication précédente, tel que les groupes R₅ sont identiques et représentent préférentiellement un radical méthyle, éthyle, 2-hydroxyéthyle, 2-méthoxyéthyle, phényle.

9. Composé bis hydrazone dicationique selon l'une des revendications précédentes tel qu'il est choisi parmi les composé de formules : 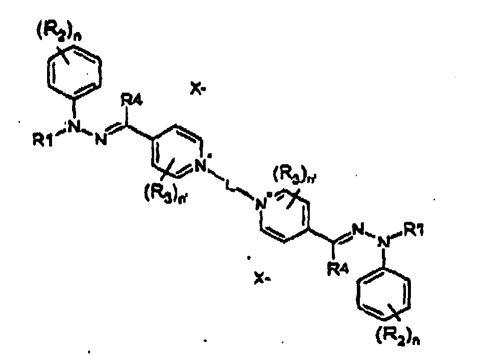 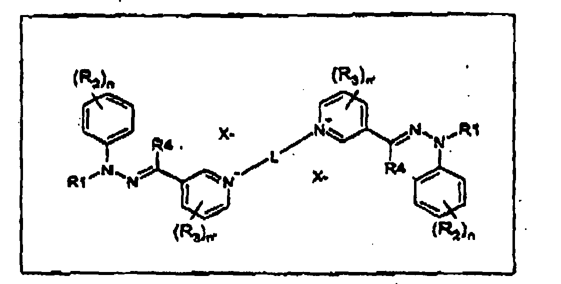 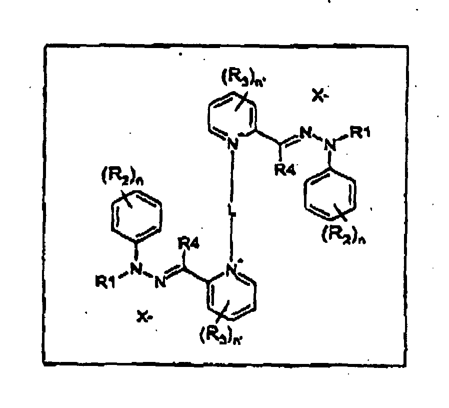 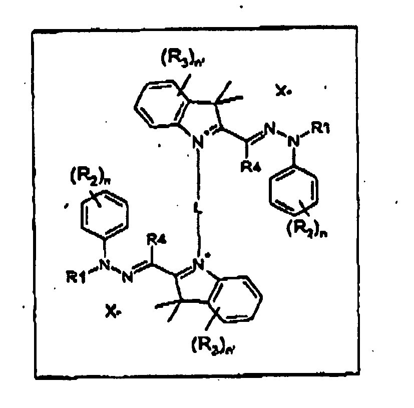 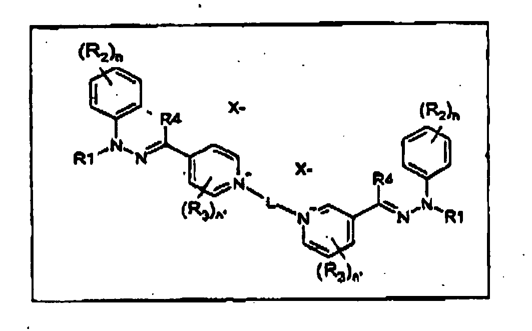 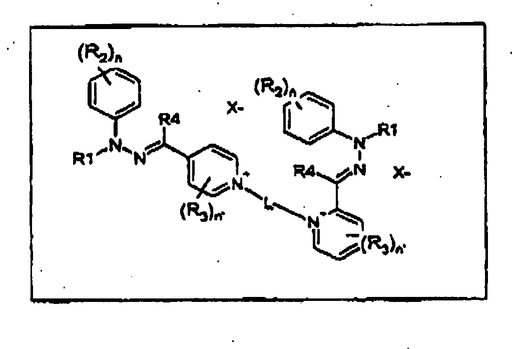 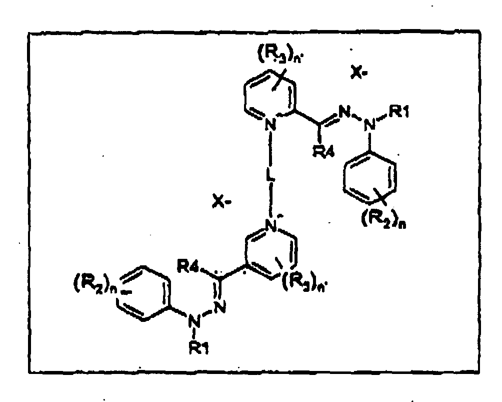 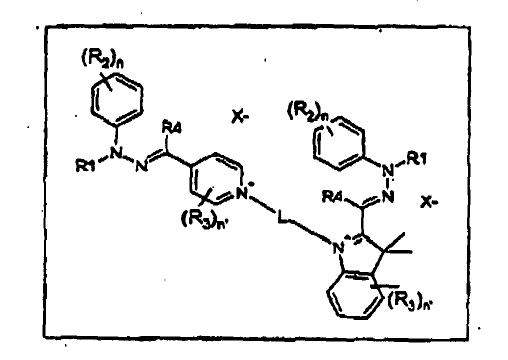 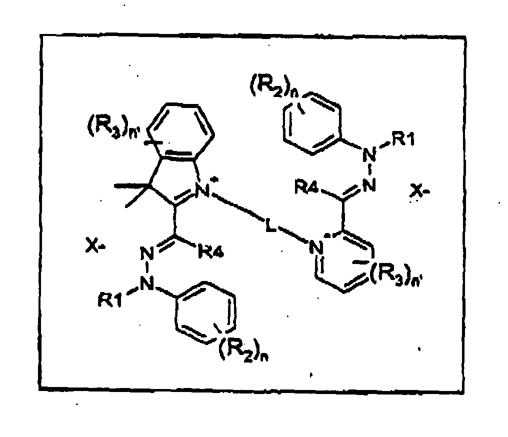 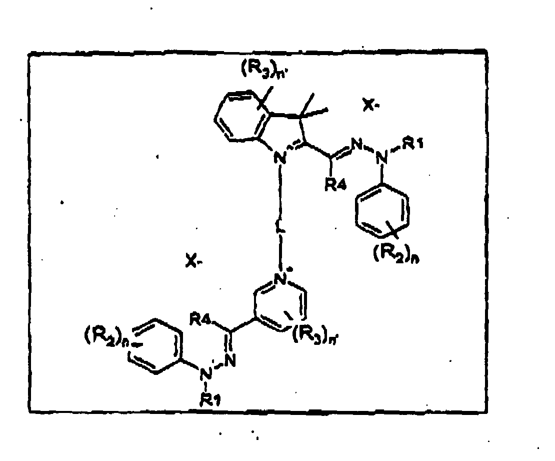 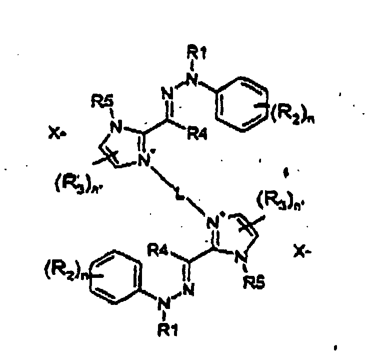 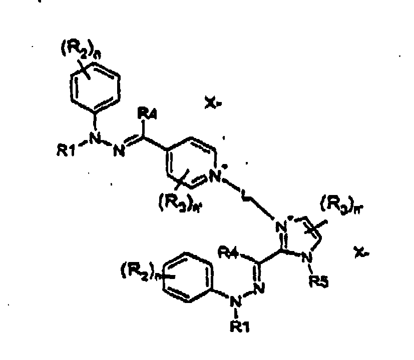 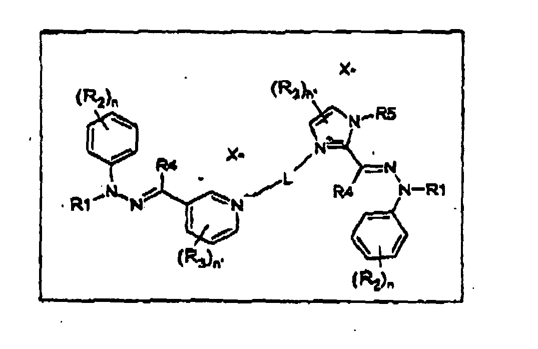 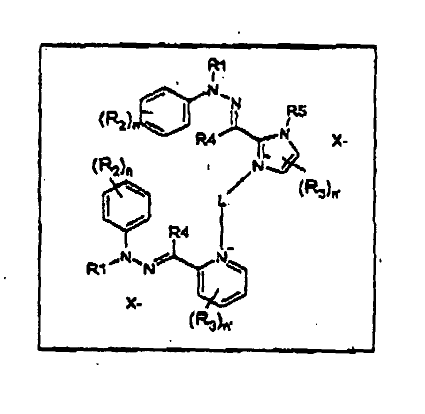 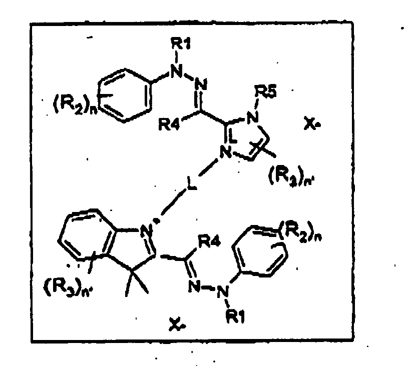 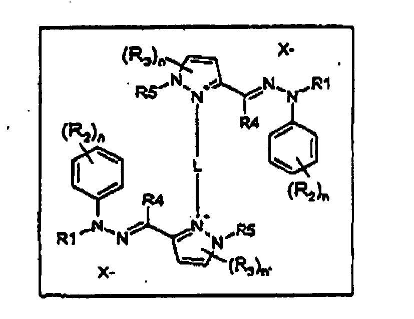 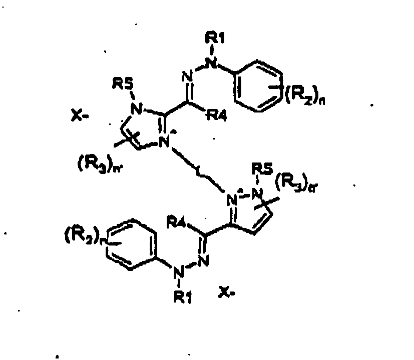 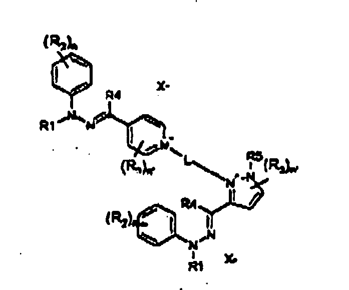 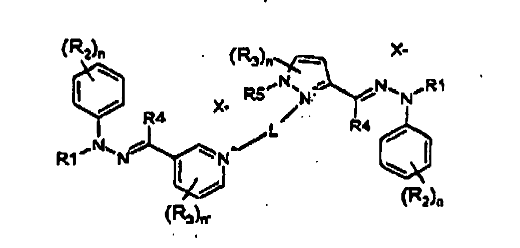 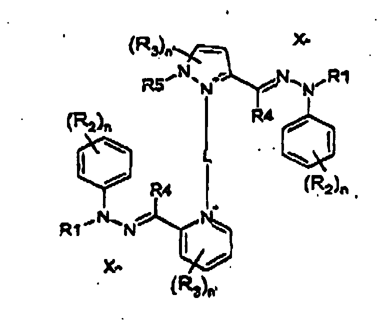 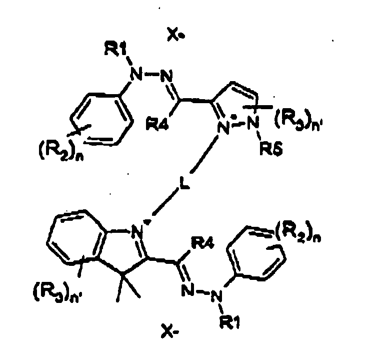 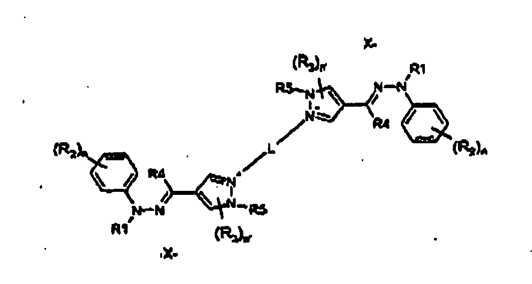 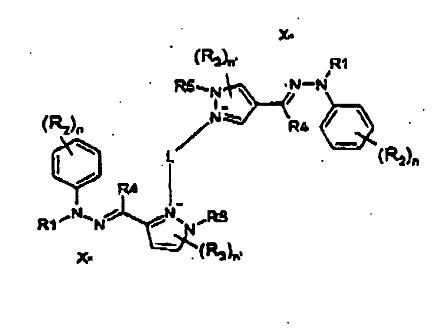 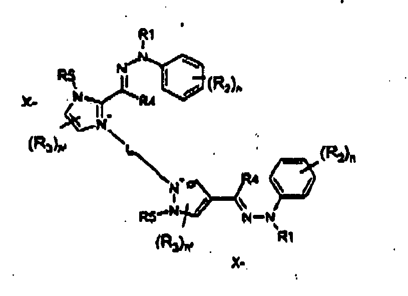 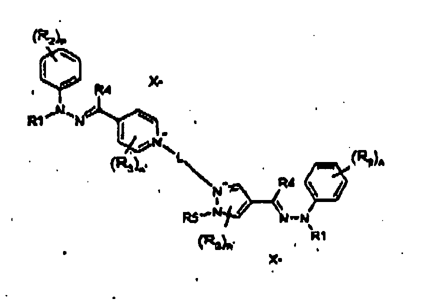 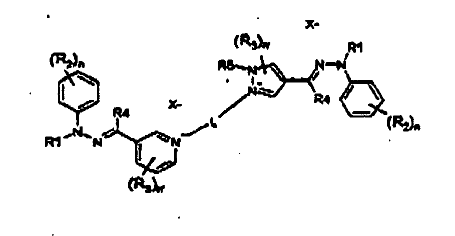 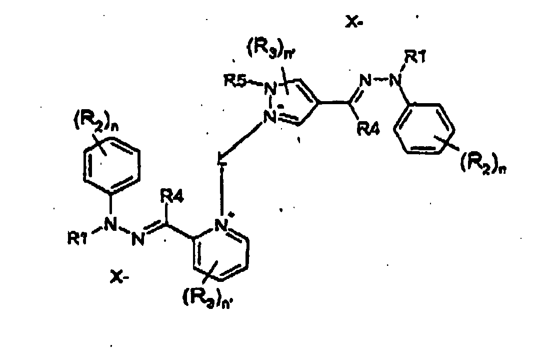 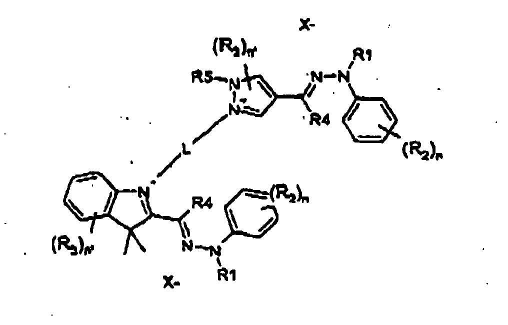 dans lesquelles les radicaux R₁, R₂, R₃, R₄, R₅, X, n, n' et L sont tels que définis précédemment.

10. Composé bis hydrazone dicationique selon l'une des revendications précédentes tel qu'il est choisi parmi les composés suivants :
- le dibromure de 4-{(E)-(methyl(phenyl)hydrazono]methyl}-1-[3-(4-{(E)[methyl(phenyl)hydrazono]methyl}pyridinium-1-yl)propyl]pyridinium,
- le dibromure de 4-{(E)-[methyl(phenyl)hydrazono]methyl}-1-[4-(4-{(E)-[methyl(phenyl)hydrazono]methyl}pyridinium-1-yl)butyl)pyridinium,
- le dibromure de 4-{(E)-[methyl(phenyl)bydrazono[methyl}-1-[5-(4-{(E)-[methyl(phenyl)hydrazono]methyl}pyridinium-1-yl)pentyl]pyridinium,
- le dibromure de 4-{(E)-(methyl(phenyl)hydrazono)methyl)-1-[6-(4-{(E)-[methyl(phenyl)bydrazono]methyl}pyridinium-1-yl)hexyl]pyridinium,
- le dibromure de 4-{(E)-(methyl(phenyl)hydrazono]methyl}-1-[6-(4-{(E)-[methyl(phenyl)hydrazono]methyl}quinolinium-1-yl)hexyl]quinolinum,
- le dibromure de 4-{(E)-[methyl(phenyl)hydrazono]methyl}-1-[6-(4-{(E)-[methyl(phenyl)hydrazono]methyl}pyridinium-1-yl)hexyl]quinolinium,
- le dibromure de 1-methyl-3-[5-(1-methyl-2-{(E)-[methyl(phenyl)hydrazono]methyl}-1H-imidazol-3-ium-3-yl)pentyl]-2-{(E)-[methyl(phenyl)hydrazono]methyl}-1H-imidazol-3-ium;
- le dibromure de 1-methyl-3-[4-(1-methyl-2-{(E)-[methyl(phenyl)hydrazono]methyl}-1H-benzimidazol-3-ium-3-yl)butyl]-2-{(E)-[methyl(phenyl)hydrazono]methyl}-1H-benzimidazol-3-ium,
- le dibromure de 1-[6-(1-methyl-2-{(E)-[methyl(phenyl)hydrazono]methyl}-1H-imidazol-3-ium-3-yl)hexyl]-4-{(E)-[methyl(phenyl)hydrazono]methyl}quinolinium,
- le dibromure-1-[6-(1-methyl-2-{(E)-[methyl(phenyl)hydrazono]methyl)-1H-benzimidazol-3-ium-3-yl)hexyl]-4-{(E)-[methyl(phenyl)hydrazono]methyl}quinolinium,
- le dibromure de 1,1'-pentane-1,5-diylbis(4-{(E)-[methyl(phenyl)hydrazono]methyl}quinolinium),
- le dibromure de 1,1'-butane-1,4-diylbis(4-{(E)[methyl(phenyl)hydrazono]methyl}quinolinium),
- le dibromure de 1,1'-propane-1,3-diylbis(4-{(E)-[methyl(phenyl)hydrazono]methyl}quinolinium),
- le dichlorure de 2-{(E)-[{4-[(4-methoxyphenyl)(methyl)amino]phenyl}(methyl)hydrazono]methyl}-3,3-dimethyl-1-[6-(4-{(E)-[methyl(phenyl)hydrazono]methyl}pyridinium-1-yl)hexyl]-3H-indolium,
- le dichlorure de 1,1'-hexane-1,6-diylbis(2-{(E)-[{4-[(4-methoxyphenyl)(methyl)amino]phenyl}(methyl)hydrazono]methyl}-3,3-dimethyl-3H-indolium).

11. Composition tinctoriale pour la teinture des fibres kératiniques comprenant dans un milieu de teinture approprié au moins un composé bis-hydrazone dicationique selon l'une des revendications 1 à 10.

12. Composition tinctoriale selon la revendication 11 comprenant de 0,001 à 10%, de préférence de 0,01 à 10% en poids d'au moins un composé bis-hydrazone dicationique par rapport au poids total de la composition.

13. Composition selon l'une des revendications 11 ou 12 telle qu'elle comprend au moins un colorant direct additionnel.

14. Composition tinctoriale selon l'une des revendications 11 à 13 telle qu'elle contient au moins un précurseur de colorant d'oxydation choisi parmi les bases d'oxydation et les coupleurs.

15. Composition tinctoriale selon la revendication 14 telle qu'elle comprend au moins une base d'oxydation choisie parmi les paraphénylènediamines, les bis-phénylalkylènediamines, les paraaminophénols, les orthoaminophénols, les bases hétérocycliques et leurs sels d'addition.

16. Composition tinctoriale selon la revendication 15, dans laquelle la ou les bases d'oxydation sont présentes en une quantité comprise entre 0,001 à 20 % en poids et de préférence entre 0,005 et 6 % en poids par rapport au poids total de la composition.

17. Composition tinctoriale selon l'une des revendications 14 à 16 telle qu'elle comprend au moins un coupleur choisi parmi les métaphénylènediamines, les métaaminophénols, les métadiphénols, les coupleurs naphtaléniques, les coupleurs hétérocycliques et leurs sels d'addition.

18. Composition selon la revendication 17, telle que le ou les coupleurs sont présents en une quantité comprise entre 0,001 et 20 % de préférence entre 0,005 et 6 % en poids par rapport au poids total de la composition.

19. Composition selon l'une des revendications 11 à 13 telle qu'elle comprend au moins un solvant hydroxylé tel que l'éthanol, le propylène glycol, le glycérol, les mono éthers de polyols.

20. Composition selon l'une des revendications 11 à 19 telle qu'elle comprend au moins un adjuvant choisi parmi les agents tensio-actifs anioniques, cationiques, non-ioniques, amphotères, zwitterioniques ou leurs mélanges, les polymères anioniques, cationiques, non-ioniques, amphotères, zwitterioniques ou leurs mélanges, les agents épaississants minéraux ou organiques, et en particulier les épaississants associatifs polymères anioniques, cationiques, non ioniques et amphotères, les agents antioxydants, les agents de pénétration, les agents séquestrants, les parfums, les tampons, les agents dispersants, les agents conditionneurs tels que les silicones volatiles ou non volatiles, modifiées ou non modifiées, les agents filmogènes, les céramides, les agents conservateurs, les agents opacifiants.

21. Composition selon l'une des revendications 11 à 20 telle qu'elle comprend au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène.

22. Procédé de teinture des fibres kératiniques, tel qu'il comprend les étapes d'appliquer la composition tinctoriale selon l'une des revendications 11 à 21 sur les fibres kératiniques, puis à laisser pauser pendant une période suffisante pour obtenir la coloration désirée.

23. Utilisation de la composition selon l'une des revendications 11 à 22 sur les fibres kératiniques, pour obtenir des teintures présentant une bonne résistance aux agents extérieurs et aux shampooings.

## Claims

1. Dicationic bis-hydrazone compound of general formula (I)
DYE -L- DYE
in which each of the chromophores DYE, which may be identical or different, corresponds to formulae (Ia), (Ib), (Ic), (Id), (Ie), (If) and (Ig) below: 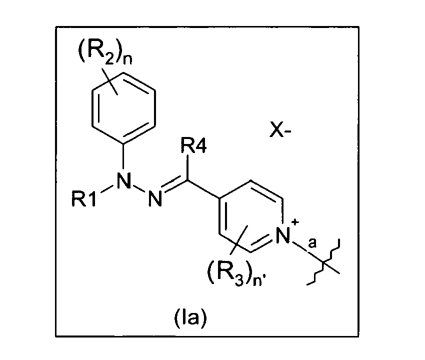 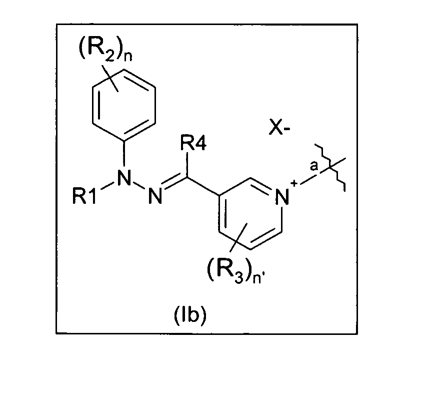 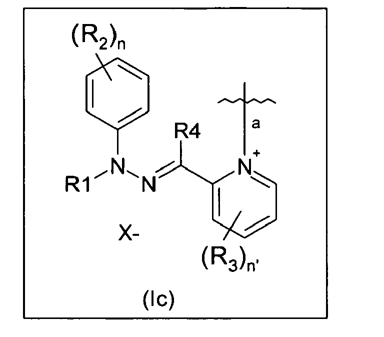 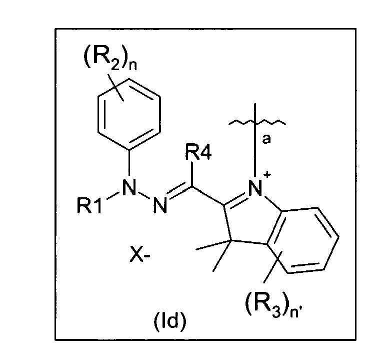 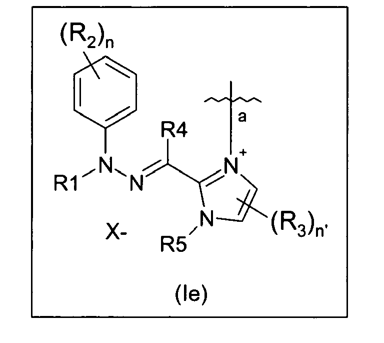 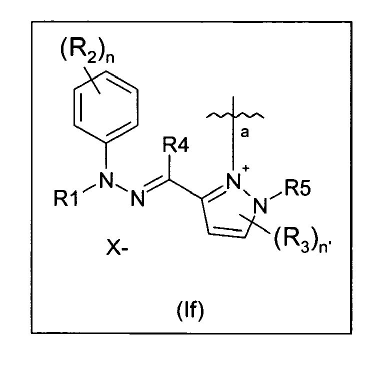 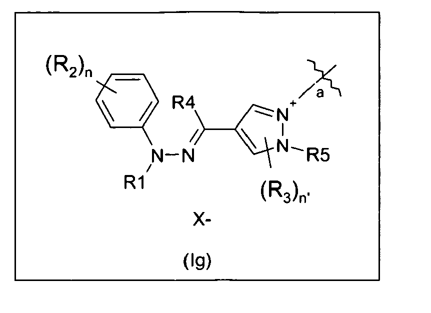 in which:
- the groups R₁ and R₅, independently of each other, represent a linear or branched, saturated or unsaturated C₁-C₂₀ and preferably C₁-C₁₆ hydrocarbon-based chain, which can form one or more optionally substituted, optionally aromatic 3- to 7-membered carbon-based rings, this chain being optionally substituted, optionally interrupted with one or more hetero atoms or with one or more groups bearing a hetero atom, these hetero atoms preferably being chosen from oxygen and nitrogen;
- the groups R₂ and R₃, independently of each other, represent:
• a halogen atom chosen from bromine, chlorine and fluorine;
• a linear or branched, saturated or unsaturated C₁-C₁₆ hydrocarbon-based chain, which can form one or more optionally aromatic 3- to 6-membered carbon-based rings, this chain being optionally substituted, optionally interrupted with one or more hetero atoms or with one or more groups bearing at least one hetero atom, these hetero atoms preferably being chosen from oxygen and nitrogen;
• a hydroxyl group;
• a C₁-C₄ alkoxy group; a C₂-C₄ (poly)hydroxyalkoxy group; an alkoxycarbonyl group (RO-CO-) in which R represents a C₁-C₄ alkyl radical or an alkylcarbonyloxy radical (RCO-O-) in which R represents a C₁-C₄ alkyl radical; an optionally substituted aryloxy group;
• an amino group, an amino group substituted with one or more C₁-C₄ alkyl radicals, which may be identical or different, optionally bearing at least one hydroxyl group, the two alkyl radicals possibly forming, with the nitrogen atom to which they are attached, a saturated or unsaturated, optionally substituted 5- or 6-membered heterocycle, optionally bearing another nitrogen or non-nitrogen hetero atom; an alkylcarbonylamino group (RCO-NR-) in which the radicals R, independently of each other, represent a C₁-C₄ alkyl radical; a carbamoyl group ((R)₂N-CO-) in which the radicals R, independently of each other, represent a hydrogen atom or a C₁-C₄ alkyl radical; a ureido group (N(R)₂-CO-NR'-) in which the radicals R and R', independently of each other, represent a hydrogen atom or a C₁-C₄ alkyl radical; a sulfonamide group ((R)₂N-SO₂-) in which the radicals R, independently of each other, represent a hydrogen atom or a C₁-C₄ alkyl radical; an alkylthio group (R-S-) in which the group R represents a C₁-C₄ alkyl radical; an alkylsulfonylamino group (RSO₂-NR'-) in which the radicals R and R', independently of each other, represent a hydrogen atom or a C₁-C₄ alkyl radical;
• a nitro group; a cyano group; a halogen atom, preferably chlorine or fluorine;
• a trifluoromethyl group (CF₃);
- R₁ and R₂ may also form, with the nitrogen atom substituted with R₁, a saturated or unsaturated, substituted or unsubstituted 5- or 6-membered heterocycle;
- two adjacent radicals R₂ may form, together with the carbon atoms to which they are attached, a saturated or unsaturated, substituted or unsubstituted 5- or 6-membered aromatic ring or heterocycle;
- two adjacent radicals R₃ can together form a saturated or unsaturated, substituted or unsubstituted 5- or 6-membered aromatic ring or heterocycle;
- the groups R₄, independently of each other, represent:
• a hydrogen atom;
• a linear or branched, saturated or unsaturated C₁-C₁₆ hydrocarbon-based chain, which can form one or more optionally aromatic 3- to 6-membered carbon-based rings, this chain being optionally substituted, optionally interrupted with one or more hetero atoms or with one or more groups bearing at least one hetero atom preferably chosen from oxygen and nitrogen;
• an amino group, an amino group substituted with one or more C₁-C₄ alkyl radicals, which may be identical or different, optionally bearing at least one hydroxyl group, the two alkyl radicals possibly forming, with the nitrogen atom to which they are attached, a saturated or unsaturated, optionally substituted 5- or 6-membered heterocycle, optionally bearing another nitrogen or non-nitrogen hetero atom; an alkylcarbonylamino group (RCO-NR-) in which the radicals R, independently of each other, represent a C₁-C₄ alkyl radical ; a ureido group (N(R)₂-CO-NR' -) in which the radicals R and R', independently of each other, represent a hydrogen atom or a C₁-C₄ radical; an alkylsulfonylamino group (RSO₂-NR'-) in which the radicals R and R', independently of each other, represent a hydrogen atom or a C₁-C₄ alkyl radical;
- n is an integer between 0 and 5,
- n' is an integer between 0 and 4,
- the bond a in formulae (Ia), (Ib), (Ic), (Id), (Ie), (If) and (Ig) links each of the chromophores DYE to the linker L of formula (I),
- X is an organic or mineral anion or mixture of anions for equilibrating the charge(s) of the compound (Ia), (Ib), (Ic), (Id), (Ie), (If) or (Ig);
- the group L is chosen from alkylene radicals (CₙH₂ₙ) containing from 1 to 60 carbon atoms, preferably from 2 to 40 carbon atoms and more preferably from 2 to 20 carbon atoms, optionally substituted and/or interrupted with one or more hetero atoms,
L not comprising any peroxide, nitro, diazo or nitroso groups, the linker L being linked to the quaternized nitrogen atom of each of the chromophores DYE via a carbon atom, L not being cationic.

2. Dicationic bis-hydrazone compound according to Claim 1, such that the groups R₁ are identical and represent a C₁-C₄ alkyl radical optionally substituted with one or more radicals chosen from hydroxyl, C₁-C₂ alkoxy, amino and C₁-C₂ (di)alkylamino radicals ; an alkylcarbonyl radical (R-CO-) in which the radical R represents a C₁-C₄ alkyl radical ; a carbamoyl group ((R)₂N-CO-) in which the radicals R, independently of each other, represent a hydrogen atom or a C₁-C₄ alkyl radical ; an alkylsulfonyl radical (R-SO₂-) in which the radical R represents a C₁-C₄ alkyl radical.

3. Dicationic bis-hydrazone compound according to Claim 2, such that the groups R₁ are identical and represent a methyl, ethyl, 2-hydroxyethyl, 2-methoxyethyl, acetyl (CH₃CO-) or methylsulfonyl (CH₃SO₂-) radical.

4. Dicationic bis-hydrazone compound according to Claim 1, such that the groups R₁ and R₂ form, with the nitrogen atom substituted with R₁, a saturated or unsaturated 5- or 6-membered heterocycle substituted with one or more alkyl radicals.

5. Dicationic bis-hydrazone compound according to Claim 1, such that the groups R₂ and R₃ represent, independently of each other:
- a hydrogen atom ;
- a halogen atom ;
- a linear or branched C₁-C₄ alkyl radical optionally substituted with one or more radicals chosen from hydroxyl, C₁-C₂ alkoxy, C₂-C₄ (poly)hydroxyalkoxy, amino, C₁-C₂ (di)alkylamino, thio (-SH), (C₁-C₄)alkylsulfinyl, (C₁-C₄)alkylsulfonyle and (C₁-C₄) thioalkyl radicals ;
- a phenyl radical optionally substituted with one or more radicals chosen from hydroxyl, C₁-C₂ alkoxy, C₂-C₄ (poly) hydroxyalkoxy, amino and C₁-C₂(di)alkylamino radicals, or a halogen atom such as chlorine, fluorine or bromine;
- a C₁-C₄ alkoxy radical ;
- a (C₁-C₄) alkylsulfonylamino radical ;
- a C₂-C₄ (poly)hydroxyalkoxy radical ;
- an amino radical ;
- a C₁-C₂ (di)alkylamino radical ;
- a C₂-C₄ (poly)hydroxyalkylamino radical ;
- an alkylcarbonyl radical (R-CO-) in which the radical R represents a C₁-C₄ alkyl radical;
- a carbamoyl radical ((R)₂N-CO-) in which the radicals R, independently of each other, represent a hydrogen atom or a C₁-C₄ alkyl radical;
- an alkylsulfonylamino radical (RSO₂N-) in which the radical R represents a C₁-C₄ alkyl radical ;
- an aminosulfonyl radical ((R)₂NSO₂-) in which the radicals R, independently of each other, represent a hydrogen atom or a C₁-C₄ alkyl radical ;
- an alkylthio radical (RS-) in which the radical R represents a C₁-C₄ alkyl radical ;
- a thio radical (HS-)
- an alkylsulfinyl radical (RSO-) in which the radical R represents a C₁-C₄ alkyl radical ;
- an alkylsulfonyl radical (R-SO₂-) in which the radical R represents a C₁-C₄ alkyl radical;
- an alkylcarbonylamino radical (RCONR'-) in which the radical R represents a hydrogen atom or a C₁-C₄ alkyl radical and the radical R' represents a hydrogen atom or a C₁-C₄ alkyl radical.

6. Dicationic bis-hydrazone compound according to Claim 5, such that the groups R₂ and R₃ represent, independently of each other, a hydrogen atom or a methyl, ethyl, 2-hydroxyethyl, 2-methoxyethyl, acetyl (CH₃CO-), methylsulfonyl (CH₃SO₂-), amide (CH₃CONH-), hydroxyl, amino, methylamino, dimethylamino , 2-hydroxyethylamino, methoxy, ethoxy or phenyl radical.

7. Dicationic bis-hydrazone compound according to one of the preceding claims, such that the groups R₅ are identical and represent a C₁-C₄, alkyl radical optionally substituted with one or more radicals chosen from hydroxyl, C₁-C₂ alkoxy, amino and C₁-C₂ (di)alkylamino radicals; an optionally substituted phenyl radical.

8. Dicationic bis-hydrazone compound according to the preceding claim, such that the groups R₅ are identical and preferentially represent a methyl, ethyl, 2-hydroxyethyl, 2-methoxyethyl or phenyl radical.

9. Dicationic bis-hydrazone compound according to one of the preceding claims, such that it is chosen from the compounds of formulae: 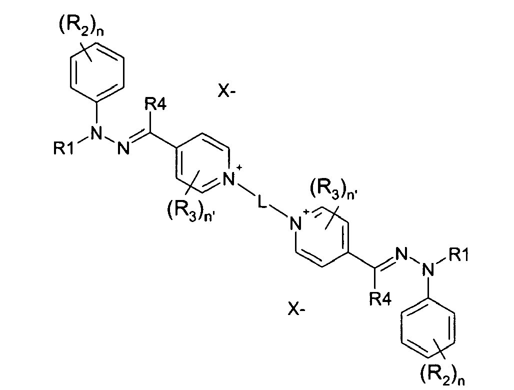 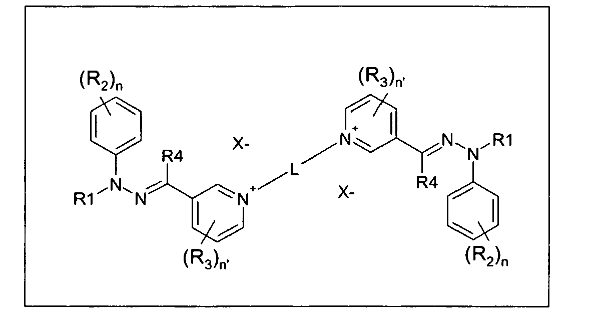 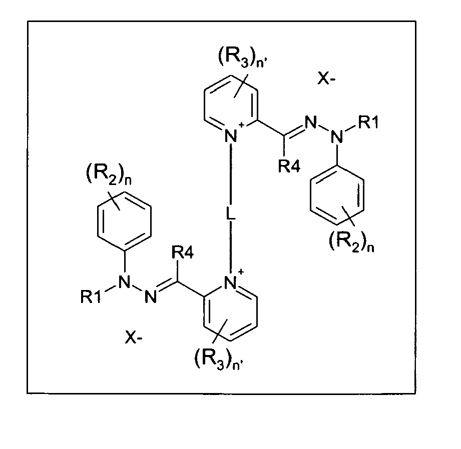 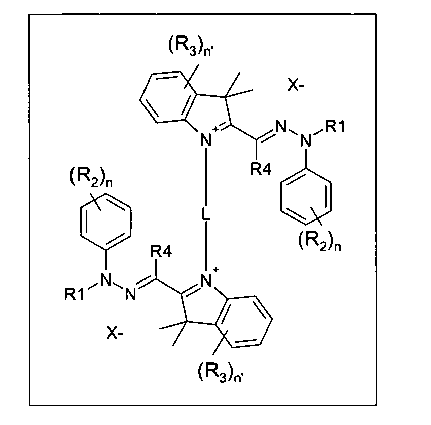 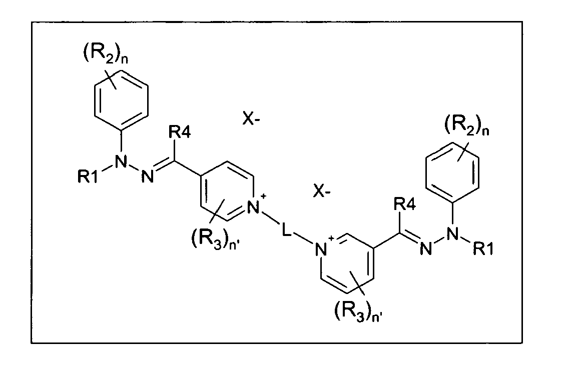 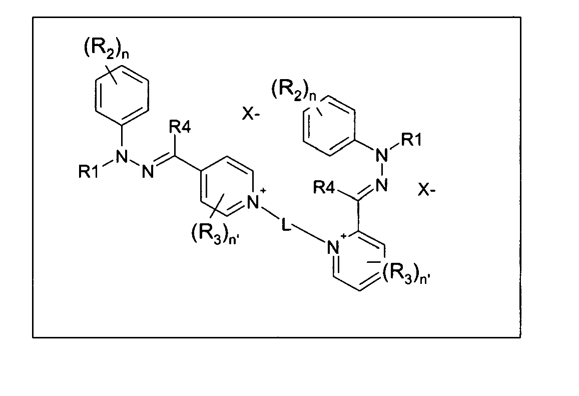 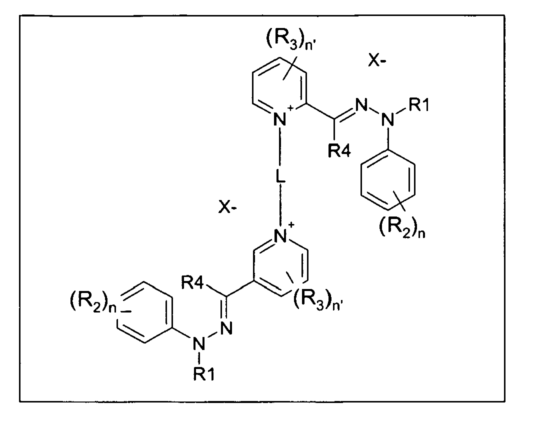 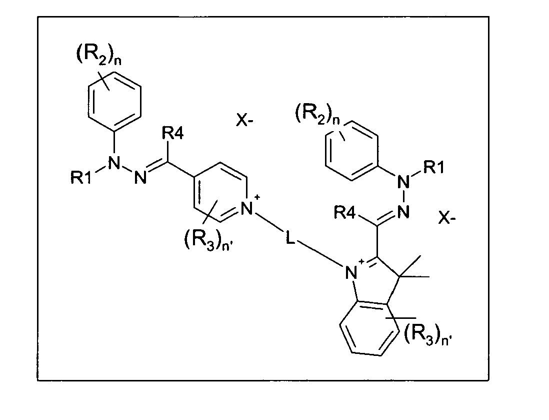 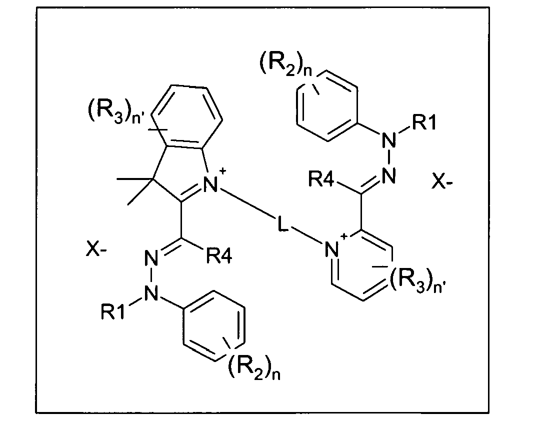 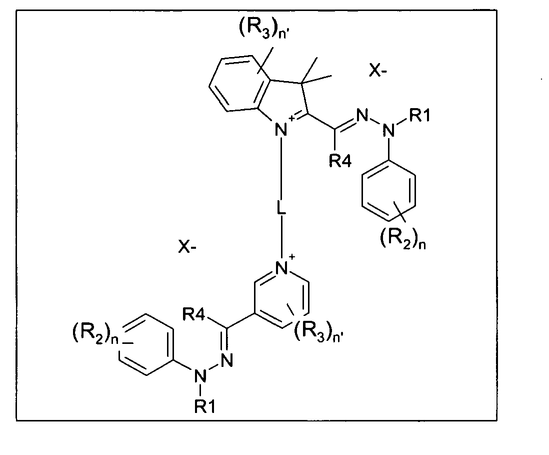 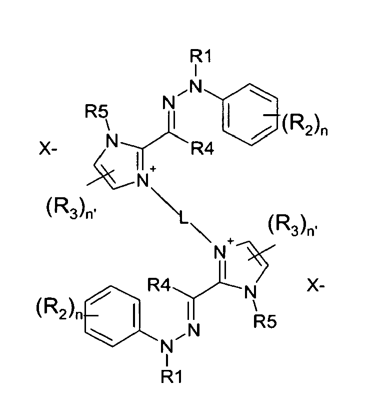 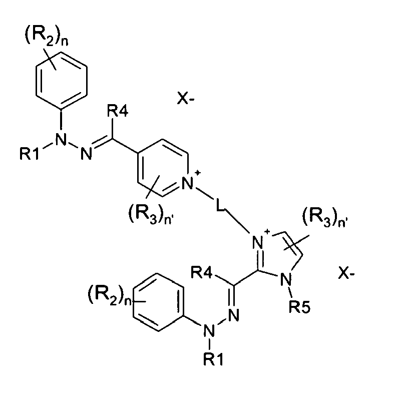 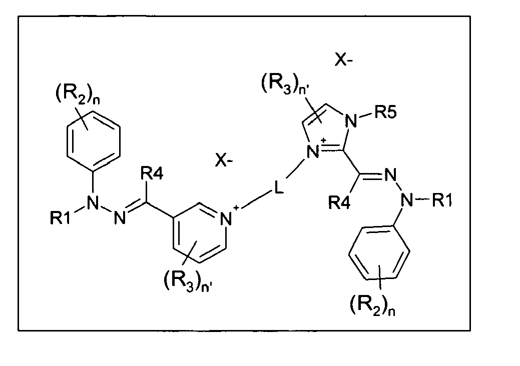 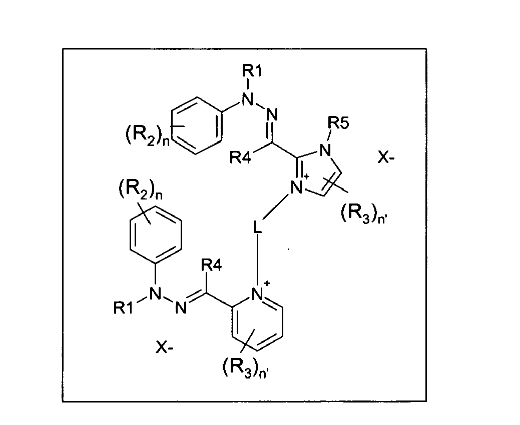 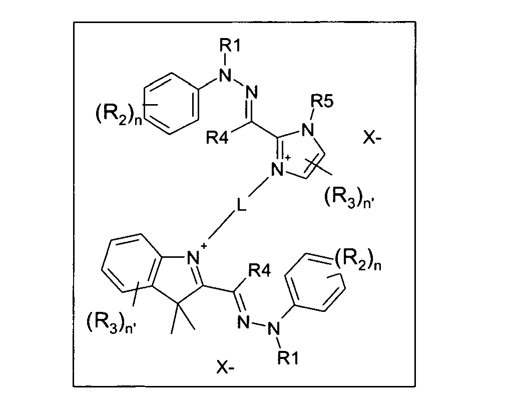 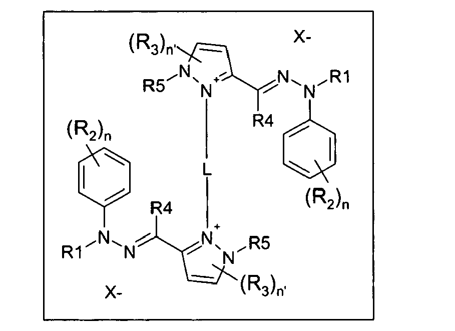 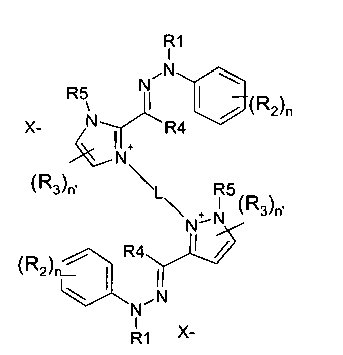 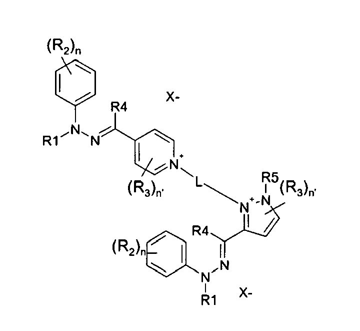 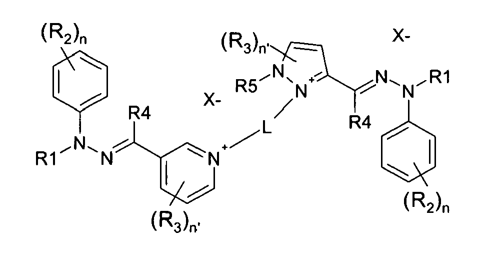 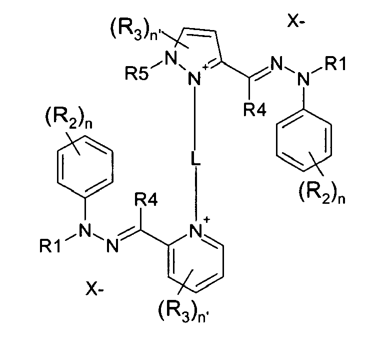 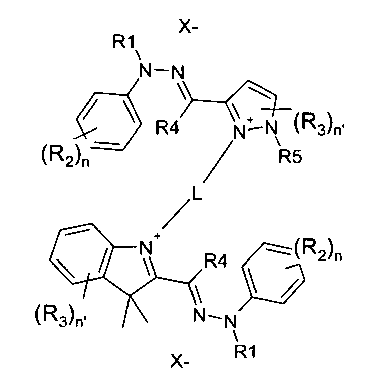 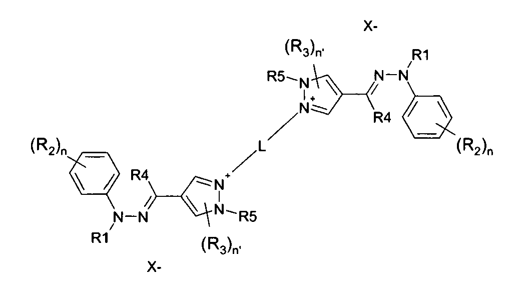 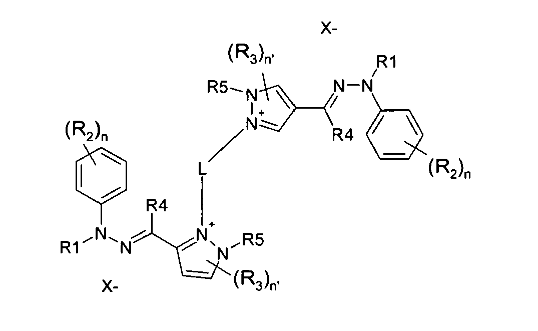 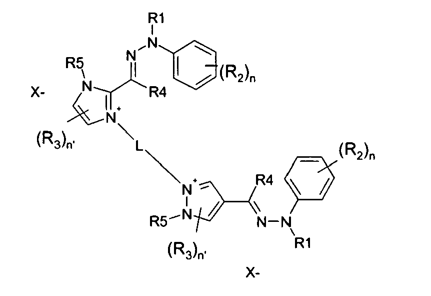 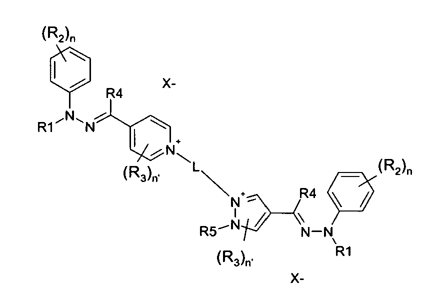 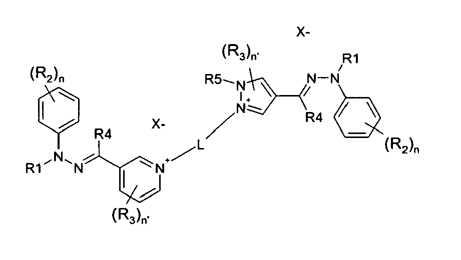 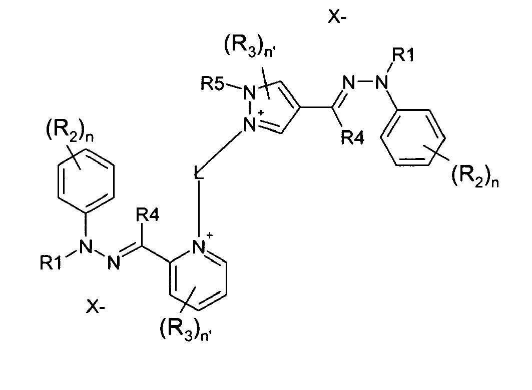 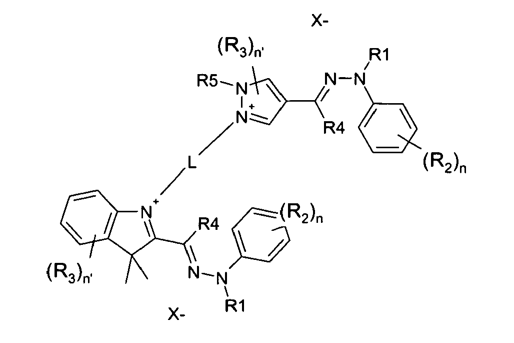 in which the radicals R₁, R₂, R₃, R₄, R₅, X, n, n' and L are as defined above.

10. Dicationic bis-hydrazone compound according to one of the preceding claims, such that it is chosen from the following compounds:
- 4-{(E)-[methyl(phenyl)hydrazono]methyl}-1-[3-(4-{(E)-[methyl(phenyl)hydrazono]methyl}pyridinium-1-yl)propyl]pyridinium dibromide,
- 4-{(E)-[methyl(phenyl)hydrazono]methyl}-1-[4-(4-{(E)-[methyl(phenyl)hydrazono]methyl}pyridinium-1-yl)butyl]pyridinium dibromide,
- 4-{(E)-[methyl(phenyl)hydrazono]methyl}-1-[5-(4-{(E)-[methyl(phenyl)hydrazono]methyl}pyridinium-1-yl)pentyl]pyridinium dibromide,
- 4-{(E)-[methyl(phenyl)hydrazono]methyl}-1-[6-(4-{(E)-[methyl(phenyl)hydrazono]methyl}pyridinium-1-yl)hexyl]pyridinium dibromide,
- 4-{(E)-[methyl(phenyl)hydrazono]methyl}-1-[6-(4-{(E)-[methyl(phenyl)hydrazono]methyl}quinolinium-1-yl)hexyl]quinolinium dibromide,
- 4-{(E)-[methyl(phenyl)hydrazono]methyl}-1-[6-(4-{(E)-[methyl(phenyl)hydrazono]methyl}pyridinium-1-yl)hexyl]quinolinium dibromide,
- 1-methyl-3-[5-(1-methyl-2-{(E)-[methyl(phenyl)hydrazono]methyl}-1H-imidazol-3-ium-3-yl)pentyl]-2-{(E)-[methyl (phenyl)hydrazono]methyl}-1H-imidazol-3-ium dibromide,
- 1-methyl-3-[4-(1-methyl-2-{(E)-[methyl(phenyl)hydrazono]methyl}-1H-benzimidazol-3-ium-3-yl)butyl]-2-{(E)-[methyl(phenyl)hydrazono]methyl}-1H-benzimidazol-3-ium dibromide,
- 1-[6-(1-methyl-2-{(E) - [methyl(phenyl)hydrazono]methyl}-1H-imidazol-3-ium-3-yl)hexyl]-4-{(E) - [methyl(phenyl)hydrazono]methyl}quinolinium dibromide,
- 1-[6-(1-methyl-2-{(E)-[methyl(phenyl)hydrazono]methyl}-1H-benzimidazol-3-ium-3-yl)hexyl]-4-{(E)-[methyl(phenyl)hydrazono]methyl}quinolinium dibromide,
- 1,1'-pentane-1,5-diylbis(4-{(E)[methyl(phenyl)hydrazono]methyl}quinolinium) dibromide,
- 1,1'-butane-1,4-diylbis(4-{(E)[methyl(phenyl)hydrazono]methyl}quinolinium) dibromide,
- 1,1'-propane-1,3-diylbis(4-{(E)[methyl(phenyl)hydrazono]methyl}quinolinium) dibromide,
- 2-{(E) [(4-[(4-methoxyphenyl)(methyl)amino]phenyl)-(methyl)hydrazono]methyl}-3,3-dimethyl-1-[6-(4-{(E)-[methyl(phenyl)hydrazono]methyl}pyridinium-1-yl)-hexyl]-3H-indolium dichloride,
- 1,1'-hexane-1, 6-diylbis (2-{(E)[{4-[(4-methoxyphenyl)-(methyl)amino]phenyl}(methyl)hydrazono]methyl}-3,3-dimethyl-3H-indolium) dichloride.

11. Dye composition for dyeing keratin fibres, comprising, in a suitable dyeing medium, at least one dicationic bis-hydrazone compound according to one of Claims 1 to 10.

12. Dye composition according to Claim 11, comprising from 0.001 to 10% and preferably from 0.01 to 10% by weight of at least one dicationic bis-hydrazone compound relative to the total weight of the composition.

13. Composition according to either of Claims 11 and 12, such that it comprises at least one additional direct dye.

14. Dye composition according to one of Claims 11 to 13, such that it contains at least one oxidation dye precursor chosen from oxidation bases and couplers.

15. Dye composition according to Claim 14, such that it comprises at least one oxidation base chosen from para-phenylenediamines, bis(phenyl)alkylenediamines, para-aminophenols, ortho-aminophenols and heterocyclic bases, and the addition salts thereof.

16. Dye composition according to Claim 15, in which the oxidation base(s) is (are) present in an amount of between 0.001% and 20% by weight and preferably between 0.005% and 6% by weight relative to the total weight of the composition.

17. Dye composition according to one of Claims 14 to 16, such that it comprises at least one coupler chosen from meta-phenylenediamines, meta-aminophenols, meta-diphenols, naphthalene-based couplers and heterocyclic couplers, and the addition salts thereof.

18. Composition according to Claim 17, such that the coupler(s) is (are) present in an amount of between 0.001% and 20% and preferably between 0.005% and 6% by weight relative to the total weight of the composition.

19. Composition according to one of Claims 11 to 18, such that it comprises at least one hydroxylated solvent such as ethanol, propylene glycol, glycerol or polyol monoethers.

20. Composition according to one of Claims 11 to 19, such that it comprises at least one adjuvant chosen from anionic, cationic, nonionic, amphoteric and zwitterionic surfactants or mixtures thereof, anionic, cationic, nonionic, amphoteric or zwitterionic polymers or mixtures thereof, mineral or organic thickeners, and in particular anionic, cationic, nonionic and amphoteric polymeric associative thickeners, antioxidants, penetrating agents, sequestering agents, fragrances, buffers, dispersants, conditioning agents such as volatile or non-volatile, modified or unmodified silicones, film-forming agents, ceramides, preserving agents and opacifiers.

21. Composition according to one of Claims 11 to 20, such that it comprises at least one oxidizing agent chosen from hydrogen peroxide, urea peroxide, alkali metal bromates, persalts, peracids and oxidase enzymes, and preferably hydrogen peroxide.

22. Process for dyeing keratin fibres, such that it includes the steps of applying the dye composition according to one of Claims 11 to 21 to the keratin fibres, and then leaving it to act for a period long enough to obtain the desired coloration.

23. Use of the composition according to one of Claims 11 to 22 on keratin fibres, to obtain dyeing results that show good resistance to external agents and to shampoo.

## Patentansprüche

1. Dikationisches Bis-hydrazon der allgemeinen Formel (I):
COL-L-COL
worin jedes der Chromophoren COL, die gleich oder verschieden sind, den folgenden Formeln (Ia), (Ib), (Ic), (Id), (Ie), (If) und (Ig) entsprechen: 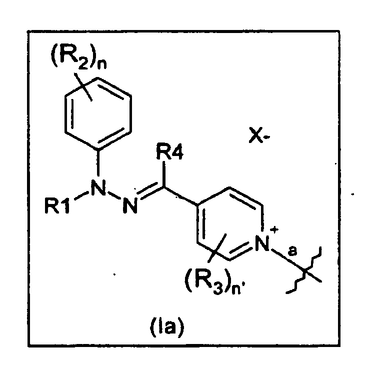 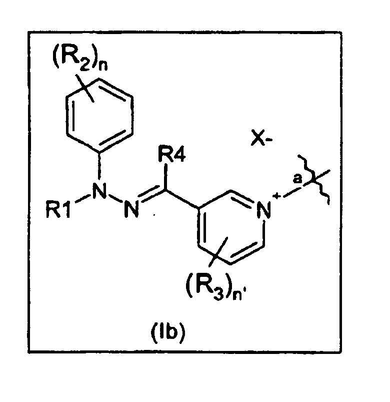 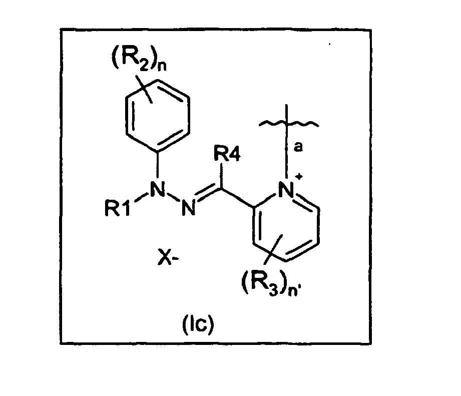 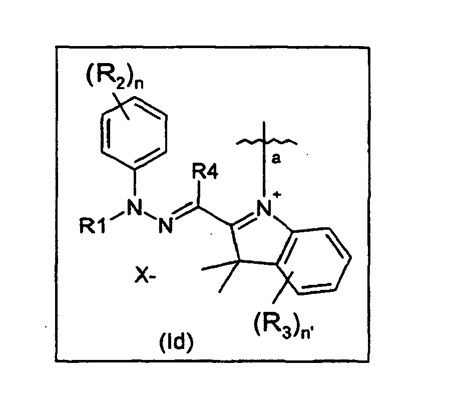 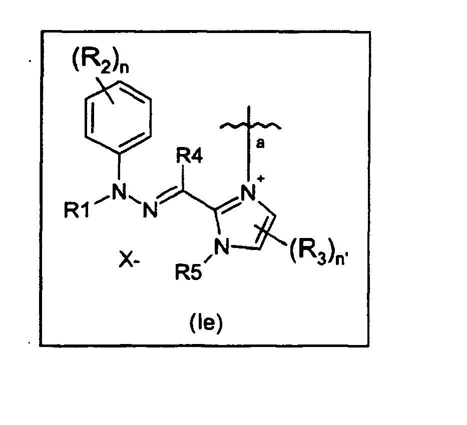 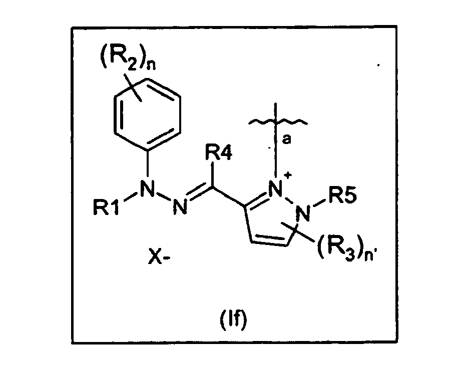 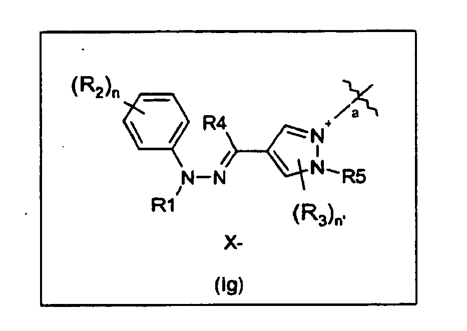 in den Formeln:
- die Gruppen R₁ und R₅ bedeuten unabhängig voneinander eine C₁₋₂₀-Kohlenwasserstoffkette und vorzugsweise eine C₁₋₁₆-Kohlenwasserstoffkette, die linear oder verzweigt, gesättigt oder ungesättigt ist, wobei sie einen oder mehrere 3-bis 7-giedrige Kohlenstoffringe bilden kann, die gegebenenfalls substituiert und gegebenenfalls aromatisch sind, wobei die Kette gegebenenfalls substituiert ist und gegebenenfalls durch ein oder mehrere Heteroatome oder eine oder mehrere Gruppen, die Heteroatome tragen, unterbrochen ist, wobei es sich bei diesen Heteroatomen vorzugsweise um Sauerstoff und Stickstoff handelt;
- die Gruppen R₂ und R₃ bedeuten unabhängig voneinander:
• ein Halogenatomatom, das unter Brom, Chlor oder Fluor ausgewählt ist;
• eine geradkettige oder verzweigte, gesättigte oder ungesättigte C₁₋₁₆-Kohlenwasserstoffkette, die einen oder mehrere 3- bis 6-giedrige, gegebenenfalls aromatische Kohlenstoffringe bilden kann, wobei die Kette gegebenenfalls substituiert ist und gegebenenfalls durch ein oder mehrere Heteroatome oder eine oder mehrere Gruppen, die Heteroatome tragen, unterbrochen ist, wobei es sich bei diesen Heteroatomen vorzugsweise um Sauerstoff und Stickstoff handelt;
• eine Hydroxygruppe;
• eine Alkoxy(C₁₋₄)gruppe; eine (Poly)hydroxyalkoxy(C₂-₄)-gruppe; eine Alkoxycarbonylgruppe (RO-CO-), bei der die Gruppe R eine Alkyl(C₁₋₄)gruppe bedeutet, eine Alkylcarbonyloxygruppe (RCO-O-), bei der die Gruppe R eine Alkyl(C₁₋₄)gruppe bedeutet; eine gegebenenfalls substituierte Aryloxygruppe;
• eine Aminogruppe, eine Aminogruppe, die mit einer oder mehreren, identischen oder verschiedenen Alkyl(C₁₋₄)-gruppen substituiert ist, die gegebenenfalls mindestens eine Hydroxygruppe tragen, wobei die beiden Alkylgruppen gegebenenfalls mit dem Stickstoffatom, an das sie gebunden sind, einen gesättigten oder ungesättigten, gegebenenfalls substituierten 5- oder 6-gliedrigen Heterocyclus bilden können, der gegebenenfalls ein weiteres Heteroatom enthält, bei dem es sich um Stickstoff handelt oder das von Stickstoff verschieden ist; eine Alkylcarbonylaminogruppe (RCO-NR-), bei der die Gruppen R unabhängig voneinander eine Alkyl(C₁₋₄)gruppe bedeuten; eine Carbamoylgruppe ((R)₂N-CO-), bei der die Gruppen R unabhängig voneinander ein Wasserstoffatom oder eine Alkyl(C₁₋₄)-gruppe bedeuten; eine Ureidogruppe (N(R)₂-CO-NR'-), bei der die Gruppen R und R' unabhängig voneinander ein Wasserstoffatom, eine Alkyl(C₁₋₄)gruppe bedeuten; eine Sulfonamidgruppe (N(R)₂-SO₂-), bei der die Gruppen R unabhängig voneinander ein Wasserstoffatom oder eine Alkyl(C₁₋₄)gruppe bedeuten; eine Alkylthiogruppe (R-S-), bei der die Gruppe R eine Alkyl(C₁₋₄)gruppe bedeutet; eine Alkylsulfonylaminogruppe (RSO₂-NR'-), bei der die Gruppen R und R' unabhängig voneinander ein Wasserstoffatom oder eine Alkyl(C₁₋₄)gruppe bedeuten;
• eine Nitrogruppe; eine Cyanogruppe; ein Halogenatom und vorzugsweise Chlor, Fluor;
• eine Trifluormethylgruppe (CF₃);
- die Gruppen R₁ und R₂ können auch gemeinsam mit dem mit R₁ substituierten Stickstoffatom einen gesättigten oder ungesättigten, 5-oder 6-gliedrigen Heterocyclus bilden, der unsubstituiert vorliegt oder substituiert ist;
- zwei aneinander angrenzende Gruppen R₂ können auch gemeinsam und mit den Kohlenstoffatomen, an die sie gebunden sind, einen gesättigten oder ungesättigten, 5-oder 6-gliedrigen aromatischen Ring oder Heterocyclus bilden, der unsubstituiert vorliegt oder substituiert ist;
- zwei aneinander angrenzende Gruppen R₃ können gemeinsam einen gesättigten oder ungesättigten, 5-oder 6-gliedrigen aromatischen Ring oder Heterocyclus bilden, der unsubstituiert vorliegt oder substituiert ist;
- die Gruppen R₄ bedeuten unabhängig voneinander:
• ein Wasserstoffatom;
• eine geradkettige oder verzweigte, gesättigte oder ungesättigte C₁₋₁₆-Kohlenwasserstoffkette, die einen oder mehrere 3- bis 6-giedrige, gegebenenfalls aromatische Kohlenstoffringe bilden kann, wobei die Kette gegebenenfalls substituiert ist und gegebenenfalls durch ein oder mehrere Heteroatome oder eine oder mehrere Gruppen, die Heteroatome tragen, unterbrochen ist, wobei es sich bei diesen Heteroatomen vorzugsweise um Sauerstoff und Stickstoff handelt;
• eine Aminogruppe, eine Aminogruppe, die mit einer oder mehreren, identischen oder verschiedenen Alkyl(C₁₋₄)-gruppen substituiert ist, die gegebenenfalls mindestens eine Hydroxygruppe tragen, wobei die beiden Alkylgruppen gegebenenfalls mit dem Stickstoffatom, an das sie gebunden sind, einen gesättigten oder ungesättigten, gegebenenfalls substituierten 5- oder 6-gliedrigen Heterocyclus bilden können, der gegebenenfalls ein weiteres Heteroatom enthält, bei dem es sich um Stickstoff handelt oder das von Stickstoff verschieden ist; eine Alkylcarbonylaminogruppe (RCO-NR-), bei der die Gruppen R unabhängig voneinander eine Alkyl(C₁₋₄)gruppe bedeuten; eine Ureidogruppe (N(R)₂-CO-NR'-), bei der die Gruppen R und R' unabhängig voneinander ein Wasserstoffatom oder eine Alkyl(C₁₋₄)gruppe bedeuten; eine Alkylsulfonylaminogruppe (RSO₂-NR'-) bei der die Gruppen R und R' unabhängig voneinander ein Wasserstoffatom oder eine Alkyl(C₁₋₄)-gruppe bedeuten;
- n ist eine ganze Zahl von 0 bis 5;
- n' ist eine ganze Zahl von 0 bis 4;
- die Bindung α der Formeln (Ia), (Ib), (Ic), (Id), (Ie), (If) und (Ig) verbindet jedes Chromophor COL mit der Verbindungsgruppe L der Formel (I);
- X bedeutet ein organisches oder anorganisches Anion oder ein Gemisch von organischen oder anorganischen Anionen, mit dem die Ladung oder die Ladungen der Verbindungen der Formeln (Ia), (Ib), (Ic), (Id), (Ie), (If) und (Ig) ausgeglichen werden können;
- die Gruppe L ist unter den Alkylengruppen (CₙH₂ₙ) ausgewählt, die 1 bis 60 Kohlenstoffatome, vorzugsweise 2 bis 40 Kohlenstoffatome und noch bevorzugter 2 bis 20 Kohlenstoffatome aufweisen und die gegebenenfalls mit einem oder mehreren Heteroatomen unterbrochen und/oder substituiert sind, wobei die Gruppe L keine Peroxidgruppe, Nitrogruppe, Diazogruppe oder Nitrosogruppe enthält, wobei die Verbindungsgruppe L über ein Kohlenstoffatom an das quaternisierte Stickstoffatom jedes Chromophors COL gebunden ist und wobei die Gruppe L nicht kationisch ist.

2. Dikationisches Bis-hydrazon nach Anspruch 1, bei dem die Gruppen R₁ gleich sind und bedeuten: eine C₁₋₄-Alkylgruppe, die gegebenenfalls mit einer oder mehreren Gruppen substituiert ist, die unter den Gruppen Hydroxy, Alkoxy(C₁₋₂), Amino, (Di)alkylamino(C₁₋₂) ausgewählt sind; eine Alkylcarbonylgruppe (R-CO-), bei der die Gruppe R eine Alkyl(C₁₋₄)gruppe bedeutet; eine Carbamoylgruppe ((R)₂N-CO-), bei der die Gruppen R unabhängig voneinander ein Wasserstoffatom oder eine Alkyl(C₁₋₄)gruppe bedeuten; eine Alkylsulfonylgruppe (R-SO₂-), bei der die Gruppe R eine Alkyl(C₁₋₄)gruppe bedeutet.

3. Dikationisches Bis-hydrazon nach Anspruch 2, bei dem die Gruppen R₁ gleich sind und Methyl, Ethyl, 2-Hydroxyethyl, 2-Methoxyethyl, Acetyl (CH₃CO-), Methylsulfonyl (CH₃SO₂-) bedeuten.

4. Dikationisches Bis-hydrazon nach Anspruch 1, bei dem die Gruppen R₁ et R₂ gemeinsam mit dem mit R₁ substituierten Stickstoffatom eine gesättigten oder ungesättigten, 5- oder 6-gliedrigen Heterocyclus bilden, der mit einer oder mehreren Alkylgruppen substituiert ist.

5. Dikationisches Bis-hydrazon nach Anspruch 1, bei dem die Gruppen R₂ et R₃ unabhängig voneinander bedeuten:
- ein Wasserstoffatom;
- ein Halogenatom;
- eine geradkettige oder verzweigte C₁₋₄-Alkylgruppe, die gegebenenfalls mit einer oder mehreren Gruppen substituiert ist, die unter den Gruppen Hydroxy, Alkoxy(C₁₋₂), (Poly)hydroxyalkoxy(C₂₋₄), Amino, (Di)alkylamino(C₁₋₂), Thio (-SH), Alkyl(C₁₋₄)-sulfinyl, Alkyl(C₁₋₄)sulfonyl, Thioalkyl(C₁₋₄) ausgewählt sind;
- eine Phenylgruppe, die gegebenenfalls mit einer oder mehreren Gruppen substituiert ist, die unter den Gruppen Hydroxy, Alkoxy(C₁₋₂), (Poly)hydroxyalkoxy(C₂₋₄), Amino, (Di)alkylamino(C₁₋₂) oder einem Halogenatom, wie Chlor, Fluor oder Brom, ausgewählt sind;
- eine Alkoxy(C₁₋₄)gruppe;
- eine Alkyl(C₁₋₄)sulfonylaminogruppe;
- eine (Poly)hydroxyalkoxy(C₂₋₄)gruppe;
- eine Aminogruppe;
- eine (Di)alkylamino(C₁₋₂)gruppe;
- eine (Poly)hydroxyalkylamino(C₂₋₄)gruppe;
- eine Alkylcarbonylgruppe (R-CO-), bei der die Gruppe R eine Alkyl(C₁₋₄)gruppe bedeutet;
- eine Carbamoylgruppe ((R)₂N-CO-), bei der die Gruppen R unabhängig voneinander ein Wasserstoffatom, eine Alkyl(C₁₋₄)-gruppe bedeuten;
- eine Alkylsulfonylaminogruppe (RSO₂N-) bei der die Gruppe R eine Alkyl(C₁₋₄)gruppe bedeutet;
- eine Aminosulfonylgruppe ((R)₂NSO₂⁻), bei der die Gruppen R unabhängig voneinander ein Wasserstoffatom, eine Alkyl(C₁₋₄)-gruppe bedeuten;
- eine Alkylthiogruppe (RS-), bei der die Gruppe R eine Alkyl(C₁₋₄)gruppe bedeutet;
- eine Thiogruppe (HS-);
- eine Alkylsulfinylgruppe (RSO-), bei der die Gruppe R eine Alkyl(C₁₋₄)gruppe bedeutet;
- eine Alkylsulfonylgruppe (R-SO₂-), bei der die Gruppe R eine Alkyl(C₁₋₄)gruppe bedeutet;
- eine Alkylcarbonylaminogruppe (RCONR'-), bei der die Gruppe R ein Wasserstoffatom oder eine Alkyl(C₁₋₄)gruppe bedeutet und die Gruppe R' ein Wasserstoffatom oder eine Alkyl(C₁₋₄)-gruppe bedeutet.

6. Dikationisches Bis-hydrazon nach Anspruch 5, bei dem die Gruppen R₂ et R₃ unabhängig voneinander ein Wasserstoffatom, Methyl, Ethyl, 2-Hydroxyethyl, 2-Methoxyethyl, Acetyl (CH₃CO-), Methylsulfonyl (CH₃SO₂-), Amid (CH₃CONH-), Hydroxy, Amino, Methylamino, Dimethylamino, 2-Hydroxyethylamino, Methoxy, Ethoxy, Phenyl bedeuten.

7. Dikationisches Bis-hydrazon nach einem der vorhergehenden Ansprüche, bei dem die Gruppen R₅ gleich sind und eine C₁₋₄-Alkylgruppe, die gegebenenfalls mit einer oder mehreren Gruppen substituiert ist, die unter den Gruppen Hydroxy, Alkoxy(C₁₋₂), Amino, (Di)alkylamino(C₁₋₂) ausgewählt sind; oder eine Phenylgruppe, die gegebenenfalls substituiert ist, bedeuten.

8. Dikationisches Bis-hydrazon nach dem vorhergehenden Anspruch, bei dem die Gruppen R₅ gleich sind und vorzugsweise Methyl, Ethyl, 2-Hydoxyetyl, 2-Methoxyethyl, Phenyl bedeuten.

9. Dikationisches Bis-hydrazon nach einem der vorhergehenden Ansprüche, das unter den Verbindungen der folgenden Formeln ausgewählt ist: 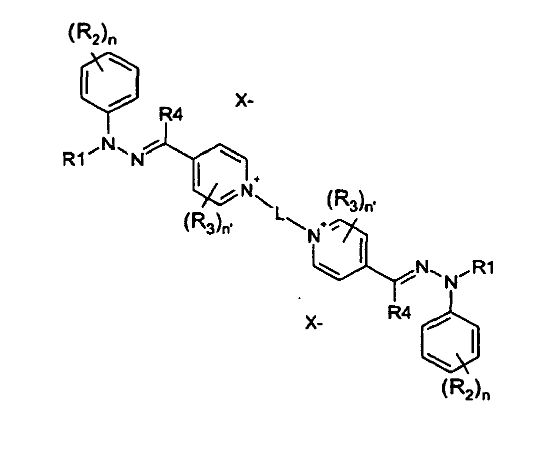 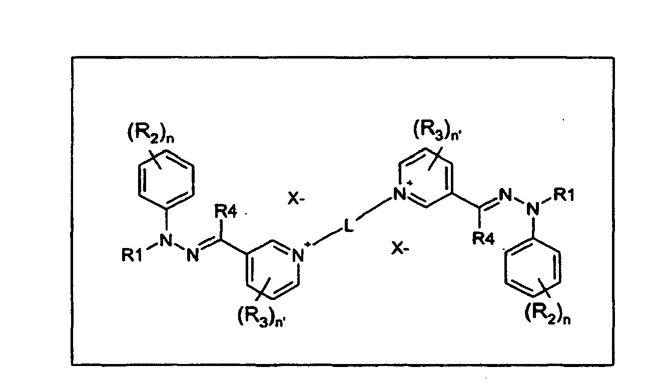 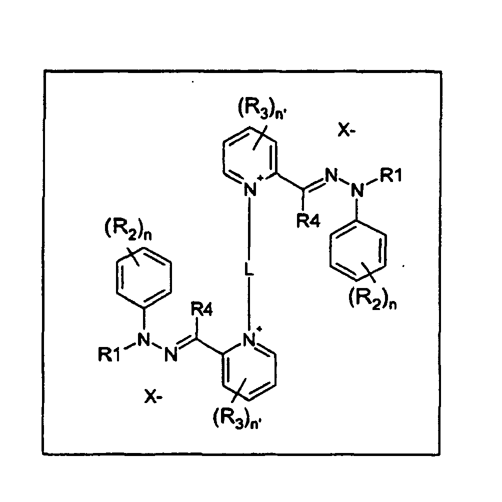 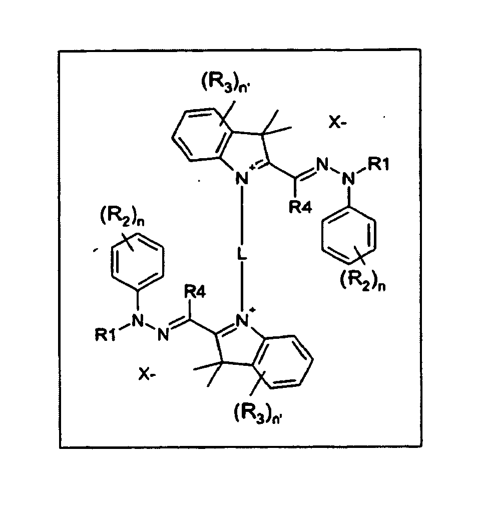 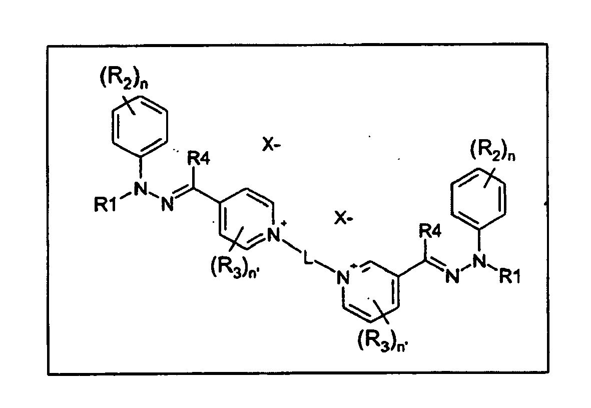 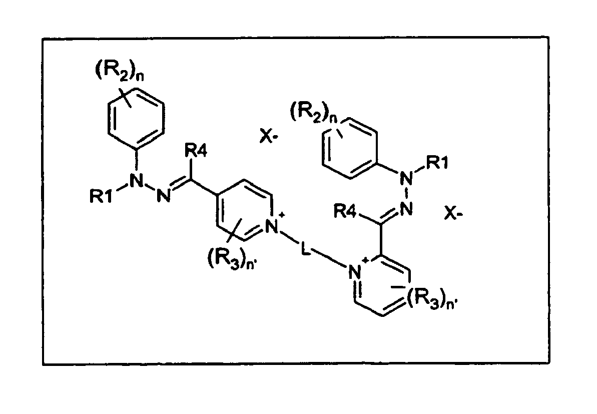 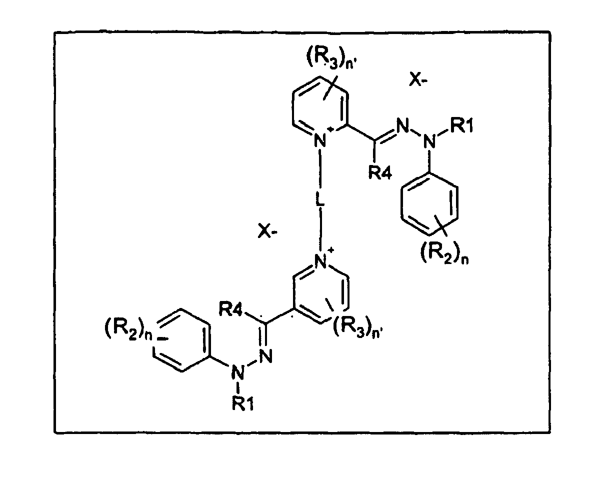 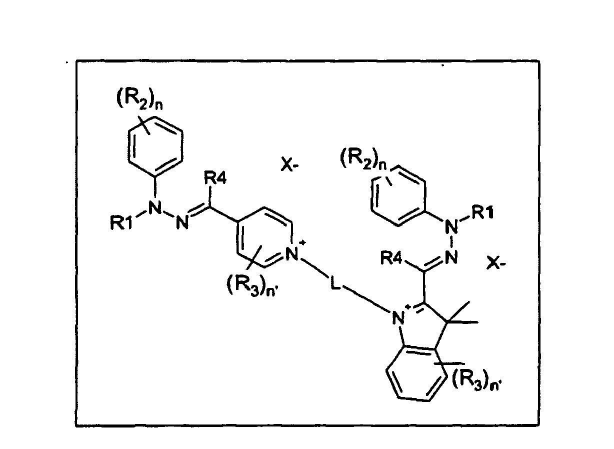 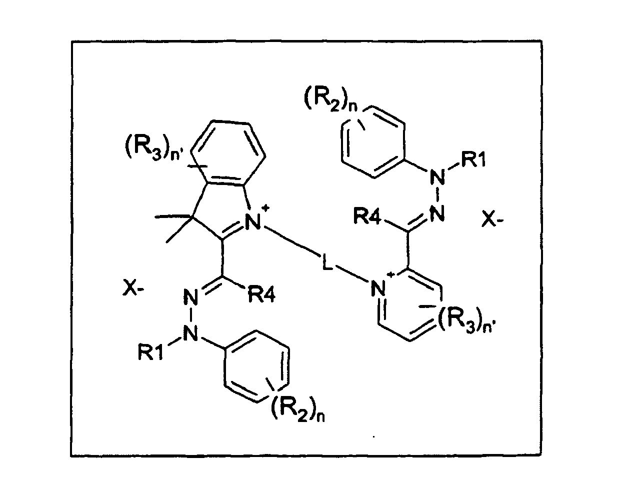 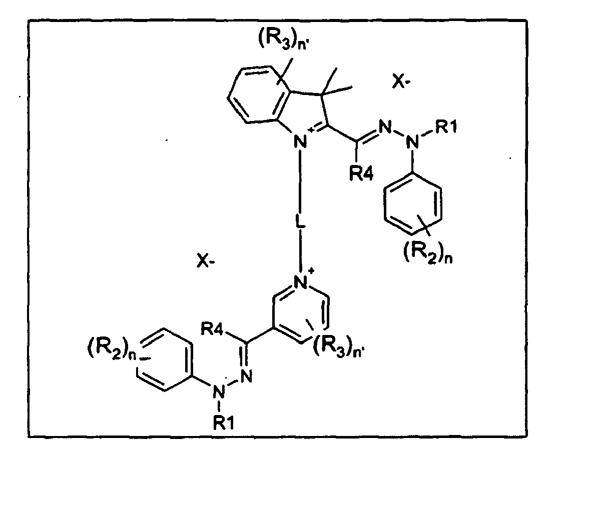 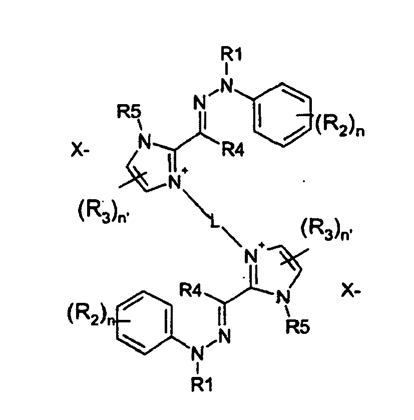 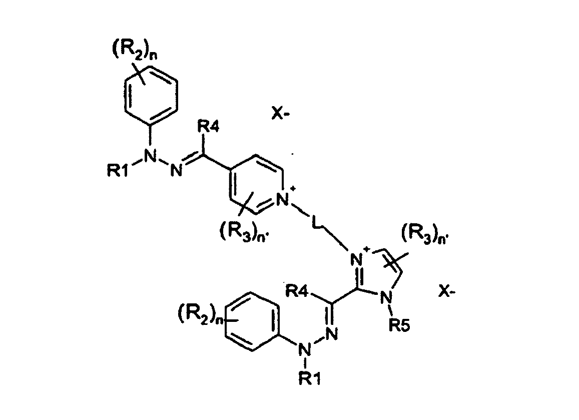 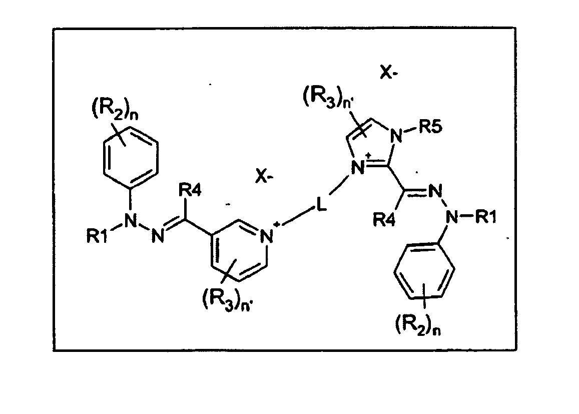 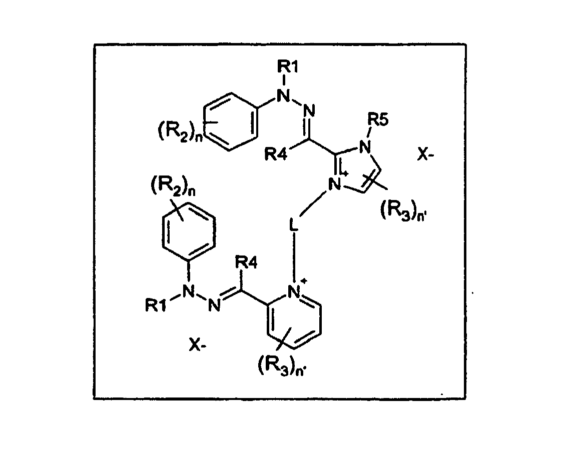 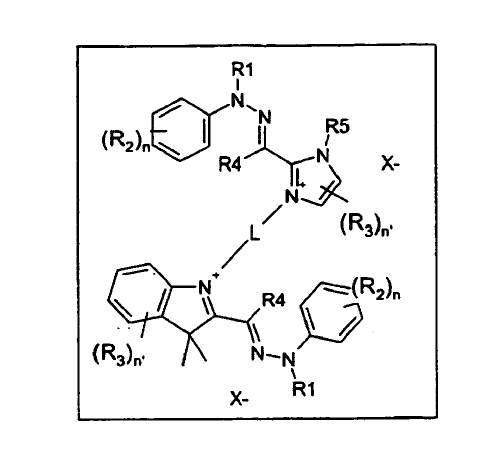 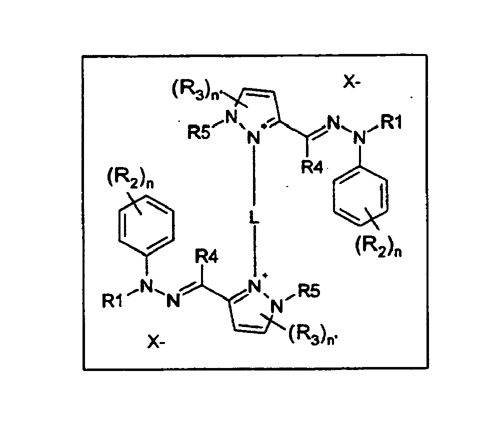 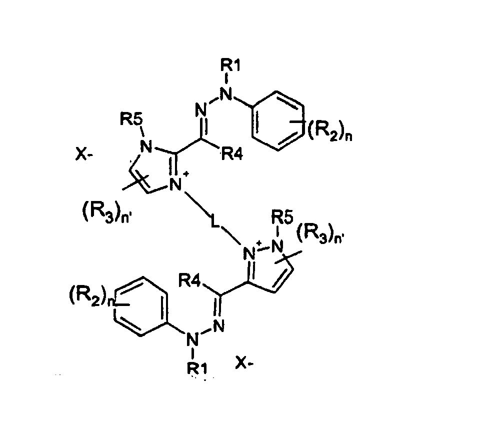 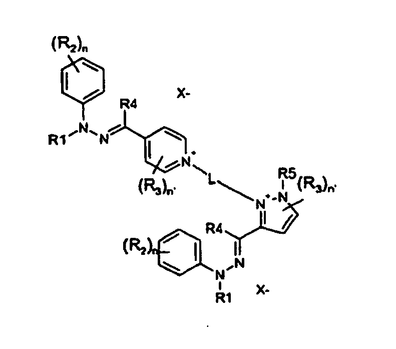 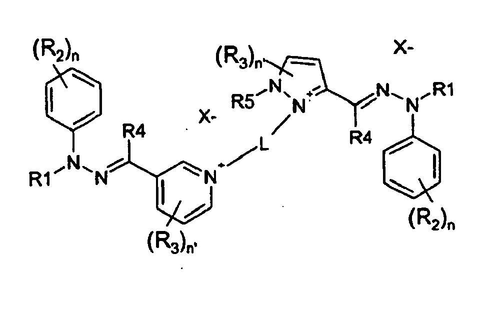 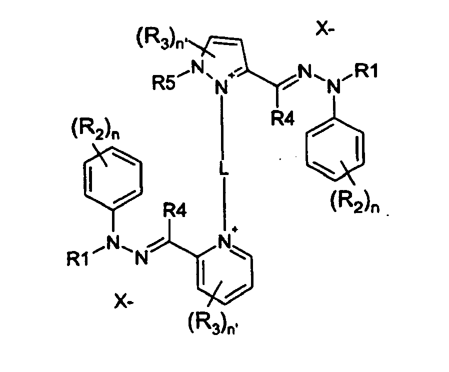 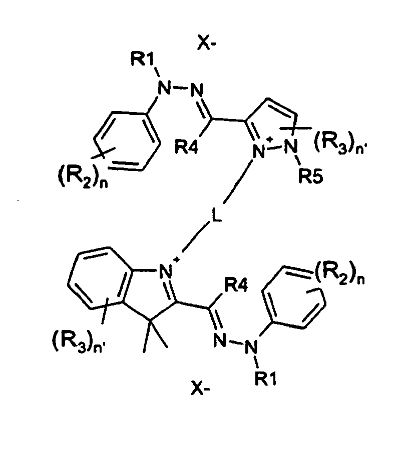 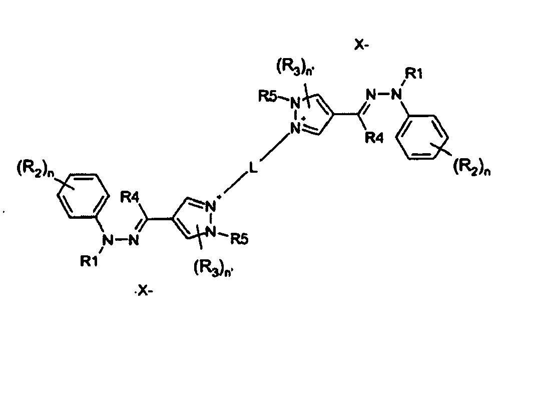 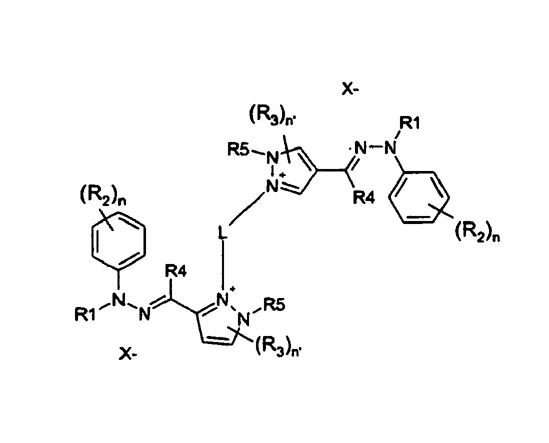 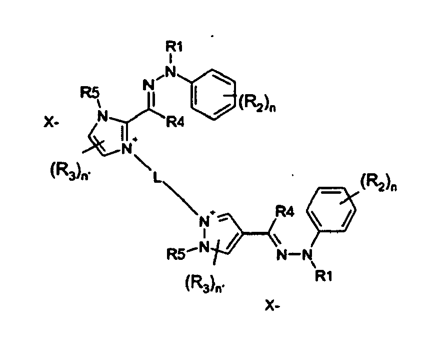 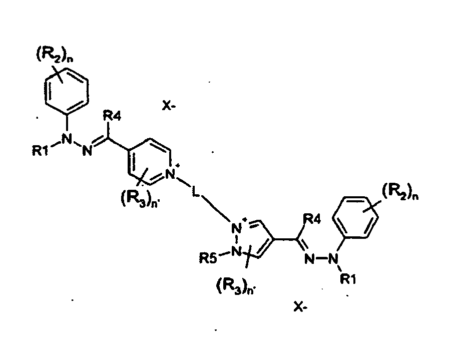 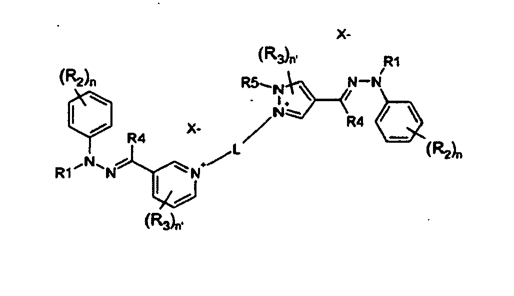 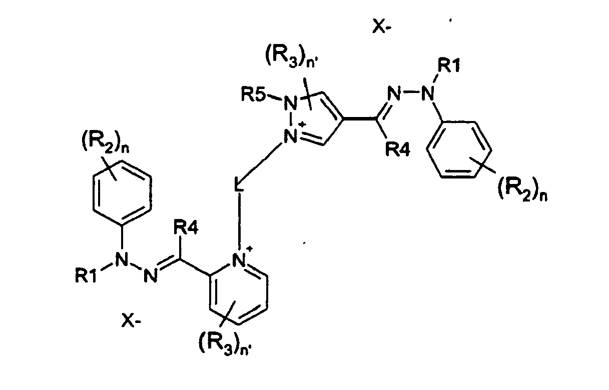 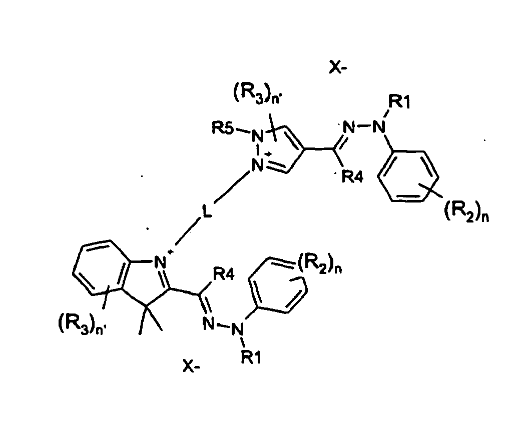 worin die Gruppen R₁, R₂, R₃, R₄, R₅, X, n, n' und L die oben angegebenen Bedeutungen aufweisen.

10. Dikationisches Bis-hydrazon nach einem der vorhergehenden Ansprüche, das unter den Verbindungen der folgenden Formeln ausgewählt ist:
- 4-{(E)-[Methyl(phenyl)hydrazono]methyl}-1-[3-(4-{(E)-[methyl-(phenyl)hydrazono]methyl}pyridinium-1-yl)propyl]pyridiniumdibromid,
- 4-{(E)-[Methyl(phenyl)hydrazono]methyl}-1-[4-(4-{(E)-[methyl-(phenyl)hydrazono]methyl}pyridinium-1-yl)butyl]pyridiniumdibromid,
- 4-{(E)-[Methyl(phenyl)hydrazono]methyl}-1-[5-(4-{(E)-[methyl-(phenyl)hydrazono]methyl}pyridinium-1 -yl)pentyl]pyridiniumdibromid,
- 4-{(E)-[Methyl(phenyl)hydrazono]methyl}-1-[6-(4-{(E)-[methyl(phenyl)hydrazono]methyl}pyridinium-1-yl)hexyl]-pyridiniumdibromid,
- 4-{(E)-[Methyl(phenyl)hydrazono]methyl}-1-[6-(4-{(E)-[methyl-(phenyl)hydrazono]methyl}-chinolinium-1-yl)hexyl]-chinoliniumdibromid,
- 4-{(E)-[Methyl(phenyl)hydrazono]methyl}-1-[6-(4-{(E)-[methyl-(phenyl)hydrazono]methyl}pyridinium-1 -yl)hexyl]chinoliniumdibromid,
- 1-Methyl-3-[5-(1-methyl-2-{(E)-[methyl(phenyl)hydrazono]-methyl}-1H-imidazol-3-ium-3-yl)pentyl]-2-{(E)-[methyl-(phenyl)hydrazono]methyl}-1H-imidazol-3-iumdibromid,
- 1-Methyl-3-[4-(1-methyl-2-{(E)-[methyl(phenyl)hydrazono]-methyl}-1H-benzimidazol-3-ium-3-yl)butyl]-2-{(E)-[methyl-(phenyl)hydrazono]methyl}-1H-benzimidazol-3-iumdibromid,
- 1-[6-(1-Methyl-2-{(E)-[methyl(phenyl)hydrazono]methyl}-1H-imidazol-3-ium-3-yl)hexyl]-4-{(E)-[methyl(phenyl)hydrazono]-methyl}chinoliniumdibromid,
- 1-[6-(1-Methyl-2-{(E)-[methyl(phenyl)hydrazono]methyl}-1H-benzimidazol-3-ium-3-yl)hexyl]-4-{(E)-[methyl(phenyl)-hydrazono]methyl-chinoliniumdibromid,
- 1,1'-Pentan-1,5-diylbis(4-{(E)-[methyl(phenyl)hydrazono]-methylchinolinium)-dibromid,
- 1,1'-Butan-1,4-diylbis(4-{(E)[methyl(phenyl)hydrazono]-methyl}chinolinium)-dibromid,
- 1,1'-Propan-1,3-diylbis(4-{(E)-[methyl(phenyl)hydrazono]-methyl}-chinolinium)-dibromid,
- 2-{(E)-[{4-[(4-Methoxyphenyl)(methyl)amino]phenyl}(methyl)-hydrazono]methyl}-3,3-dimethyl-1-[6-(4- {(E)-[methyl(phenyl)-hydrazono]methyl}pyridinium-1 -yl)hexyl]-3H-indoliumdichlorid,
- 1,1'-Hexan-1,6-diylbis(2-{(E)-[{4-[(4-methoxyphenyl)(methyl)-amino]phenyl}(methyl)hydrazono] methyl}-3,3-dimethyl-3H-indolium)-dichlorid.

11. Farbmittelzusammensetzung zum Färben von Keratinfasern, die in einem zum Färben geeigneten Medium mindestens ein dikationisches Bis-hydrazon nach einem der Ansprüche 1 bis 10 enthält.

12. Farbmittelzusammensetzung nach Anspruch 11, die 0,001 bis 10 Gew.-% und vorzugsweise 0,01 bis 10 Gew.-% mindestens eines dikationischen Bis-hydrazons, bezogen auf das Gesamtgewicht der Zusammensetzung, enthält.

13. Zusammensetzung nach einem der Ansprüche 11 oder 12, die mindestens einen ergänzenden Direktfarbstoff enthält.

14. Farbmittelzusammensetzung nach einem der Ansprüche 11 bis 13, die mindestens ein Farbstoffvorprodukt eines Oxidationsfarbstoffes enthält, das unter den Oxidationsbasen und Kupplern ausgewählt ist.

15. Farbmittelzusammensetzung nach Anspruch 14, die mindestens eine Oxidationsbase enthält, die unter den p-Phenylendiaminen, Bisphenylalkylendiaminen, p-Aminophenolen, o-Aminophenolen, o-Phenylendiaminen, heterocyclischen Basen und deren Additionssalzen ausgewählt ist.

16. Farbmittelzusammensetzung nach Anspruch 15, wobei die Oxidationsbase(n) in einem Mengenanteil im Bereich von 0,001 bis 20 Gew.-% und vorzugsweise 0,005 bis 6 Gew.-%, bezogen auf das Gesamtgewicht der Zusammensetzung, enthalten sind.

17. Farbmittelzusammensetzung nach einem der Ansprüche 14 bis 16, die mindestens einen Kuppler enthält, der unter den m-Phenylendiaminen, m-Aminophenolen, m-Dihydroxybenzolen, Naphthalinkupplern, heterocyclischen Kupplern und deren Additionssalzen ausgewählt ist.

18. Zusammensetzung nach Anspruch 17, wobei der oder die Kuppler in einem Mengenanteil im Bereich von 0,001 bis 20 Gew.-% und vorzugsweise 0,005 bis 6 Gew.-%, bezogen auf das Gesamtgewicht der Zusammensetzung, enthalten sind.

19. Zusammensetzung nach einem der Ansprüche 11 bis 18, die mindestens ein hydroxyliertes Lösemittel enthält, wie Ethanol, Propylenglycol, Glycerin und Monoether von Polyolen.

20. Zusammensetzung nach einem der Ansprüche 11 bis 19, die ferner mindestens einen Zusatzstoff enthält, der unter den anionischen, kationischen, nichtionischen, amphoteren oder zwitterionischen grenzflächenaktiven Stoffen oder deren Gemischen, anionischen, kationischen, nichtionischen, amphoteren oder zwitterionischen Polymeren oder deren Gemischen, anorganischen oder organischen Verdickungsmitteln und insbesondere anionischen, kationischen, nichtionischen und amphoteren, assoziativen polymeren Verdickungsmitteln, Antioxidationsmitteln, Penetrationsmitteln, Maskierungsmitteln, Parfums, Puffern, Dispergiermitteln, Konditioniermitteln, wie beispielsweise flüchtigen oder nicht flüchtigen, modifizierten oder nicht modifizierten Siliconen, Filmbildnern, Ceramiden, Konservierungsmitteln und Trübungsmitteln ausgewählt ist.

21. Zusammensetzung nach einem der Ansprüche 11 bis 20, die ferner mindestens ein Oxidationsmittel enthält, das unter Wasserstoffperoxid, Harnstoffperoxid, Alkalimetallbromaten, Salzen von Persäuren, Persäuren und Oxidasen und vorzugsweise Wasserstoffperoxid ausgewählt ist.

22. Verfahren zum Färben von Keratinfasern, das die Schritte umfasst, die Farbmittelzusammensetzung nach einem der Ansprüche 11 bis 21 auf die Keratinfasern aufzubringen und dann während einer für die Bildung der Färbung ausreichenden Zeitspanne einwirken zu lassen.

23. Verwendung der Zusammensetzung nach einem der Ansprüche 11 bis 22 an Keratinfasern, um Färbungen zu bilden, die gegenüber von außen einwirkenden Agentien und Haarwäschen sehr beständig sind.
